# EUROPEAN PATENT SPECIFICATION

(11) **EP 3 245 197 B1**
(45) Date of publication and mention of the grant of the patent: **03.07.2019**
(21) Application number: 16737826.4
(22) Date of filing: 13.01.2016
(51) Int. Cl.: A23L 27/20, A23L 27/30, A23L 29/30

(54) **COMPOSITIONS AND METHODS FOR MODULATING SWEET TASTE**
ZUSAMMENSETZUNGEN UND VERFAHREN ZUR MODULIERUNG DES SÜSSGESCHMACKS
COMPOSITIONS ET PROCÉDÉS PERMETTANT DE MODULER LE GOÛT SUCRÉ

(30) Priority: 13.01.2015 US 201562102790 P
(43) Date of publication of application: 22.11.2017
(73) Proprietor: Chromocell Corporation, North Brunswick, NJ 08902 (US)
(72) Inventor: LUO, Robert, Zhiyong, New City, NY 10956 (US); CURRAN, Kevin, Somerset, NJ 08873 (US); ZHOU, Hao, Paramus, NJ 07652 (US); HAYDEN, Stuart, Manalapan, NJ 07726 (US); SHAH, Purvi, Bridgewater, NJ 08807 (US)
(74) Representative: Schulz Junghans Patentanwälte PartGmbB
(86) International application number: PCT/US2016/013269
(87) International publication number: WO 2016/115263

(56) References cited:
- WO-A1-2014/153000
- US-A1- 2013 084 252
- US-B1- 6 441 173
- US-B2- 8 133 887
- AOKI ET AL.: 'A convenient synthesis of dihydrocoumarins from phenols and cinnamic acid derivatives.' TETRAHEDRON vol. 61, no. 39, 2005, pages 9291 - 9297, XP027861641 Retrieved from the Internet: <URL:http://www.sciencedirect.com/ science /article/pii/S004040200501255X> [retrieved on 2016-02-17]

## Description

### Field of the Invention

The present disclosure relates to flavor in edible compositions.

### Background of the Invention

There is an increasing demand worldwide to have broader available choices of reduced sugar content in foods and beverages, whether for taste preference, lifestyle reasons or for certain individuals (e.g., diabetic patients) for health-related goals. Accordingly, the health benefits associated with the reduction of sugar content in foods and beverages are desirable. The use of non-caloric artificial and natural high-potency sweeteners to reduce the level of sweeteners such as caloric and non-caloric sweeteners in foods is limited due to temporal and/or flavor issues, *e.g.,* slow onset of sweetness, sweetness linger, bitter, metallic or licorice taste. It is, therefore, desirable to provide compounds that may be added to food products, consumer products and pharmaceuticals which allow for the use of reduced amounts of caloric sweeteners (*e.g.,* sugars) while maintaining desirable sweet taste and avoiding the flavor issues associated with sugar substitutes.

US Patent Application 2013/0084252 describes the use of neoflavonoid compounds for intensifying the sensory impression of sweetness of an edible composition comprising a sweetener.

### Summary of the Invention

A first aspect of the invention is an edible composition as defined in the claims. A second aspect of the invention is a method of enhancing the sweet taste of a sweetener in an edible composition, as defined in the claims. A third aspect of the invention is a method of preparing an edible composition, as defined in the claims. A fourth aspect of the invention is a tabletop sweetener as defined in the claims. A fifth aspect of the invention is a delivery system as defined in the claims.

The present disclosure also provides methods of reducing the amount of a sweetener in an edible composition. The present disclosure further provides a method of enhancing, modulating or potentiating the sweet taste of an edible composition, such as a food, consumer or pharmaceutical product, in a subject. The present disclosure also provides a method of modulating, particularly enhancing or potentiating the activation of a sweet taste receptor. A further aspect of the present disclosure provides compounds or mixtures thereof for masking or reducing unpleasant taste sensations, in particular for masking or reducing the bitter taste sensation of a bitter-tastant.

One aspect of the present disclosure provides compounds for modulating sweet taste (*e.g.,* enhancing sweet taste) of a sweet tastant. In some embodiments, the compound is a neoflavonoid compound. In some embodiments, the neoflavonoid compound has a molecular weight less than about 1000, about 500, or about 300 Daltons. In certain embodiments, the neoflavonoid compound is a compound of Formula (I), comestibly or biologically acceptable salts, solvates, enantiomers, or derivatives thereof, or combinations thereof. In certain embodiments, the neoflavonoid compound is Compound 1, or comestibly or biologically acceptable salts, enantiomers, solvates, or derivatives thereof. The present disclosure also includes edible compositions comprising sweet taste modulating compounds such as the compounds of Formula (I). In addition, the present disclosure also includes edible compositions comprising sweet taste modulating compounds such as Compound 1 or comestibly or biologically acceptable salts, solvates, enantiomers, or derivatives thereof.

Such taste modulators may be combined with any suitable sweetener, such as a natural caloric sweetener, a natural high-potency sweetener, a synthetic sweetener including a synthetic high-potency sweetener, sugar alcohols, rare sugars, sweetener enhancers or combinations thereof, to provide a composition having enhanced sweetness. In some embodiments, the compound of Formula (I), comestibly or biologically acceptable salts, solvates, enantiomers, or derivatives thereof, or combinations thereof is present in the composition in an amount at or below its sweetness threshold. In other embodiments, Compound 1, or comestibly or biologically acceptable salts, solvates, enantiomers, or derivatives thereof is present in the composition in an amount at or below its sweetness threshold. In embodiments wherein the compound is present in the composition in an amount at or below its sweetness threshold, the compound does not act as a sweetener. The compositions may further comprise at least one sweet taste improving composition.

In another aspect, the present disclosure provides a method of enhancing the sweetness of a sweetener comprising combining (i) at least one sweetener, such as a natural caloric sweetener, a natural high-potency sweetener, a synthetic sweetener including a synthetic high-potency sweetener, sugar alcohols, rare sugars, sweetener enhancers or combinations thereof, and (ii) a neoflavonoid sweet taste modulator of the present disclosure, such as a compound of Formula (I), comestibly or biologically acceptable salts, solvates, enantiomers, or derivatives thereof, or enantiomers thereof, or combinations thereof. In some embodiments, the neoflavonoid sweet taste modulator of the present disclosure is Compound 1 comestibly or biologically acceptable salts, solvates, enantiomers, or derivatives thereof. The method may further comprise combining (iii) at least one sweet taste improving composition. The compounds may be added in an amount at or below their sweetness threshold.

The edible compositions include beverage compositions, concentrates (for use in, *e.g.,* beverage compositions), food products, pharmaceutical preparations and table-top sweeteners.

Particular embodiments of the disclosure are set forth in the following numbered paragraphs:
1. A compound of Formula (I): 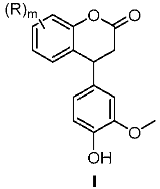 where R is -OH, C₁-C₄ alkyl, -CO₂H, acyl or formyl; m is 2 or 3; and at least one R is not OH; or a comestibly acceptable salt, enantiomer, derivative, or solvate thereof.
2. The compound of Paragraph 1, wherein said compound is (R)5-hydroxy-4-(4-hydroxy-3-methoxyphenyl)-7-methylchroman-2-one, or a comestibly acceptable solvate thereof.
3. A method of enhancing the sweet taste of a sweetener in an edible composition, wherein said method comprises adding an effective amount of a compound of Formula (I), Compound 1, or a comestibly or biologically acceptable salt, solvate, enantiomer, or derivative thereof, or a combination of any of the foregoing compounds, to said edible composition, such that the perception of sweetness intensity of said sweetener is enhanced.
4. The method of Paragraph 3, wherein an effective amount of Compound 1 or a solvate thereof is added.
5. The method of any one of Paragraphs 3 - 4, wherein the sweetener is a caloric sweetener, an artificial sweetener, an artificial high-potency sweetener, a natural high-potency sweetener, a sugar alcohol, a rare sugar, or a combination thereof.
6. The method of Paragraph 5, wherein the caloric sweetener is a carbohydrate selected from sucrose, high fructose corn or starch syrup, glucose, and fructose.
7. The method of Paragraph 6, wherein the caloric sweetener is high fructose corn or starch syrup.
8. The method of Paragraph 6, wherein the caloric sweetener is sucrose.
9. The method of Paragraph 5, wherein the sugar alcohol is a polyol selected from erythritol, sorbitol, mannitol and xylitol.
10. The method of Paragraph 5, wherein the artificial high-potency sweetener is sucralose, acesulfame potassium or another salt thereof, aspartame, alitame, sodium or calcium salt of saccharin, neohesperidin dihydrochalcone, sodium cyclamate, neotame, or advantame, or a salt of any of the foregoing sweeteners.
11. The method of Paragraph 5, wherein the natural high-potency sweetener is a steviol glycoside, rebaudioside A, rebaudioside B, rebaudioside C (dulcoside B), rebaudioside D, rebaudioside E, rebaudioside F, rebaudioside I, rebaudioside H, rebaudioside L, rebaudioside K, rebaudioside J, rebaudioside N, rebaudioside O, rebaudioside M, dulcoside A, rubusoside, stevia leaf extract, stevioside, a glycosylated steviol glycoside, a mogroside, mogroside V, isomogroside, mogroside IV, Luo Han Guo fruit extract, siamenoside, monatin or any salt thereof (monatin SS, RR, RS, SR), curculin, glycyrrhizic acid or any salt thereof, thaumatin, monellin, mabinlin, brazzein, hernandulcin, phyllodulcin, glycyphyllin, phloridzin, trilobatin, baiyunoside, osladin, polypodoside A, pterocaryoside A, pterocaryoside B, mukurozioside, phlomisoside I, periandrin I, abrusoside A, or cyclocarioside I or a mixture of any of the foregoing sweeteners.
12. The method of Paragraph 5, wherein the rare sugar is a D-Psicose, D-Turanose, D-allose, D-Tagatose, D-Sorbose, L-fructose, L-glucose, D-sorbose, L-fructose, L-talose, L-ribose, L-arabinose or a mixture thereof.
13. The method of any one of Paragraphs 3-12, wherein said compound of Formula (I), or Compound 1, or a comestibly or biologically acceptable salt, solvate, enantiomer, or derivative thereof, or a combination of any of the foregoing compounds, is present in the edible composition at a concentration of about 1 ppm to about 100 ppm.
14. The method of Paragraph 13, wherein said compound of Formula (I) or Compound 1, or a comestibly or biologically acceptable salt, solvate, enantiomer, or derivative thereof, or a combination of any of the foregoing compounds, is present in the edible composition at a concentration of about 1ppm to about 60 ppm.
15. The method of Paragraph 13, wherein said compound of Formula (I) or Compound 1, or a comestibly or biologically acceptable salt, solvate, enantiomer, or derivative thereof, or a combination of any of the foregoing compounds, is present in the edible composition at a concentration of about 1 ppm to about 50 ppm or about 5 ppm to about 50 ppm.
16. The method of any one of Paragraphs 3-12, wherein said compound of Formula (I), or Compound 1, or a comestibly or biologically acceptable salt, solvate, enantiomer, or derivative thereof, or a combination of any of the foregoing compounds, is present in the edible composition at a concentration of about 20 ppm to about 3,000 ppm.
17. The method of Paragraph 16, wherein said compound of Formula (I) or Compound 1, or a comestibly or biologically acceptable salt, solvate, enantiomer, or derivative thereof, or a combination of any of the foregoing compounds, is present in the edible composition at a concentration of about 100 ppm to about 1,000 ppm.
18. The method of Paragraph 17, wherein said compound of Formula (I), or Compound 1, or a comestibly or biologically acceptable salt, solvate, enantiomer, or derivative thereof, or a combination of any of the foregoing compounds, is present in the edible composition at a concentration of about 100 ppm to about 300 ppm.
19. The method of any one of Paragraphs 3-12, wherein said compound of Formula (I) or Compound 1, or a comestibly or biologically acceptable salt, solvate, enantiomer, or derivative thereof, or a combination of any of the foregoing compounds, is present in the edible composition at a concentration of about 5 ppm, about 10 ppm, about 15 ppm, about 20 ppm, about 25 ppm, about 30 ppm, about 35 ppm, about 40 ppm, about 45 ppm, or about 50 ppm.
20. The method of any one of Paragraphs 3 - 19, wherein the edible composition is at a pH of about 2.0 to about 8.5.
21. The method of Paragraph 20, wherein the edible composition is at a pH of about 2.0 to about 4.0 or about 6.0 to about 8.0.
22. The method of Paragraph 21, wherein the edible composition is at a pH of about 30 or about 7.0.
23. The method of any one of Paragraphs 3 - 21, wherein the perception of sweetness intensity of said sweetener is enhanced by about 5% to about 50% in an edible composition at a pH of about 6.5 to about 7.5.
24. The method of any one of Paragraphs 3 - 21, wherein the perception of sweetness intensity of said sweetener is enhanced by about 5% to about 50% in an edible composition at a pH of about 2.5 to about 3.5.
25. The method of any one of Paragraphs 3 - 21, wherein the perception of sweetness intensity of said sweetener in the edible composition is enhanced by about 5% to about 100%.
26. The method of Paragraph 25, wherein the perception of sweetness intensity of said sweetener in the edible composition is enhanced by about 5% to about 75%.
27. The method of Paragraph 26, wherein the perception of sweetness intensity of said sweetener in the edible composition is enhanced by about 10% to about 60%.
28. The method of Paragraph 27, wherein the perception of sweetness intensity of said sweetener in the edible composition is enhanced by about 10% to about 50%.
29. The method of Paragraph 28, wherein the perception of sweetness intensity of said sweetener in the edible composition is enhanced by about 10% to about 25%.
30. The method of any one of Paragraphs 3-29, wherein the edible composition is a beverage.
31. The method of Paragraph 30, wherein the beverage is a non-alcoholic beverage.
32. The method of any one of Paragraphs 3-31, wherein the edible composition further comprises one or more of an antioxidant, a vitamin, glucosamine, a dietary fiber, a hydration agent, a probiotic, a prebiotic, a phytosterol, an omega-3 oil, a fatty acid, a saponin, a natural or synthetic preservative, a mineral, a weight management agent, an osteoporosis management agent, a phytoestrogen, a long chain primary aliphatic saturated alcohol, a phytosterol or a combination of any of the foregoing.
33. The method of any one of Paragraphs 3-32, wherein said edible composition further comprises one or more sweet taste improving additive.
34. The method of Paragraph 33, wherein said sweet taste improving additive is selected from the group consisting of a carbohydrate, a polyol, a glycoside, an amino acid, a sugar acid, a polyamino acid, a nucleotide, a salt, an organic acid, an organic ester, a flavoring agent, a sweet flavor, an alcohol, a flavonoid, a bitter compound, a protein, a protein hydrolysate, an emulsifier, a surfactant and a polymer.
35. The method of Paragraph 34, wherein the sweet taste improving additive is a sweet flavor.
36. The method of Paragraph 35, wherein the sweet taste improving additive is a polyol.
37. The method of Paragraph 36, wherein the polyol is erythritol.
38. The method of Paragraph 37, wherein the ratio of erythritol to the compounds of Formula (I), Compound 1, or a comestibly or biologically acceptable salt, solvate, enantiomer, or derivative thereof, or a combination of any of the foregoing compounds, is about 1:1 to about 800:1 by weight.
39. The method of Paragraph 38, wherein the ratio of erythritol to the compound of Formula (I), Compound 1, or a comestibly or biologically acceptable salt, solvate, enantiomer, or derivative thereof, or a combination of any of the foregoing compounds, is about (30-200):1 or about (50-100):1 by weight.
40. The method of Paragraph 33, wherein the sweet taste improving additive is an amino acid.
41. The method of Paragraph 40, wherein the amino acid is glycine, alanine, lysine, glutamic acid, taurine, serine or proline.
42. The method of Paragraph 40 or 41, wherein the amino acid is present in a concentration of about 10 ppm to about 25,000 ppm.
43. The method of Paragraph 42, wherein the amino acid is present in a concentration of about 100 ppm to about 5,000 ppm.
44. The method of Paragraph 33, wherein said sweet taste improving additive is a salt.
45. The method of Paragraph 44, wherein said salt is NaCl, KCl or MgCl₂.
46. The method of any one of Paragraphs 3 - 45, wherein the perception of sweetness intensity of said sweetener is enhanced, as measured in an in-vitro assay for a sweet, responsive assay.
47. The method of any one of Paragraphs 3 - 45, wherein the perception of sweetness intensity of said sweetener is enhanced, as measured in an in-vivo assay for a sweet, responsive assay.
48. A method of reducing the bitter taste of a bitter tastant in an edible composition, wherein said method comprises adding an effective amount of a compound of Formula (I), Compound 1, or a comestibly or biologically acceptable salt, solvate, enantiomer, or derivative thereof, or a combination of any of the foregoing compounds, to said edible composition, such that the perception of bitter taste of said bitter tastant is reduced.
49. The method of any one of Paragraphs 3 - 48, wherein the compound of Formula (I), Compound 1, or a comestibly or biologically acceptable salt, solvate, enantiomer, or derivative thereof, or a combination of any of the foregoing compounds, are used to decrease off-taste in an edible composition.
50. The method of Paragraph 49, wherein the off-taste is bitter, metallic or astringent.
51. A composition comprising a compound of Formula (I), Compound 1, or a comestibly or biologically acceptable salt, solvate, enantiomer, or derivative thereof, or a combination of any of the foregoing compounds, wherein said composition is edible and capable of enhancing the sweet taste of a sweetener.
52. The composition of Paragraph 51, wherein the composition further comprises a sweetener.
53. The composition of any one of Paragraphs 51-52, wherein the sweetener is a caloric sweetener, an artificial sweetener, a natural high-potency sweetener, or a combination of any of the foregoing sweeteners.
54. The composition of Paragraph 53, wherein the caloric sweetener is a carbohydrate selected from sucrose, high fructose corn or starch syrup, glucose, and fructose.
55. The composition of Paragraph 53 wherein the caloric sweetener is high fructose corn or starch syrup.
56. The composition of Paragraph 53 wherein the caloric sweetener is sucrose.
57. The composition of Paragraph 53, wherein the caloric sweetener is a polyol selected from erythritol, sorbitol, mannitol and xylitol.
58. The composition of Paragraph 53, wherein the artificial sweetener is sucralose, acesulfame potassium or another salt thereof, aspartame, alitame, a sodium or calcium salt of saccharin, neohesperidin dihydrochalcone, sodium cyclamate, neotame, or advantame, or a salt thereof.
59. The composition of Paragraph 53, wherein the natural high-potency sweetener is steviol glycoside, rebaudioside A, rebaudioside B, rebaudioside C (dulcoside B), rebaudioside D, rebaudioside E, rebaudioside F, rebaudioside I, rebaudioside H, rebaudioside L, rebaudioside K, rebaudioside J, rebaudioside N, rebaudioside O, Rebaudioside M, dulcoside A, rubusoside, stevia leaf extract, stevioside, a glycosylated steviol glycoside, mogroside V, isomogroside, mogroside IV, Luo Han Guo fruit extract, siamenoside, monatin or any salt thereof (monatin SS, RR, RS, SR), curculin, glycyrrhizic acid or any salt thereof, thaumatin, monellin, mabinlin, brazzein, hernandulcin, phyllodulcin, glycyphyllin, phloridzin, trilobatin, baiyunoside, osladin, polypodoside A, pterocaryoside A, pterocaryoside B, mukurozioside, phlomisoside I, periandrin I, abrusoside A, or cyclocarioside I or a mixture of any of the foregoing sweeteners.
60. The composition of any one of Paragraphs 51-59, wherein said composition comprises Compound 1 or a comestibly acceptable salt or solvate thereof.
61. The composition of any one of Paragraphs 51-59, wherein said compound of Formula (I), Compound 1, or a comestibly or biologically acceptable salt, solvate, enantiomer, or derivative thereof, or a combination of any of the foregoing compounds, is present in the edible composition at a concentration of about 0.1 ppm to about 100 ppm.
62. The composition of Paragraph 61, wherein said compound of Formula (I), Compound 1, or a comestibly or biologically acceptable salt, solvate, enantiomer, or derivative thereof, or a combination of any of the foregoing compounds, is present in the edible composition at a concentration of about 5 ppm to about 60 ppm.
63. The composition of Paragraph 62, wherein said compound of Formula (I), Compound 1, or a comestibly or biologically acceptable salt, solvate, enantiomer, or derivative thereof, or a combination of any of the foregoing compounds, is present in the edible composition at a concentration of about 10 ppm to about 50 ppm.
64. The composition of any one of Paragraphs 51-59, wherein said compound of Formula (I), Compound 1, or a comestibly or biologically acceptable salt, solvate, enantiomer, or derivative thereof, or a combination of any of the foregoing compounds, is present in the edible composition at a concentration of about 30 ppm to about 3,000 ppm.
65. The composition of Paragraph 64, wherein said compound of Formula (I), Compound 1, or a comestibly or biologically acceptable salt, solvate, enantiomer, or derivative thereof, or a combination of any of the foregoing compounds, is present in the edible composition at a concentration of about 100 ppm to about 1,000 ppm.
66. The composition of Paragraph 65, wherein said compound of Formula (I), Compound 1, or a comestibly or biologically acceptable salt, solvate, enantiomer, or derivative thereof, or a combination of any of the foregoing compounds, is present in the edible composition at a concentration of about 100 ppm to about 300 ppm.
67. The composition of Paragraph 61, wherein said compound of Formula (I), Compound 1, or a comestibly or biologically acceptable salt, solvate, enantiomer, or derivative thereof, or a combination of any of the foregoing compounds, is present in the edible composition at a concentration of about 1 ppm, about 5 ppm, about 10 ppm, about 15 ppm, about 20 ppm, about 25 ppm, about 30 ppm, about 35 ppm, about 40 ppm, about 45 ppm, or about 50 ppm.
68. The composition of any one of Paragraphs 51-67, wherein the edible composition is at a pH of about 2.0 to about 8.5.
69. The composition of Paragraph 68, wherein the edible composition is at a pH of about 2.0 to about 4.0, about 3.0 to about 7.0, or about 6.0 to about 8.0.
70. The composition of Paragraph 69, wherein the edible composition is at a pH of about 3.0 or about 7.0.
71. The composition of any one of Paragraphs 51-70, wherein the perception of sweetness intensity of said sweetener in the edible composition is enhanced by about 5% to about 100%.
72. The composition of Paragraph 71, wherein the perception of sweetness intensity of said sweetener in the edible composition is enhanced by about 5% to about 70%.
73. The composition of Paragraph 72, wherein the perception of sweetness intensity of said sweetener in the edible composition is enhanced by about 5% to about 50%.
74. The composition of Paragraph 73, wherein the perception of sweetness intensity of said sweetener in the edible composition is enhanced by about 10% to about 60%.
75. The composition of Paragraph 74, wherein the perception of sweetness intensity of said sweetener in the edible composition is enhanced by about 10% to about 50%.
76. The composition of Paragraph 75, wherein the perception of sweetness intensity of said sweetener in the edible composition is enhanced by about 10% to about 40%.
77. The composition of any one of Paragraphs 51-76, wherein the edible composition is a beverage.
78. The composition of Paragraph 77, wherein the beverage is a non-alcoholic beverage.
79. The composition of any one of Paragraphs 51 - 78, wherein the edible composition further comprises one or more of an antioxidant, a vitamin, glucosamine, a fiber, a hydration agent, a probiotic, a prebiotic, a phytosterol, an omega-3 oil, a fatty acid, a saponin, a natural or synthetic preservative, a mineral, a weight management agent, an osteoporosis management agent, a phytoestrogen, a long chain primary aliphatic saturated alcohol, a phytosterol or a combination of any of the foregoing.
80. The composition of any one of Paragraphs 51-79, wherein said edible composition further comprises one or more sweet taste improving additive.
81. The composition of Paragraph 80, wherein said sweet taste improving additive is selected from the group consisting of a carbohydrate, a polyol, a glycoside, an amino acid, a sugar acid, a polyamino acid, a nucleotide, a salt, an organic acid, an organic ester, a flavoring agent, a sweet flavor, an alcohol, a flavonoid, a bitter compound, a protein, a protein hydrolysate, an emulsifier, a surfactant and a polymer.
82. The composition of Paragraph 81, wherein the sweet taste improving additive is a polyol.
83. The composition of Paragraph 82, wherein the polyol is erythritol.
84. The composition of Paragraph 83, wherein the ratio of erythritol to the compound of Formula (I), Compound 1, or a comestibly or biologically acceptable salt, solvate, enantiomer, or derivative thereof, or a combination of any of the foregoing compounds, is about 1:1 to about 800:1 by weight.
85. The composition of Paragraph 84, wherein the ratio of erythritol to the compound of Formula (I), or Compound 1, or a comestibly or biologically acceptable salt, solvate, enantiomer, or derivative thereof, or a combination of any of the foregoing compounds, is about (30-200):1 or about (50-100):1 by weight.
86. The composition of Paragraph 81, wherein the sweet taste improving additive is an amino acid.
87. The composition of Paragraph 86, wherein the amino acid is glycine, alanine, taurine, serine or proline.
88. The composition of Paragraph 86 or 87, wherein the amino acid is present in a concentration of about 10 ppm to about 25,000 ppm.
89. The composition of Paragraph 88, wherein the amino acid is present in a concentration of about 100 ppm to about 1000 ppm.
90. The composition of Paragraph 80, wherein said sweet taste improving additive is a salt.
91. The composition of Paragraph 90, wherein said salt is NaCl, KCl or MgCl₂.
92. A method of preparing an edible composition comprising:
   (a) providing a comestibly acceptable carrier; and
   (b) adding to said comestibly acceptable carrier a compound of Formula (I), Compound 1, or a comestibly or biologically acceptable salt, solvate, enantiomer, or derivative thereof, or a combination of any of the foregoing compounds.
93. The method of Paragraph 92, wherein the comestibly acceptable carrier comprises a sweetener.
94. The method of any one of Paragraphs 92-93, wherein the sweetener is a caloric sweetener, an artificial sweetener, a natural high-potency sweetener, or a combination of any of the foregoing sweeteners.
95. The method of Paragraph 94, wherein the caloric sweetener is a carbohydrate selected from sucrose, high fructose corn or starch syrup, glucose, and fructose.
96. The method of Paragraph 94 wherein the caloric sweetener is high fructose corn or starch syrup.
97. The method of Paragraph 94, wherein the caloric sweetener is a polyol selected from erythritol, sorbitol, mannitol and xylitol.
98. The method of Paragraph 94, wherein the artificial sweetener is sucralose, acesulfame potassium or another salt thereof, aspartame, alitame, a sodium or calcium salt of saccharin, neohesperidin dihydrochalcone, sodium cyclamate, neotame, or advantame, or a salt thereof.
99. The method of Paragraph 94, wherein the natural high-potency sweetener is steviol glycoside, rebaudioside A, rebaudioside B, rebaudioside C (dulcoside B), rebaudioside D, rebaudioside E, rebaudioside F, rebaudioside I, rebaudioside H, rebaudioside L, rebaudioside K, rebaudioside J, rebaudioside N, rebaudioside O, rebaudioside M, dulcoside A, rubusoside, stevia leaf extract, stevioside, a glycosylated steviol glycoside, mogroside V, isomogroside, mogroside IV, Luo Han Guo fruit extract, siamenoside, monatin or one of its salts (monatin SS, RR, RS, SR), curculin, glycyrrhizic acid or one its salts, thaumatin, monellin, mabinlin, brazzein, hernandulcin, phyllodulcin, glycyphyllin, phloridzin, trilobatin, baiyunoside, osladin, polypodoside A, pterocaryoside A, pterocaryoside B, mukurozioside, phlomisoside I, periandrin I, abrusoside A, or cyclocarioside I or a mixture thereof.
100. The composition of Paragraph 77 wherein said composition is a beverage selected from the group consisting of a non-carbonated beverage, a carbonated beverage, a cola, a root beer, a fruit flavored beverage, a citrus-flavored beverage, a fruit juice, a fruit-containing beverage, a vegetable juice, a vegetable containing beverage, tea, coffee, a dairy beverage, a sports drink, an energy drink, and enhanced or flavored water.
101. The composition of Paragraph 51, wherein the composition is used in food, a beverage product, a pharmaceutical composition, a nutritional product, a functional product, a dietary supplement, an over-the-counter medication, or an oral care product.
102. A tabletop sweetener composition comprising a sweetener and a sweet taste modulator according to Formula (I), Compound 1, or a comestibly or biologically acceptable salt, solvate, enantiomer, or derivative thereof, or a combination of any of the foregoing compounds.
103. The tabletop sweetener composition of Paragraph 102, further comprising at least one bulking agent, additive, anti-caking agent, functional ingredient or a combination thereof.
104. The tabletop sweetener composition of Paragraph 102, wherein the tabletop sweetener composition is in the form of a liquid.
105. The composition of Paragraph 101, wherein the food or beverage product is selected from the group consisting of soup, a powdered soft drink, a bakery product, a chewing gum, a confection, a cereal, an edible gel, jam or jelly, a spread, ketchup, a dairy product, a frozen dairy product, a gelatin/pudding, and an ice-cream.
106. A delivery system selected from the group consisting of a co-crystallized flavor composition with a sugar or a polyol, an agglomerated flavor composition, a compacted flavor composition, a dried flavor composition, a particle flavor composition, a spheronized flavor composition, a granular flavor composition or a liquid flavor composition, wherein the flavor composition comprises a compound of Formula (I), Compound 1, or a comestibly or biologically acceptable salt, solvate, enantiomer, or derivative thereof, or a combination of any of the foregoing compounds.
107. A method of reducing the bitter taste of a bitter tastant in an edible composition, wherein said method comprises adding an effective amount of a compound of Formula (I) Compound 1, or a comestibly or biologically acceptable salt, solvate, enantiomer, or derivative thereof, or a combination of any of the foregoing compounds, to said edible composition, such that the perception of bitter taste of said bitter tastant is reduced.
108. A composition comprising a compound of Formula (I), Compound 1, or a comestibly or biologically acceptable salt, solvate, enantiomer, or derivative thereof, or a combination of any of the foregoing compounds, wherein said composition is edible and capable of reducing the bitter taste of a bitter tastant.
109. A composition comprising a compound of Formula (I), Compound 1, or a comestibly or biologically acceptable salt, solvate, enantiomer, or derivative thereof, or a combination of any of the foregoing compounds, wherein said composition is edible and capable of enhancing the sweet taste of a sweetener and reducing the bitter taste of a bitter tastant.
110. The composition of Paragraph 109, wherein the composition is a beverage.

### Brief Description of the Drawings

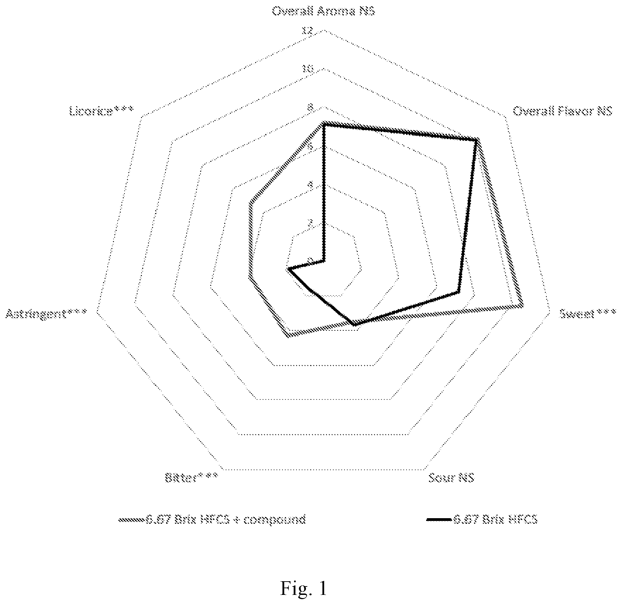

**Figure 1** depicts descriptive analysis assessment of 7 taste attributes for Compound 1 in a lemon-lime matrix sweetened with high fructose corn syrup solution at pH 7. Compound 1 is compared in spider plot format to a control sweetener solution.

### Detailed Description of the Invention

In order that the disclosure described herein may be fully understood, the following detailed description is set forth.

Unless defined otherwise, all technical and scientific terms used herein have the same meaning as those commonly understood by one of ordinary skill in the art to which this disclosure belongs. Although methods and materials similar or equivalent to those described herein can be used in the practice or testing of the present disclosure, suitable methods and materials are described below. The materials, methods and examples are illustrative only, and are not intended to be limiting.

Throughout this specification, the word "comprise" or variations such as "comprises" or "comprising" will be understood to imply the inclusion of a stated integer or groups of integers but not the exclusion of any other integer or group of integers.

The term "aliphatic" refers to straight chain or branched hydrocarbons that are completely saturated or that contain one or more units of unsaturation. For example, aliphatic groups include substituted or unsubstituted linear or branched alkyl, alkenyl, and alkynyl groups. Unless indicated otherwise, the term "aliphatic" encompasses both substituted and unsubstituted hydrocarbons.

The term "alkoxy" refers to O-alkyl substituent, wherein the alkyl portion may be optionally substituted. Examples of alkoxy substituents include, but are not limited to, methoxy, ethoxy, n-propoxy, isopropoxy and n-butoxy. Also, explicitly included within the scope of the term "alkoxy" are O-alkenyl or O-alkynyl groups. In all cases, the alkyl, alkene, and alkyne portions may be optionally substituted.

The term "alkyl" refers to both straight and branched saturated chains containing, for example, 1-3, 1-6, 1-9, or 1-12 carbon atoms. An alkyl group may be optionally substituted.

The term "alkenyl" refers to both straight and branched saturated chains containing, for example, 2-3, 2-6, 2-9, or 2-12 carbon atoms, and at least one carbon-carbon double bond. An alkenyl group may be optionally substituted.

The term "alkynyl" refers to both straight and branched saturated chains containing, for example, 2-3, 2-6, 2-9, or 2-12 carbon atoms, and at least one carbon-carbon triple bond. An alkynyl group may be optionally substituted.

The term "bitter" or "bitter taste" as used herein refers to the perception or gustatory sensation resulting following the detection of a bitter tastant. The following flavor attributes may contribute to bitter taste: astringent, bitter-astringent, metallic, bitter-metallic, as well as off-tastes aftertastes and undesirable tastes including but not limited to freezer-burn and card-board taste or a combination of these. It is noted that, in the art, the term "off-taste" is often synonymous with "bitter taste."

The term "bitter tastant" refers to a compound that activates or that can be detected by a bitter taste receptor and/or confers the perception of a bitter taste in a subject. A "bitter tastant" also refers to a multiplicity of compounds that combine to activate or be detected by a bitter taste receptor and/or confer the perception of a bitter taste in a subject. Those of skill in the art can readily identify and understand what is meant by a bitter tastant. Bitter tastants may include substances which also possess or primarily possess a sweet taste. Examples of such substances include steviol glycosides and acesulfame potassium.

The term "comestibly or biologically acceptable salt" refers to any comestibly or biologically acceptable salt, ester, or salt of such ester, of a compound of the present disclosure, which, upon ingestion, is capable of providing (directly or indirectly) a compound of the present disclosure, or a metabolite, residue or portion thereof, characterized by the ability to enhance the perception of a sweet taste attributed to a sweetener and/or reduce the perception of bitter taste due to a bitter tastant. Similarly, the term "comestibly or biologically acceptable derivative" refers to any comestibly or biologically acceptable derivative of a compound of the present disclosure, which, upon ingestion, is capable of providing (directly or indirectly) a compound of the present disclosure, or a metabolite, residue or portion thereof, characterized by the ability to enhance the perception of a sweet taste attributable to a sweetener and reduce the perception of a bitter taste attributed to a bitter tastant. A "comestible product" is a product suitable for oral use, such as eating or drinking. Therefore, a comestibly acceptable compound is an edible compound. If a comestibly or biologically acceptable salt of a compound of the present disclosure is used, such salt is preferably derived from inorganic or organic acids and bases. Examples of such salts include, but are not limited to, those derived from appropriate bases, including alkali metal (e.g., sodium and potassium), alkaline earth metal (e.g., magnesium), ammonium and N⁺(C₁₋₄ alkyl)₄ salts.

The term "combination" as it relates to compounds of the disclosure refers to two or more compounds of Formula (I) (including Compound 1), or comestibly or biologically acceptable salts, solvates, or enantiomers thereof.

The term "solvate" refers to a physical association of a compound of this disclosure with one or more solvent molecules that does not interfere with the biological activity of the compound. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolatable solvates. Non-limiting examples of suitable solvates include ethanolates, hydrates, and the like. A "hydrate" is a solvate wherein the solvent molecule is water and includes a hemihydrate, a monohydrate, a sesquihydrate, a dihydrate, etc.

The term "diet" collectively refers to the food products and/or beverages consumed by a subject. A subject's "diet" also includes any consumer products or pharmaceutical compositions the subject ingests.

The term "flavor modifier" refers to a compound or a mixture of compounds that, when added to an edible composition, such as a food product, changes the individual characteristics of a food flavor (odor and/or taste). Flavor modification effects can include increasing, decreasing, masking, eliminating, reducing, enhancing or changing the perception of relevant sensorial characteristics of flavor in the edible composition. The ability of flavor modifiers to modify flavor may be independent of their aromatic or taste characteristics.

The term "pharmaceutically active ingredient" refers to a compound in a pharmaceutical composition which is biologically active.

The term "replace" or "replacing" refers to substituting one compound for another compound in or in the preparation of, for example, an edible composition, such as food product. It includes complete and partial replacements or substitutions.

An aliphatic group may contain one or more substituents. Examples of suitable substituents on a saturated or unsaturated carbon of an aliphatic group include, but are not limited to, halogen, -CF₃, -R', -OR', -OH, -SH, -SR', protected OH (such as acyloxy), -NO₂, -CN, -NH₂, -NHR', -N(R')₂, -NHCOR', -NHCONH₂, -NHCONHR', -N HCON(R')₂, -NRCOR', -NHCO₂H, -NHCO₂R', -CO₂R', -CO₂H, -COR', -CONH₂, -CO NHR', -CON(R')₂, -S(O)₂H, -S(O)₂R', -S(O)₃H, -S(O)₃R', -S(O)₂NH2' -S(O)H, -S(O)R' , -S(O)₂NHR', -S(O)₂N(R')₂, -NHS(O)₂H, or -NHS(O)₂R' =O, =S, =NNHR', =NN(R')₂, =N-OR', =NNHCOR', =NNHCO₂R', =NNHSO₂R', =N-CN, or =NR', wherein R' is selected from H, aliphatic, carbocyclyl, heterocyclyl, aryl, aralkyl, heteroaryl, or heteroaralkyl and each R' is optionally substituted with one or more halogen, nitro, cyano, amino, -NH-(unsubstituted aliphatic), -N-(unsubstituted aliphatic)₂, carboxy, carbamoyl, hydroxy, -O-(unsubstituted aliphatic), -SH, -S-(unsubstituted aliphatic), CF₃, -S(O)₂NH₂' unsubstituted aliphatic, unsubstituted carbocyclyl, unsubstituted heterocyclyl, unsubstituted aryl, unsubstituted aralkyl, unsubstituted heteroaryl, or unsubstituted heteroaralkyl. Guided by this specification, the selection of suitable substituents is within the knowledge of one skilled in the art.

As defined herein, the compounds of the disclosure are intended to include all stereochemical forms of the compound, including geometric isomers (*i.e.,* E, Z) and optical isomers (*i.e.,* R, S). Single stereochemical isomers as well as enantiomeric mixtures of the present compounds are within the scope of the disclosure and are specifically contemplated. Unless otherwise stated, formulas depicted herein are also meant to include compounds which differ only in the presence of one or more isotopically enriched atoms. For example, compounds having the present formulas except for the replacement of a hydrogen by a deuterium or tritium, or the replacement of a carbon by a ¹³C- or ¹⁴C-enriched carbon are within the scope of this disclosure.

One aspect of present disclosure provides edible compositions comprising a sweet taste modulator of the present disclosure, including food products, consumer products, and pharmaceutical compositions comprising said compounds, and methods of preparing a such compositions. The present disclosure also provides methods of reducing the amount of a sweetener in an edible composition, methods for reducing caloric intake, methods of enhancing or potentiating sweet taste of a sweetener, methods of blocking a bitter taste, methods of enhancing or potentiating the activity of a sweet taste receptor, and methods of synthesizing sweet taste modulators. The present disclosure also includes reducing the amount of a sweetener in an edible composition or diet by replacing an amount of sugar or the other sweetener with an amount of one or more compounds or one or more extracts containing compounds of the present disclosure.

### Sweet Taste Modulators

According to one aspect, the disclosure provides compounds for modulating sweet taste (e.g., enhancing or potentiating the sweet taste of a sweetener).

As used herein, the term "sweet taste modulators" refers to flavor substances with taste modifying properties. Sweetness enhancers are understood to be a type of "sweet taste modulator" where perception of sweetness is increased in a manner not solely attributable to the inherent sweetness of the sweetness enhancer alone. The term "sweetness enhancer" is understood to include at least compositions capable of enhancing or intensifying the perception of sweet taste of sweetener compositions or sweetened compositions. The term "sweetness enhancer" is synonymous with the terms "sweet taste modulating compound," "sweet taste potentiator," "sweetness potentiator," "sweetness amplifier," and "sweetness intensifier." Generally, the sweet taste modulating compounds provided herein (which serve to enhance the perception of sweetness) may enhance or potentiate the sweet taste of sweeteners without providing any noticeable sweet taste by themselves at acceptable use levels; however, the sweetness enhancers may themselves provide sweet taste at concentrations above a sweetness threshold level. It is noted that the sweetness enhancers may be effective as enhancers even if present at concentrations above their sweetness threshold level. In such embodiments, there is major contribution of the sweetness enhancer to the sweetness of the composition via enhancement of the inherently sweet taste attributed to a sweetener, where the sweetener is also present in the composition. As used herein, the term "sweetness threshold level" is understood to include at least the concentration at which the sweetness is perceptible as sweet in the edible compositions. The sweetness threshold level varies for different edible compositions (*e.g.,* in different matrices), and may be varied with respect to the individual perceiving the sweetness.

In all embodiments of the present disclosure, the sweet taste modulator(s) of the present disclosure is a different compound from than the sweetener of the composition. Accordingly, although an ingredient may be characterized as both a sweet taste modulator and a sweetener, in all embodiments of the disclosure, the sweet taste modulator and the sweetener are different ingredients, *i.e.,* the sweet taste modulator and the sweetener are not the same compound.

Each embodiment of the disclosure described herein may be taken alone or in combination with one or more other embodiments of the disclosure.

The present disclosure provides neoflavonoid compounds for modulating or potentiating the sweet taste of a sweetener. The neoflavonoid compounds of this disclosure are capable of modulating or potentiating the sweet taste of a sweetener. The neoflavonoid compound may have a molecular weight less than about 1000, about 500, or about 300 Daltons. In certain embodiments, the neoflavonoid compound is a compound of Formula (I): 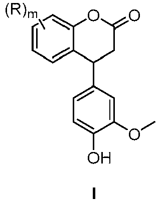 wherein R is -OH, C₁-C₄ alkyl, -CO₂H, acyl or formyl; m is 2 or 3; and at least one R is not OH; or a comestibly acceptable salt, derivative, enantiomer or solvate thereof or a combination of any of the foregoing compounds.

In some embodiments of Formula (I) the Compound is:

| | |
|---|---|
| Compound 1 | 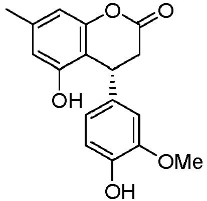 |
| | (R)5-hydroxy-4-(4-hydroxy-3-methoxyphenyl)-7-methylchroman-2-one, |

or a comestibly or biologically acceptable salt or solvate thereof.

The term "comestibly or biologically acceptable derivative" refers to any comestibly or biologically acceptable derivative of a sweet taste modulator of the present disclosure, which, upon ingestion, is capable of providing (directly or indirectly) a sweet taste modulator or bitter blocker of the present disclosure, or a polymorph, metabolite, residue or portion thereof, or a combination of sweet taste modulators, characterized by the ability to enhance the perception of a sweet taste attributed to a sweetener or block the perception of bitter taste attributed to a bitter tastant. A "comestible product" is a product suitable for oral use, such as eating or drinking. Therefore, a comestibly acceptable compound is an edible compound.

Sweet taste modulators synergize with sweeteners to enhance or potentiate the perception of sweet taste due to the sweetener. When sweet taste modulators are used above their sweetness threshold level, they synergize with sweeteners to enhance or potentiate the perception of sweet taste due to the sweetener. In such cases, the overall sweetness of a composition comprising a sweet taste modulator, or a combination of sweet taste modulators, and a sweet compound is higher than the mere addition of inherent sweetness due to each of the sweet taste modulators, or the combination of sweet taste modulators, and a sweet compound. For example, if a sweet taste modulator with a sweetness equivalent to 1% sucrose is added to a 5% sucrose solution, the perceived sweetness of the resulting composition would be greater than that of a 6% sucrose solution -with any perceived sweetness greater than a 6% sucrose solution being attributable to the sweetness enhancing properties of the sweet taste modulators. Such an increase in perceived sweetness may be referred to as synergistic, not additive.

The terms "sweetness threshold," and "sweetness recognition threshold," are used interchangeably, herein, and refer to the level at which the lowest known concentration of a certain sweet compound is perceivable as sweet by the human sense of taste. This sweetness recognition threshold also encompasses the sweetness detection threshold, referring to the level at which the lowest known concentration of a certain sweet compound is perceivable by the human sense of taste. The sweetness threshold can vary from person to person. The sweetness threshold can also vary from matrix to matrix (*e.g.,* different sweetness thresholds in water and a carbonated beverage). For example, a sweetness threshold level for sucrose in water could be around 1% or 1.5%. In some embodiments, the sweetness enhancers of Formula (I), Compound 1, or a comestibly or biologically acceptable salt, solvate, or enantiomer thereof, or a combination of any of the foregoing compounds, are used at concentrations below their sweetness threshold.

The terms "effective concentration" and "effective amount" are used interchangeably herein and refer to an amount sufficient to produce a desired property or result. For example, an effective amount of a sweet taste modulator, or a combination of sweet taste modulators, of the present disclosure is an amount capable of modulating (*e.g.,* enhancing) the perception of sweet taste associated with a sweetener. The term "effective amount" of a sweet taste modulator, or a combination of sweet taste modulators, of the disclosure also refers to an amount which, when added to an edible composition, enhances the sweet taste of, *e.g.,* a sugar, thereby allowing for the maintenance of the perception of a desired sweet flavor of the edible composition. The term "effective amount" also refers to the amount of a sweet taste modulator, or a combination of sweet taste modulators, of the present disclosure capable of modulating (*e.g.,* enhancing) the perception of a sweet taste associated with either a sweetener in a food product or an inherently sweet food product. The sweet taste modulators, or the combination of sweet taste modulators, of the present disclosure may impart a sweetness or taste at certain concentrations and no perceptible sweetness or taste at other concentrations. For example, the sweet taste modulator, or the combination of sweet taste modulators, may be present in an amount such that the taste, such as sweetness, of the sweet taste modulator, or the combination of sweet taste modulators, is imperceptible. The compositions discussed herein include an effective amount of the sweet taste modulator, or the combination of sweet taste modulators. An effective amount of the sweet taste modulator, or the combination of sweet taste modulators, includes an amount sufficient to enhance the perception of sweetness intensity of a sweetener.

In general, a sweet taste modulator, or a combination of sweet taste modulators, of the present disclosure (*e.g.,* a compound of Formula (I), or Compound 1, or a comestibly or biologically acceptable salt, solvate, or enantiomer thereof, or a combination of any of the foregoing compounds) enhances the sweetness of a sweetener when the compound is present at a concentration between about 0.001 ppm and about 1000 ppm. In some embodiments, the sweet taste modulator, or the combination of sweet taste modulators, of the present disclosure (*e.g.,* a compound of Formula (I), or Compound 1, or a comestibly or biologically acceptable salt, solvate, or enantiomer thereof, or a combination of any of the foregoing compounds) enhances the sweetness of a sweetener when the compound is present at a concentration between about 0.005 ppm to about 500 ppm; about 0.01 ppm to about 100 ppm; about 0.05 ppm to about 50 ppm; about 0.1 ppm to about 5 ppm; about 0.1 ppm to about 10 ppm; about 0.1 ppm to about 100 ppm; about 1 ppm to about 10 ppm; about 1 ppm to about 30 ppm; about 1 ppm to about 50 ppm; about 10 ppm to about 20 ppm; about 10 ppm to about 25 ppm; about 10 ppm to about 30 ppm; about 1 ppm to about 50 ppm; or about 5 ppm to about 50 ppm; about 10 ppm to about 50 ppm; about 30 ppm to about 50 ppm; about 1 ppm to about 60 ppm; about 100 ppm to about 300 ppm; about 100 ppm to about 1000 ppm; or about 30 ppm to about 3000 ppm. In some embodiments, the sweet taste modulator, or the combination of sweet taste modulators, of the present disclosure (*e.g.,* a compound of Formula (I), or Compound 1, or a comestibly or biologically acceptable salt, solvate, or enantiomer thereof, or a combination of any of the foregoing compounds) enhances the sweetness of a sweetener when the compound, or the combination of sweet taste modulators, is present at a concentration between about 50 ppm to about 100 ppm; about 100 ppm to about 200 ppm; about 200 ppm to about 300 ppm; about 300 ppm to about 400 ppm; about 400 ppm to about 500 ppm; about 500 ppm to about 600 ppm; about 600 ppm to about 700 ppm; about 700 ppm to about 800 ppm; about 800 ppm to about 900 ppm; about 900 ppm to about 1000 ppm; about 1000 ppm to about 1100 ppm; about 1100 ppm to about 1200 ppm; about 1200 ppm to about 1300 ppm; about 1300 ppm to about 1400 ppm; about 1400 ppm to about 1500 ppm; about 1500 ppm to about 1600 ppm; about 1600 ppm to about 1700 ppm; about 1700 ppm to about 1800 ppm; about 1800 ppm to about 1900 ppm; about 1900 ppm to about 2000 ppm; about 2000 ppm to about 2100 ppm; about 2100 ppm to about 2200 ppm; about 2200 ppm to about 2300 ppm; about 2300 ppm to about 2400 ppm; about 2400 ppm to about 2500 ppm; about 2500 ppm to about 2600 ppm; about 2600 ppm to about 2700 ppm; about 2700 ppm to about 2800 ppm; about 2800 ppm to about 2900 ppm, or about 2900 ppm to about 3000 ppm.
In yet other embodiments, the sweet taste modulator, or the combination of sweet taste modulators, of the present disclosure enhances the sweetness of a sweetener when the compound, or the combination of compounds, is present at a concentration of about 0.1 ppm to about 30 ppm; about 1 ppm to about 30 ppm; or about 1 ppm to about 50 ppm. In additional embodiments, the sweet taste modulator, or the combination of sweet taste modulators, of the present disclosure (*e.g.,* a compound of Formula (I), or Compound 1, or a comestibly or biologically acceptable salt, solvate, or enantiomer thereof, or a combination of any of the foregoing compounds) enhances the sweetness of a sweetener when the compound, or the combination of compounds, is present at a concentration of about 0.1 ppm to about 5 ppm; about 0.1 ppm to about 4 ppm; about 0.1 ppm to about 3 ppm; about 0.1 ppm to about 2 ppm; about 0.1 ppm to about 1 ppm; about 0.5 ppm to about 5 ppm; about 0.5 ppm to 4 about ppm; about 0.5 ppm to about 3 ppm; about 0.5 ppm to about 2 ppm; about 0.5 ppm to about 1.5 ppm; about 0.5 ppm to about 1 ppm; about 5 ppm to about 15 ppm; about 6 ppm to about 14 ppm; about 7 ppm to about 13 ppm; about 8 ppm to about 12 ppm; about 9 ppm to about 11 ppm; about 25 ppm to about 35 ppm; about 26 ppm to about 34 ppm; about 27 ppm to about 33 ppm; about 28 ppm to about 32 ppm; or about 29 ppm to about 31 ppm.

In yet other embodiments, the sweet taste modulator, or the combination of sweet taste modulators, of the present disclosure (*e.g.,* a compound of Formula (I), or Compound 1, or a comestibly or biologically acceptable salt, solvate, or enantiomer thereof, or a combination of any of the foregoing compounds) enhances the sweetness of a sweetener when the compound, or the combination of compounds, is present at a concentration of about 0.1 ppm, about 0.5 ppm, about 1 ppm, about 2 ppm, about 3 ppm, about 4 ppm, about 5 ppm, about 6 ppm, about 7 ppm, about 8 ppm, about 9 ppm, about 10 ppm, about 11 ppm, about 12 ppm, about 13 ppm, about 14 ppm, about 15 ppm, about 16 ppm, about 17 ppm, about 18 ppm, about 19 ppm, about 20 ppm, about 21 ppm, about 22 ppm, about 23 ppm, about 24 ppm, about 25 ppm, about 26 ppm, about 27 ppm, about 28 ppm about, 29 ppm, about 30 ppm, about 31 ppm, about 32 ppm, about 33 ppm, about 34 ppm, about 35 ppm, about 36 ppm, about 37 ppm, about 38 ppm, about 39 ppm, about 40 ppm, about 41 ppm, about 42 ppm, about 43 ppm, about 44 ppm, about 45 ppm, about 46 ppm, about 47 ppm, about 48 ppm, about 49 ppm, about 50 ppm, about 51 ppm, about 52 ppm, about 53 ppm, about 54 ppm, about 55 ppm, about 56 ppm, about 57 ppm, about 58 ppm, about 59 ppm, about 60 ppm, about 61 ppm, about 62 ppm, about 63 ppm, about 64 ppm, about 65 ppm, about 66 ppm, about 67 ppm, about 68 ppm, about 69 ppm, about 70 ppm, about 71 ppm, about 72 ppm, about 73 ppm, about 74 ppm, about 75 ppm, about 76 ppm, about 77 ppm, about 78 ppm, about 79 ppm, about 80 ppm, about 81 ppm, about 82 ppm, about 83 ppm, about 84 ppm, about 85 ppm, about 86 ppm, about 87 ppm, about 88 ppm, about 89 ppm, about 90 ppm, about 91 ppm, about 92 ppm, about 93 ppm, about 94 ppm, about 95 ppm, about 96 ppm, about 97 ppm, about 98 ppm, about 99 ppm, about 100 ppm, about 101 ppm, about 102 ppm, about 103 ppm, about 104 ppm, about 105 ppm, about 106 ppm, about 107 ppm, about 108 ppm, about 109 ppm, about 110 ppm, about 111 ppm, about 112 ppm, about 113 ppm, about 114 ppm, about 115 ppm, about 116 ppm, about 117 ppm, about 118 ppm, about 119 ppm, about 120 ppm, about 121 ppm, about 122 ppm, about 123 ppm, about 124 ppm, about 125 ppm, about 126 ppm, about 127 ppm, about 128 ppm, about 129 ppm, about 130 ppm, about 131 ppm, about 132 ppm, about 133 ppm, about 134 ppm, about 135 ppm, about 136 ppm, about 137 ppm, about 138 ppm, about 139 ppm, about 140 ppm, about 141 ppm, about 142 ppm, about 143 ppm, about 144 ppm, about 145 ppm, about 146 ppm, about 147 ppm, about 148 ppm, about 149 ppm, or about 150 ppm. In some embodiments, the compound, or the combination of compounds, is present at a concentration of about 0.1 ppm. In some embodiments, the compound, or the combination of compounds, is present at a concentration of about 0.5 ppm. In some embodiments, the compound, or the combination of compounds, is present at a concentration of about 1 ppm. In some embodiments, the compound, or the combination of compounds, is present at a concentration of about 2 ppm. In some embodiments, the compound, or the combination of compounds, is present at a concentration of about 3 ppm. In some embodiments, the compound, or the combination of compounds, is present at a concentration of about 4 ppm. In some embodiments, the compound, or the combination of compounds, is present at a concentration of about 5 ppm. In some embodiments, the compound, or the combination of compounds, is present at a concentration of about 6 ppm. In some embodiments, the compound, or the combination of compounds, is present at a concentration of about 7 ppm. In some embodiments, the compound, or the combination of compounds, is present at a concentration of about 8 ppm. In some embodiments, the compound, or the combination of compounds, is present at a concentration of about 9 ppm. In some embodiments, the compound, or the combination of compounds, is present at a concentration of about 10 ppm. In some embodiments, the compound, or the combination of compounds, is present at a concentration of about 11 ppm. In some embodiments, the compound, or the combination of compounds, is present at a concentration of about 12 ppm. In some embodiments, the compound, or the combination of compounds, is present at a concentration of about 13 ppm. In some embodiments, the compound, or the combination of compounds, is present at a concentration of about 14 ppm. In some embodiments, the compound, or the combination of compounds, is present at a concentration of about 15 ppm. In some embodiments, the compound, or the combination of compounds, is present at a concentration of about 16 ppm. In some embodiments, the compound, or the combination of compounds, is present at a concentration of about 17 ppm. In some embodiments, the compound, or the combination of compounds, is present at a concentration of about 18 ppm. In some embodiments, the compound, or the combination of compounds, is present at a concentration of about 19 ppm. In some embodiments, the compound, or the combination of compounds, is present at a concentration of about 20 ppm. In some embodiments, the compound, or the combination of compounds, is present at a concentration of about 21 ppm. In some embodiments, the compound, or the combination of compounds, is present at a concentration of about 22 ppm. In some embodiments, the compound, or the combination of compounds, is present at a concentration of about 23 ppm. In some embodiments, the compound, or the combination of compounds, is present at a concentration of about 24 ppm. In some embodiments, the compound, or the combination of compounds, is present at a concentration of about 25 ppm. In some embodiments, the compound, or the combination of compounds, is present at a concentration of about 26 ppm. In some embodiments, the compound, or the combination of compounds, is present at a concentration of about 27 ppm. In some embodiments, the compound, or the combination of compounds, is present at a concentration of about 28 ppm. In some embodiments, the compound, or the combination of compounds, is present at a concentration of about 29 ppm. In some embodiments, the compound, or the combination of compounds, is present at a concentration of about 30 ppm. In some embodiments, the compound, or the combination of compounds, is present at a concentration of about 31 ppm. In some embodiments, the compound, or the combination of compounds, is present at a concentration of about 32 ppm. In some embodiments, the compound, or the combination of compounds, is present at a concentration of about 33 ppm. In some embodiments, the compound, or the combination of compounds, is present at a concentration of about 34 ppm. In some embodiments, the compound, or the combination of compounds, is present at a concentration of about 35 ppm. In some embodiments, the compound, or the combination of compounds, is present at a concentration of about 36 ppm. In some embodiments, the compound, or the combination of compounds, is present at a concentration of about 37 ppm. In some embodiments, the compound, or the combination of compounds, is present at a concentration of about 38 ppm. In some embodiments, the compound, or the combination of compounds, is present at a concentration of about 39 ppm. In some embodiments, the compound, or the combination of compounds, is present at a concentration of about 40 ppm. In some embodiments, the compound, or the combination of compounds, is present at a concentration of about 41 ppm. In some embodiments, the compound, or the combination of compounds, is present at a concentration of about 42 ppm. In some embodiments, the compound, or the combination of compounds, is present at a concentration of about 43 ppm. In some embodiments, the compound, or the combination of compounds, is present at a concentration of about 44 ppm. In some embodiments, the compound, or the combination of compounds, is present at a concentration of about 45 ppm. In some embodiments, the compound, or the combination of compounds, is present at a concentration of about 46 ppm. In some embodiments, the compound, or the combination of compounds, is present at a concentration of about 47 ppm. In some embodiments, the compound, or the combination of compounds, is present at a concentration of about 48 ppm. In some embodiments, the compound, or the combination of compounds, is present at a concentration of about 49 ppm. In some embodiments, the compound, or the combination of compounds, is present at a concentration of about 50 ppm. In some embodiments, the compound, or the combination of compounds, is present at a concentration of about 51 ppm. In some embodiments, the compound, or the combination of compounds, is present at a concentration of about 52 ppm. In some embodiments, the compound, or the combination of compounds, is present at a concentration of about 53 ppm. In some embodiments, the compound, or the combination of compounds, is present at a concentration of about 54 ppm. In some embodiments, the compound, or the combination of compounds, is present at a concentration of about 55 ppm. In some embodiments, the compound, or the combination of compounds, is present at a concentration of about 56 ppm. In some embodiments, the compound, or the combination of compounds, is present at a concentration of about 57 ppm. In some embodiments, the compound, or the combination of compounds, is present at a concentration of about 58 ppm. In some embodiments, the compound, or the combination of compounds, is present at a concentration of about 59 ppm. In some embodiments, the compound, or the combination of compounds, is present at a concentration of about 60 ppm. In some embodiments, the compound, or the combination of compounds, is present at a concentration of about 61 ppm. In some embodiments, the compound, or the combination of compounds, is present at a concentration of about 62 ppm. In some embodiments, the compound, or the combination of compounds, is present at a concentration of about 63 ppm. In some embodiments, the compound, or the combination of compounds, is present at a concentration of about 64 ppm. In some embodiments, the compound, or the combination of compounds, is present at a concentration of about 65 ppm. In some embodiments, the compound, or the combination of compounds, is present at a concentration of about 66 ppm. In some embodiments, the compound, or the combination of compounds, is present at a concentration of about 67 ppm. In some embodiments, the compound, or the combination of compounds, is present at a concentration of about 68 ppm. In some embodiments, the compound, or the combination of compounds, is present at a concentration of about 69 ppm. In some embodiments, the compound, or the combination of compounds, is present at a concentration of about 70 ppm. In some embodiments, the compound, or the combination of compounds, is present at a concentration of about 71 ppm. In some embodiments, the compound, or the combination of compounds, is present at a concentration of about 72 ppm. In some embodiments, the compound, or the combination of compounds, is present at a concentration of about 73 ppm. In some embodiments, the compound, or the combination of compounds, is present at a concentration of about 74 ppm. In some embodiments, the compound, or the combination of compounds, is present at a concentration of about 75 ppm. In some embodiments, the compound, or the combination of compounds, is present at a concentration of about 76 ppm. In some embodiments, the compound, or the combination of compounds, is present at a concentration of about 77 ppm. In some embodiments, the compound, or the combination of compounds, is present at a concentration of about 78 ppm. In some embodiments, the compound, or the combination of compounds, is present at a concentration of about 79 ppm. In some embodiments, the compound, or the combination of compounds, is present at a concentration of about 80 ppm. In some embodiments, the compound, or the combination of compounds, is present at a concentration of about 81 ppm. In some embodiments, the compound, or the combination of compounds, is present at a concentration of about 82 ppm. In some embodiments, the compound, or the combination of compounds, is present at a concentration of about 83 ppm. In some embodiments, the compound, or the combination of compounds, is present at a concentration of about 84 ppm. In some embodiments, the compound, or the combination of compounds, is present at a concentration of about 85 ppm. In some embodiments, the compound, or the combination of compounds, is present at a concentration of about 86 ppm. In some embodiments, the compound, or the combination of compounds, is present at a concentration of about 87 ppm. In some embodiments, the compound, or the combination of compounds, is present at a concentration of about 88 ppm. In some embodiments, the compound, or the combination of compounds, is present at a concentration of about 89 ppm. In some embodiments, the compound, or the combination of compounds, is present at a concentration of about 90 ppm. In some embodiments, the compound, or the combination of compounds, is present at a concentration of about 91 ppm. In some embodiments, the compound, or the combination of compounds, is present at a concentration of about 92 ppm. In some embodiments, the compound, or the combination of compounds, is present at a concentration of about 93 ppm. In some embodiments, the compound, or the combination of compounds, is present at a concentration of about 94 ppm. In some embodiments, the compound, or the combination of compounds, is present at a concentration of about 95 ppm. In some embodiments, the compound, or the combination of compounds, is present at a concentration of about 96 ppm. In some embodiments, the compound, or the combination of compounds, is present at a concentration of about 97 ppm. In some embodiments, the compound, or the combination of compounds, is present at a concentration of about 98 ppm. In some embodiments, the compound, or the combination of compounds, is present at a concentration of about 99 ppm. In some embodiments, the compound, or the combination of compounds, is present at a concentration of about 100 ppm. In some embodiments, the compound, or the combination of compounds, is present at a concentration of about 101 ppm. In some embodiments, the compound, or the combination of compounds, is present at a concentration of about 102 ppm. In some embodiments, the compound, or the combination of compounds, is present at a concentration of about 103 ppm. In some embodiments, the compound, or the combination of compounds, is present at a concentration of about 104 ppm. In some embodiments, the compound, or the combination of compounds, is present at a concentration of about 105 ppm. In some embodiments, the compound, or the combination of compounds, is present at a concentration of about 106 ppm. In some embodiments, the compound, or the combination of compounds, is present at a concentration of about 107 ppm. In some embodiments, the compound, or the combination of compounds, is present at a concentration of about 108 ppm. In some embodiments, the compound, or the combination of compounds, is present at a concentration of about 109 ppm. In some embodiments, the compound, or the combination of compounds, is present at a concentration of about 110 ppm. In some embodiments, the compound, or the combination of compounds, is present at a concentration of about 111 ppm. In some embodiments, the compound, or the combination of compounds, is present at a concentration of about 112 ppm. In some embodiments, the compound, or the combination of compounds, is present at a concentration of about 113 ppm. In some embodiments, the compound, or the combination of compounds, is present at a concentration of about 114 ppm. In some embodiments, the compound, or the combination of compounds, is present at a concentration of about 115 ppm. In some embodiments, the compound, or the combination of compounds, is present at a concentration of about 116 ppm. In some embodiments, the compound, or the combination of compounds, is present at a concentration of about 117 ppm. In some embodiments, the compound, or the combination of compounds, is present at a concentration of about 118 ppm. In some embodiments, the compound, or the combination of compounds, is present at a concentration of about 119 ppm. In some embodiments, the compound, or the combination of compounds, is present at a concentration of about 120 ppm. In some embodiments, the compound, or the combination of compounds, is present at a concentration of about 121 ppm. In some embodiments, the compound, or the combination of compounds, is present at a concentration of about 122 ppm. In some embodiments, the compound, or the combination of compounds, is present at a concentration of about 123 ppm. In some embodiments, the compound, or the combination of compounds, is present at a concentration of about 124 ppm. In some embodiments, the compound, or the combination of compounds, is present at a concentration of about 125 ppm. In some embodiments, the compound, or the combination of compounds, is present at a concentration of about 126 ppm. In some embodiments, the compound, or the combination of compounds, is present at a concentration of about 127 ppm. In some embodiments, the compound, or the combination of compounds, is present at a concentration of about 128 ppm. In some embodiments, the compound, or the combination of compounds, is present at a concentration of about 129 ppm. In some embodiments, the compound, or the combination of compounds, is present at a concentration of about 130 ppm. In some embodiments, the compound, or the combination of compounds, is present at a concentration of about 131 ppm. In some embodiments, the compound, or the combination of compounds, is present at a concentration of about 132 ppm. In some embodiments, the compound, or the combination of compounds, is present at a concentration of about 133 ppm. In some embodiments, the compound, or the combination of compounds, is present at a concentration of about 134 ppm. In some embodiments, the compound, or the combination of compounds, is present at a concentration of about 135 ppm. In some embodiments, the compound, or the combination of compounds, is present at a concentration of about 136 ppm. In some embodiments, the compound, or the combination of compounds, is present at a concentration of about 137 ppm. In some embodiments, the compound, or the combination of compounds, is present at a concentration of about 138 ppm. In some embodiments, the compound, or the combination of compounds, is present at a concentration of about 139 ppm. In some embodiments, the compound, or the combination of compounds, is present at a concentration of about 140 ppm. In some embodiments, the compound, or the combination of compounds, is present at a concentration of about 141 ppm. In some embodiments, the compound, or the combination of compounds, is present at a concentration of about 142 ppm. In some embodiments, the compound, or the combination of compounds, is present at a concentration of about 143 ppm. In some embodiments, the compound, or the combination of compounds, is present at a concentration of about 144 ppm. In some embodiments, the compound, or the combination of compounds, is present at a concentration of about 145 ppm. In some embodiments, the compound, or the combination of compounds, is present at a concentration of about 146 ppm. In some embodiments, the compound, or the combination of compounds, is present at a concentration of about 147 ppm. In some embodiments, the compound, or the combination of compounds, is present at a concentration of about 148 ppm. In some embodiments, the compound, or the combination of compounds, is present at a concentration of about 149 ppm. In some embodiments, the compound, or the combination of compounds, is present at a concentration of about 150 ppm.

The sweet taste modulator, or the combination of sweet taste modulators, of the present disclosure (*e.g.,* a compound of Formula (I), or Compound 1, or a comestibly or biologically acceptable salt, solvate, or enantiomer thereof, or a combination of any of the foregoing compounds) may enhance the sweetness of a sweetener when the compound, or the combination of compounds, is present at a concentration of 0.1 ppm, 0.5 ppm, 1 ppm, 2 ppm, 3 ppm, 4 ppm, 5 ppm, 6 ppm, 7 ppm, 8 ppm, 9 ppm, 10 ppm, 11 ppm, 12 ppm, 13 ppm, 14 ppm, 15 ppm, 16 ppm, 17 ppm, 18 ppm, 19 ppm, 20 ppm, 21 ppm, 22 ppm, 23 ppm, 24 ppm, 25 ppm, 26 ppm, 27 ppm, 28 ppm, 29 ppm, 30 ppm, 31 ppm, 32 ppm, 33 ppm, 34 ppm, 35 ppm, 36 ppm, 37 ppm, 38 ppm, 39 ppm, 40 ppm, 41 ppm, 42 ppm, 43 ppm, 44 ppm, 45 ppm, 46 ppm, 47 ppm, 48 ppm, 49 ppm, 50 ppm , 51 ppm, 52 ppm, 53 ppm, 54 ppm, 55 ppm, 56 ppm, 57 ppm, 58 ppm, 59 ppm, 60 ppm, 61 ppm, 62 ppm, 63 ppm, 64 ppm, 65 ppm, 66 ppm, 67 ppm, 68 ppm, 69 ppm, 70 ppm, 71 ppm, 72 ppm, 73 ppm, 74 ppm, 75 ppm, 76 ppm, 77 ppm, 78 ppm, 79 ppm, 80 ppm, 81 ppm, 82 ppm, 83 ppm, 84 ppm, 85 ppm, 86 ppm, 87 ppm, 88 ppm, 89 ppm, 90 ppm, 91 ppm, 92 ppm, 93 ppm, 94 ppm, 95 ppm, 96 ppm, 97 ppm, 98 ppm, 99 ppm, 100 ppm, 101 ppm, 102 ppm, 103 ppm, 104 ppm, 105 ppm, 106 ppm, 107 ppm, 108 ppm, 109 ppm, 110 ppm, 111 ppm, 112 ppm, 113 ppm, 114 ppm, 115 ppm, 116 ppm, 117 ppm, 118 ppm, 119 ppm, 120 ppm, 121 ppm, 122 ppm, 123 ppm, 124 ppm, 125 ppm, 126 ppm, 127 ppm, 128 ppm, 129 ppm, 130 ppm, 131 ppm, 132 ppm, 133 ppm, 134 ppm, 135 ppm, 136 ppm, 137 ppm, 138 ppm, 139 ppm, 140 ppm, 141 ppm, 142 ppm, 143 ppm, 144 ppm, 145 ppm, 146 ppm, 147 ppm, 148 ppm, 149 ppm, or 150 ppm. In some embodiments, the compound, or the combination of compounds, is present at a concentration of 0.1 ppm. In some embodiments, the compound, or the combination of compounds, is present at a concentration of 0.5 ppm. In some embodiments, the compound, or the combination of compounds, is present at a concentration of 1 ppm. In some embodiments, the compound, or the combination of compounds, is present at a concentration of 2 ppm. In some embodiments, the compound, or the combination of compounds, is present at a concentration of 3 ppm. In some embodiments, the compound, or the combination of compounds, is present at a concentration of 4 ppm. In some embodiments, the compound, or the combination of compounds, is present at a concentration of 5 ppm. In some embodiments, the compound, or the combination of compounds, is present at a concentration of 6 ppm. In some embodiments, the compound, or the combination of compounds, is present at a concentration of 7 ppm. In some embodiments, the compound, or the combination of compounds, is present at a concentration of 8 ppm. In some embodiments, the compound, or the combination of compounds, is present at a concentration of 9 ppm. In some embodiments, the compound, or the combination of compounds, is present at a concentration of 10 ppm. In some embodiments, the compound, or the combination of compounds, is present at a concentration of 11 ppm. In some embodiments, the compound, or the combination of compounds, is present at a concentration of 12 ppm. In some embodiments, the compound, or the combination of compounds, is present at a concentration of 13 ppm. In some embodiments, the compound, or the combination of compounds, is present at a concentration of 14 ppm. In some embodiments, the compound, or the combination of compounds, is present at a concentration of 15 ppm. In some embodiments, the compound, or the combination of compounds, is present at a concentration of 16 ppm. In some embodiments, the compound, or the combination of compounds, is present at a concentration of 17 ppm. In some embodiments, the compound, or the combination of compounds, is present at a concentration of 18 ppm. In some embodiments, the compound, or the combination of compounds, is present at a concentration of 19 ppm. In some embodiments, the compound, or the combination of compounds, is present at a concentration of 20 ppm. In some embodiments, the compound, or the combination of compounds, is present at a concentration of 21 ppm. In some embodiments, the compound, or the combination of compounds, is present at a concentration of 22 ppm. In some embodiments, the compound, or the combination of compounds, is present at a concentration of 23 ppm. In some embodiments, the compound, or the combination of compounds, is present at a concentration of 24 ppm. In some embodiments, the compound, or the combination of compounds, is present at a concentration of 25 ppm. In some embodiments, the compound, or the combination of compounds, is present at a concentration of 26 ppm. In some embodiments, the compound, or the combination of compounds, is present at a concentration of 27 ppm. In some embodiments, the compound, or the combination of compounds, is present at a concentration of 28 ppm. In some embodiments, the compound, or the combination of compounds, is present at a concentration of 29 ppm. In some embodiments, the compound, or the combination of compounds, is present at a concentration of 30 ppm. In some embodiments, the compound, or the combination of compounds, is present at a concentration of 31 ppm. In some embodiments, the compound, or the combination of compounds, is present at a concentration of 32 ppm. In some embodiments, the compound, or the combination of compounds, is present at a concentration of 33 ppm. In some embodiments, the compound, or the combination of compounds, is present at a concentration of 34 ppm. In some embodiments, the compound, or the combination of compounds, is present at a concentration of 35 ppm. In some embodiments, the compound, or the combination of compounds, is present at a concentration of 36 ppm. In some embodiments, the compound, or the combination of compounds, is present at a concentration of 37 ppm. In some embodiments, the compound, or the combination of compounds, is present at a concentration of 38 ppm. In some embodiments, the compound, or the combination of compounds, is present at a concentration of 39 ppm. In some embodiments, the compound, or the combination of compounds, is present at a concentration of 40 ppm. In some embodiments, the compound, or the combination of compounds, is present at a concentration of 41 ppm. In some embodiments, the compound, or the combination of compounds, is present at a concentration of 42 ppm. In some embodiments, the compound, or the combination of compounds, is present at a concentration of 43 ppm. In some embodiments, the compound, or the combination of compounds, is present at a concentration of 44 ppm. In some embodiments, the compound, or the combination of compounds, is present at a concentration of 45 ppm. In some embodiments, the compound, or the combination of compounds, is present at a concentration of 46 ppm. In some embodiments, the compound, or the combination of compounds, is present at a concentration of 47 ppm. In some embodiments, the compound, or the combination of compounds, is present at a concentration of 48 ppm. In some embodiments, the compound, or the combination of compounds, is present at a concentration of 49 ppm. In some embodiments, the compound, or the combination of compounds, is present at a concentration of 50 ppm. In some embodiments, the compound, or the combination of compounds, is present at a concentration of 51 ppm. In some embodiments, the compound, or the combination of compounds, is present at a concentration of 52 ppm. In some embodiments, the compound, or the combination of compounds, is present at a concentration of 53 ppm. In some embodiments, the compound, or the combination of compounds, is present at a concentration of 54 ppm. In some embodiments, the compound, or the combination of compounds, is present at a concentration of 55 ppm. In some embodiments, the compound, or the combination of compounds, is present at a concentration of 56 ppm. In some embodiments, the compound, or the combination of compounds, is present at a concentration of 57 ppm. In some embodiments, the compound, or the combination of compounds, is present at a concentration of 58 ppm. In some embodiments, the compound, or the combination of compounds, is present at a concentration of 59 ppm. In some embodiments, the compound, or the combination of compounds, is present at a concentration of 60 ppm. In some embodiments, the compound, or the combination of compounds, is present at a concentration of 61 ppm. In some embodiments, the compound, or the combination of compounds, is present at a concentration of 62 ppm. In some embodiments, the compound, or the combination of compounds, is present at a concentration of 63 ppm. In some embodiments, the compound, or the combination of compounds, is present at a concentration of 64 ppm. In some embodiments, the compound, or the combination of compounds, is present at a concentration of 65 ppm. In some embodiments, the compound, or the combination of compounds, is present at a concentration of 66 ppm. In some embodiments, the compound, or the combination of compounds, is present at a concentration of 67 ppm. In some embodiments, the compound, or the combination of compounds, is present at a concentration of 68 ppm. In some embodiments, the compound, or the combination of compounds, is present at a concentration of 69 ppm. In some embodiments, the compound, or the combination of compounds, is present at a concentration of 70 ppm. In some embodiments, the compound, or the combination of compounds, is present at a concentration of 71 ppm. In some embodiments, the compound, or the combination of compounds, is present at a concentration of 72 ppm. In some embodiments, the compound, or the combination of compounds, is present at a concentration of 73 ppm. In some embodiments, the compound, or the combination of compounds, is present at a concentration of 74 ppm. In some embodiments, the compound, or the combination of compounds, is present at a concentration of 75 ppm. In some embodiments, the compound, or the combination of compounds, is present at a concentration of 76 ppm. In some embodiments, the compound, or the combination of compounds, is present at a concentration of 77 ppm. In some embodiments, the compound, or the combination of compounds, is present at a concentration of 78 ppm. In some embodiments, the compound, or the combination of compounds, is present at a concentration of 79 ppm. In some embodiments, the compound, or the combination of compounds, is present at a concentration of 80 ppm. In some embodiments, the compound, or the combination of compounds, is present at a concentration of 81 ppm. In some embodiments, the compound, or the combination of compounds, is present at a concentration of 82 ppm. In some embodiments, the compound, or the combination of compounds, is present at a concentration of 83 ppm. In some embodiments, the compound, or the combination of compounds, is present at a concentration of 84 ppm. In some embodiments, the compound, or the combination of compounds, is present at a concentration of 85 ppm. In some embodiments, the compound, or the combination of compounds, is present at a concentration of 86 ppm. In some embodiments, the compound, or the combination of compounds, is present at a concentration of 87 ppm. In some embodiments, the compound, or the combination of compounds, is present at a concentration of 88 ppm. In some embodiments, the compound, or the combination of compounds, is present at a concentration of 89 ppm. In some embodiments, the compound, or the combination of compounds, is present at a concentration of 90 ppm. In some embodiments, the compound, or the combination of compounds, is present at a concentration of 91 ppm. In some embodiments, the compound, or the combination of compounds, is present at a concentration of 92 ppm. In some embodiments, the compound, or the combination of compounds, is present at a concentration of 93 ppm. In some embodiments, the compound, or the combination of compounds, is present at a concentration of 94 ppm. In some embodiments, the compound, or the combination of compounds, is present at a concentration of 95 ppm. In some embodiments, the compound, or the combination of compounds, is present at a concentration of 96 ppm. In some embodiments, the compound, or the combination of compounds, is present at a concentration of 97 ppm. In some embodiments, the compound, or the combination of compounds, is present at a concentration of 98 ppm. In some embodiments, the compound, or the combination of compounds, is present at a concentration of 99 ppm. In some embodiments, the compound, or the combination of compounds, is present at a concentration of 100 ppm. In some embodiments, the compound, or the combination of compounds, is present at a concentration of 101 ppm. In some embodiments, the compound, or the combination of compounds, is present at a concentration of 102 ppm. In some embodiments, the compound, or the combination of compounds, is present at a concentration of 103 ppm. In some embodiments, the compound, or the combination of compounds, is present at a concentration of 104 ppm. In some embodiments, the compound, or the combination of compounds, is present at a concentration of 105 ppm. In some embodiments, the compound, or the combination of compounds, is present at a concentration of 106 ppm. In some embodiments, the compound, or the combination of compounds, is present at a concentration of 107 ppm. In some embodiments, the compound is present at a concentration of 108 ppm. In some embodiments, the compound, or the combination of compounds, is present at a concentration of 109 ppm. In some embodiments, the compound, or the combination of compounds, is present at a concentration of 110 ppm. In some embodiments, the compound, or the combination of compounds, is present at a concentration of 111 ppm. In some embodiments, the compound, or the combination of compounds, is present at a concentration of 112 ppm. In some embodiments, the compound, or the combination of compounds, is present at a concentration of 113 ppm. In some embodiments, the compound, or the combination of compounds, is present at a concentration of 114 ppm. In some embodiments, the compound, or the combination of compounds, is present at a concentration of 115 ppm. In some embodiments, the compound, or the combination of compounds, is present at a concentration of 116 ppm. In some embodiments, the compound, or the combination of compounds, is present at a concentration of 117 ppm. In some embodiments, the compound, or the combination of compounds, is present at a concentration of 118 ppm. In some embodiments, the compound, or the combination of compounds, is present at a concentration of 119 ppm. In some embodiments, the compound, or the combination of compounds, is present at a concentration of 120 ppm. In some embodiments, the compound, or the combination of compounds, is present at a concentration of 121 ppm. In some embodiments, the compound, or the combination of compounds, is present at a concentration of 122 ppm. In some embodiments, the compound, or the combination of compounds, is present at a concentration of 123 ppm. In some embodiments, the compound, or the combination of compounds, is present at a concentration of 124 ppm. In some embodiments, the compound, or the combination of compounds, is present at a concentration of 125 ppm. In some embodiments, the compound, or the combination of compounds, is present at a concentration of 126 ppm. In some embodiments, the compound, or the combination of compounds, is present at a concentration of 127 ppm. In some embodiments, the compound, or the combination of compounds, is present at a concentration of 128 ppm. In some embodiments, the compound, or the combination of compounds, is present at a concentration of 129 ppm. In some embodiments, the compound, or the combination of compounds, is present at a concentration of 130 ppm. In some embodiments, the compound, or the combination of compounds, is present at a concentration of 131 ppm. In some embodiments, the compound, or the combination of compounds, is present at a concentration of 132 ppm. In some embodiments, the compound, or the combination of compounds, is present at a concentration of 133 ppm. In some embodiments, the compound, or the combination of compounds, is present at a concentration of 134 ppm. In some embodiments, the compound, or the combination of compounds, is present at a concentration of 135 ppm. In some embodiments, the compound, or the combination of compounds, is present at a concentration of 136 ppm. In some embodiments, the compound, or the combination of compounds, is present at a concentration of 137 ppm. In some embodiments, the compound, or the combination of compounds, is present at a concentration of 138 ppm. In some embodiments, the compound, or the combination of compounds, is present at a concentration of 139 ppm. In some embodiments, the compound, or the combination of compounds, is present at a concentration of 140 ppm. In some embodiments, the compound, or the combination of compounds, is present at a concentration of 141 ppm. In some embodiments, the compound, or the combination of compounds, is present at a concentration of 142 ppm. In some embodiments, the compound, or the combination of compounds, is present at a concentration of 143 ppm. In some embodiments, the compound, or the combination of compounds, is present at a concentration of 144 ppm. In some embodiments, the compound, or the combination of compounds, is present at a concentration of 145 ppm. In some embodiments, the compound, or the combination of compounds, is present at a concentration of 146 ppm. In some embodiments, the compound, or the combination of compounds, is present at a concentration of 147 ppm. In some embodiments, the compound, or the combination of compounds, is present at a concentration of 148 ppm. In some embodiments, the compound, or the combination of compounds, is present at a concentration of 149 ppm. In some embodiments, the compound, or the combination of compounds, is present at a concentration of 150 ppm.

In other embodiments, the sweet taste modulator of the present disclosure (*e.g.,* a compound of Formula (I), or Compound 1, or a comestibly or biologically acceptable salt, solvate, or enantiomer thereof, or a combination of any of the foregoing compounds) enhances the sweetness of a sweetener when the compound is present at a concentration of more than about 0.5 ppm, about 1 ppm, about 5 ppm, about 10 ppm, about 15 ppm, about 20 ppm, about 25 ppm, about 30 ppm, or about 35 ppm, up to, for example, about 35 ppm or about 50 ppm. In some embodiments, the compound, or the combination of compounds, is present at a concentration of more than 0.5 ppm. In some embodiments, the compound, or the combination of compounds, is present at a concentration of more than 1 ppm. In some embodiments, the compound, or the combination of compounds, is present at a concentration of more than 5 ppm. In some embodiments, the compound, or the combination of compounds, is present at a concentration of more than 10 ppm. In some embodiments, the compound, or the combination of compounds, is present at a concentration of more than 15 ppm. In some embodiments, the compound, or the combination of compounds, is present at a concentration of more than 20 ppm. In some embodiments, the compound, or the combination of compounds, is present at a concentration of more than 25 ppm. In some embodiments, the compound, or the combination of compounds, is present at a concentration of more than 30 ppm. In some embodiments, the compound, or the combination of compounds, is present at a concentration of more than 35 ppm. In some embodiments, the compound, or the combination of compounds, is present at a concentration of more than 50 ppm. In additional embodiments, the sweet taste modulator of the present disclosure (*e.g.,* a compound of Formula (I), or Compound 1, or a comestibly or biologically acceptable salt, solvate, or enantiomer thereof, or a combination of any of the foregoing compounds) enhances the sweetness of a sweetener when the compound, or the combination of compounds, is present at a concentration less than about 50 ppm, about 35 ppm, about 30 ppm, about 25 ppm, about 20 ppm, about 15 ppm, about 10 ppm, about 5 ppm, about 1 ppm, or about 0.5 ppm. In some embodiments, the compound, or the combination of compounds, is present at a concentration less than 50 ppm. In some embodiments, the compound, or the combination of compounds, is present at a concentration less than 35 ppm. In some embodiments, the compound, or the combination of compounds, is present at a concentration less than 30 ppm. In some embodiments, the compound, or the combination of compounds, is present at a concentration less than 25 ppm. In some embodiments, the compound, or the combination of compounds, is present at a concentration less than 20 ppm. In some embodiments, the compound, or the combination of compounds, is present at a concentration less than 15 ppm. In some embodiments, the compound, or the combination of compounds, is present at a concentration less than 10 ppm. In some embodiments, the compound, or the combination of compounds, is present at a concentration less than 5 ppm. In some embodiments, the compound, or the combination of compounds, is present at a concentration less than 1 ppm. In some embodiments, the compound, or the combination of compounds, is present at a concentration less than 0.5 ppm. In yet additional embodiments, the sweet taste modulator, or the combination of sweet taste modulators, of the present disclosure enhances the sweetness of a sweetener when the compound is present at a concentration less than about 35 ppm, about 10 ppm, or about 1 ppm.

The terms "parts per million" and "ppm" are used in the food industry to refer to a low concentration of a solution. For example, one gram of solute in 1000 mL of solvent has a concentration of 1000 ppm and one thousandth of a gram (0.001g) of solute in 1000 mL of solvent has a concentration of one ppm. Accordingly, a concentration of one milligram per liter (*i.e.,* 1 mg/L) is equal to 1 ppm. A concentration of 1 mg% is 1 mg/100mL. Accordingly, a concentration of 1 mg% is equal to 10 ppm.

The sweet taste modulators, or the combination of sweet taste modulators, of the disclosure may be combined with known naturally occurring and/or synthetic sweet taste modulators when used in embodiments (*e.g.,* edible compositions and methods) described herein.

### Sweeteners

In compositions and methods of the disclosure that comprise a sweetener, the sweetener can be of any type, for example a natural, non-natural, or synthetic sweetener. Non-limiting examples of such sweeteners include caloric carbohydrate sweeteners, natural carbohydrate sweeteners, non-natural carbohydrate sweeteners, natural high-potency sweeteners, non-natural high-potency sweeteners, synthetic high potency sweeteners, synthetic carbohydrate sweeteners, sugar alcohols, rare sugars and combinations thereof. In some embodiments, the at least one sweetener is chosen from caloric sweeteners. In another embodiment, the at least one sweetener is chosen from synthetic sweeteners. In another embodiment, the at least one sweetener is chosen from non-natural sweeteners. Non-limiting examples of rare sugars include D-Psicose, D-Turanose, D-allose, D-Tagatose, D-Sorbose, L-fructose, L-glucose, L-fructose, L-talose, L-ribose, and L-arabinose.

In some embodiments, the sweetener is a natural or inherent component of an edible composition. For example, the sweetener may be an inherent component of a food product or of a food stuff, such as fruit or a fruit product (*e.g.,* fruit sauce). Accordingly, the compounds, or the combination of compounds, of the present disclosure may be used in edible compositions to which no sweetener is added.

The terms "caloric sweeteners" and "caloric carbohydrate sweeteners," are used interchangeably herein, and refer to nutritive sweeteners that provide calories and include all caloric carbohydrate sweeteners, such as sugars and polyols. Non-limiting examples of suitable caloric carbohydrate sweeteners include sucrose, fructose, glucose, erythritol, maltitol, lactitol, sorbitol, mannitol, xylitol, D-tagatose, trehalose, galactose, rhamnose, cyclodextrin (*e.g*., α-cyclodextrin, β-cyclodextrin, and γ-cyclodextrin), ribulose, threose, arabinose, xylose, lyxose, allose, altrose, mannose, idose, lactose, maltose, invert sugar, isotrehalose, neotrehalose, palatinose or isomaltulose, erythrose, deoxyribose, gulose, idose, talose, erythrulose, xylulose, psicose, turanose, cellobiose, glucosamine, mannosamine, fucose, glucuronic acid, gluconic acid, glucono-lactone, abequose, galactosamine, xylo-oligosaccharides (xylotriose, xylobiose and the like), gentio-oligoscaccharides (gentiobiose, gentiotriose, gentiotetraose and the like), galacto-oligosaccharides, sorbose, nigero-oligosaccharides, fructooligosaccharides (kestose, nystose and the like), maltotetraol, maltotriol, malto-oligosaccharides (maltotriose, maltotetraose, maltopentaose, maltohexaose, maltoheptaose and the like), lactulose, melibiose, raffinose, rhamnose, ribose, isomerized liquid sugars such as high fructose corn/starch syrup (*e.g.,* HFCS55, HFCS42, or HFCS90), honey, maple syrup, coupling sugars, soybean oligosaccharides, and glucose syrup. Preferably, the sweetener is a natural sweetener chosen from glucose, fructose, sucrose, and mixtures thereof.

The term "polyol," as used herein, refers to a molecule that contains more than one hydroxyl group. A polyol may be a diol, triol, or a tetraol which contain 2, 3, and 4 hydroxyl groups, respectively. A polyol also may contain more than four hydroxyl groups, such as a pentaol, hexaol, heptaol, or the like, which contain 5, 6, or 7 hydroxyl groups, respectively. Additionally, a polyol also may be a sugar alcohol, polyhydric alcohol, or polyalcohol which is a reduced form of carbohydrate, wherein the carbonyl group (aldehyde or ketone, reducing sugar) has been reduced to a primary or secondary hydroxyl group. Non-limiting examples of polyols in some embodiments include erythritol, maltitol, mannitol, sorbitol, lactitol, xylitol, isomalt, propylene glycol, glycerol (glycerin), threitol, galactitol, palatinose, reduced isomalto-oligosaccharides, reduced xylo-oligosaccharides, reduced gentio-oligosaccharides, reduced maltose syrup, reduced glucose syrup, and sugar alcohols or any other carbohydrates capable of being reduced which do not adversely affect the taste of the edible composition.

In some embodiments, the sweetener is a carbohydrate sweetener. In such embodiments, the sweetener is chosen from sucrose, fructose, glucose, erythritol, high fructose corn or starch syrup, and mixtures thereof.

The terms "synthetic high potency sweetener" and "artificial high potency sweetener" are used interchangeably herein and refer to any composition which is not found naturally in nature and characteristically has a sweetness potency greater than sucrose, fructose, or glucose, yet have fewer or no calories. Non-limiting examples of synthetic sweeteners suitable for embodiments of this disclosure include sucralose, acesulfame potassium or other salts, aspartame, alitame, saccharin, neohesperidin dihydrochalcone, cyclamate, neotame, advantame, and salts thereof.

In some embodiments, the sweetener is a synthetic sweetener. Preferably, the synthetic sweetener is chosen from sucralose, aspartame, potassium acesulfame, and mixtures thereof.

Other sweeteners suitable for use in embodiments provided herein, for example, include natural sweeteners. The terms "natural high-potency sweetener," "NHPS," "NHPS composition," and "natural high-potency sweetener composition" are used interchangeably, herein, and refer to any sweetener found in nature which may be in raw, extracted, purified, or any other form, singularly or in combination thereof and characteristically have a sweetness potency greater than sucrose, fructose, or glucose, yet have fewer or no calories. Non-limiting examples of NHPSs suitable for embodiments of this disclosure include steviol glycoside, rebaudioside A, rebaudioside B, rebaudioside C (dulcoside B), rebaudioside D, rebaudioside E, rebaudioside F, rebaudioside I, rebaudioside H, rebaudioside L, rebaudioside K, rebaudioside J, rebaudioside N, rebaudioside O, rebaudioside M, dulcoside A, rubusoside, stevia leaf extract, stevioside, glycosylated steviol glycosides, mogrosides, mogroside V, isomogroside, mogroside IV, Luo Han Guo fruit extract, siamenoside, monatin and its salts (monatin SS, RR, RS, SR), curculin, glycyrrhizic acid and its salts, thaumatin, monellin, mabinlin, brazzein, hernandulcin, phyllodulcin, glycyphyllin, phloridzin, trilobatin, baiyunoside, osladin, polypodoside A, pterocaryoside A, pterocaryoside B, mukurozioside, phlomisoside I, periandrin I, abrusoside A, or cyclocarioside I. In some embodiments, the glycosylated steviol glycoside is 13-[(2-O-β-D-glucopyranosyl-3-O-(4-O-α-D-glucopyranosyl)-β-D-glucopyranosyl-β-D-glucopyranosyl)oxy]ent-kaur-16-en-19-oic acid-[(4-O-α-D-glucopyranosyl-β-D-glucopyranosyl)ester], 13-[(2-O-β-D-glucopyranosyl-β-D-glucopyranosyl)oxy]ent-kaur-16-en-19-oic acid-[(4-O-(4-O-(4-O-α-D-glucopyranosyl)-α-D-glucopyranosyl)-α-D-glucopyranosyl)-β-D-glucopyranosyl ester], 13-[(2-O-β-D-glucopyranosyl-3-O-(4-O-(4-O-(4-O-α-D-glucopyranosyl)-α-D-glucopyranosyl)-α-D-glucopyranosyl)-β-D-glucopyranosyl-β-D-glucopyranosyl)oxy] ent-kaur-16-en-19-oic acid β-D-glucopyranosyl ester, or 13-[(2-O-β-D-glucopyranosyl-3-O-(4-O-(4-O-(4-O-α-D-glucopyranosyl)-α-D-glucopyranosyl)-α-D-glucopyranosyl)-β-D-glucopyranosyl-β-D-glucopyranosyl)oxy]ent-kaur-16-en-19-oic acid-[(4-O-α-D-glucopyranosyl-β-D-glucopyranosyl)ester]. NHPS also includes modified NHPSs. Modified NHPSs include NHPSs which have been altered naturally. For example, a modified NHPS includes, but is not limited to, NHPSs which have been fermented, contacted with enzyme, or derivatized or substituted on the NHPS. In one embodiment, at least one modified NHPS may be used in combination with at least one NHPS. In another embodiment, at least one modified NHPS may be used without a NHPS. Thus, modified NHPSs may be substituted for a NHPS or may be used in combination with NHPSs for any of the embodiments described herein. For the sake of brevity, however, in the description of embodiments, a modified NHPS is not expressly described as an alternative to an unmodified NHPS, but it should be understood that modified NHPSs can be substituted for NHPSs in any embodiment disclosed herein.

In some embodiments, the sweetener may be used individually or in combination with other sweeteners. For example, the sweetener composition may comprise a single caloric sweetener, a single NHPS or a single synthetic sweetener; a single caloric sweetener with a single NHPS; a single caloric sweetener with a single synthetic sweetener; one or more caloric sweetener with a single NHPS; one or more caloric sweetener with a single synthetic sweetener; a single caloric sweetener with one or more NHPS; a single caloric sweetener with one or more synthetic sweeteners; a single NHPS in combination with a single synthetic sweetener; one or more NHPSs in combination with a single synthetic sweetener; a single NHPS in combination with one or more synthetic sweeteners; one or more NHPSs in combination with one or more synthetic sweeteners; or one or more caloric sweetener with one or more NHPS and one or more synthetic sweetener. A plurality of natural and/or synthetic sweeteners may be used as long as the combined effect does not adversely affect the taste of the sweetener composition or orally sweetened composition.

One of ordinary skill in the art should appreciate that the sweetener composition can be customized to obtain a desired calorie content. For example, a low-caloric or non-caloric synthetic sweetener may be combined with a caloric sweetener and/or other caloric additives to produce a sweetener composition with a preferred calorie content.

The sweetener is present in the composition in an amount greater than its sweetness threshold level. In some embodiments, the sweetener is present in an amount ranging from 0.01% to 99.9% by weight, relative to the total weight of the composition. For example, the at least one sweetener is present in an amount ranging from 2% to 50%, or for example from 4% to 50% by weight, relative to the total weight of the composition. In some embodiments, the at least one sweetener is present in about 5% to about 20% by weight. In further embodiments, the at least one sweetener is present in about 5% to about 15% by weight. In yet further embodiments, the at least one sweetener is present in about 5% to about 12% by weight in beverages, for example, in non-alcoholic beverages.

In accordance with the disclosure, the sweet taste modulator, or the combination of sweet taste modulators, of the present disclosure potentiates or enhances the sweetness of the sweetener. The composition comprising the sweetener and the sweet taste modulator, or the combination of sweet taste modulators, of the present disclosure has more sweetness intensity than a composition comprising the at least one sweetener without the sweet taste modulator, or the combination of sweet taste modulators.

As used herein, the term "sweetness intensity" is understood to mean any perceptible sweetness. The composition comprising the sweetener and the sweet taste modulator, or the combination of sweet taste modulators, of the present disclosure is perceptibly sweeter than a composition comprising the sweetener without the sweet taste modulator, or the combination of sweet taste modulators. For example, a composition comprising the sweetener and the sweet taste modulator, or the combination of sweet taste modulators, of the present disclosure may be slightly sweeter, moderately sweeter, or significantly sweeter than a composition comprising the sweetener without the sweet taste modulator, or the combination of sweet taste modulators. As discussed above, in embodiments where the sweet taste modulators of Formula (I), Compound 1, or a comestibly or biologically acceptable salt, solvate, or enantiomer thereof, or a combination of any of the foregoing compounds, are used above their sweetness threshold, the increase in sweetness intensity is synergistic, not additive.

The sweetness of a composition may be based on (*i.e.,* relative to) a known sweet standard. Sweet compounds based on such sweet standards include, but are not limited to, for example natural, non-natural, or synthetic sweeteners. Non-limiting examples of such sweeteners include caloric carbohydrate sweeteners, natural carbohydrate sweeteners, non-natural carbohydrate sweeteners, natural high-potency sweeteners, non-natural high-potency sweeteners, synthetic high potency sweeteners, synthetic carbohydrate sweeteners, and combinations thereof. For example, the sweetness of a composition may be based on to a 5% sucrose solution. In such cases, a composition comprising the sweetener and a sweet taste modulator, or a combination of sweet taste modulators, of the present disclosure may be perceived as having a sweetness equivalent to a 5.5% sucrose solution. In other embodiments, the composition comprising the sweetener and a sweet taste modulator, or a combination of sweet taste modulators, of the present disclosure may be perceived as having a sweetness equivalent to a 6.0%, 6.5%, 7.0%, 7.5%, 8.0%, 8.5%, 9.0%, 9.5%, or 10% sucrose solution. Suitable sweet standards include, but are not limited to, sucrose standards, fructose standards and glucose standards. Each of these standards may be used at concentrations which include, but are not limited to, 0.5%, 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, or 10% solution. In some embodiments, the sweetness intensity of the composition comprising the sweetener and a sweet taste modulator, or a combination of sweet taste modulators, of the present disclosure increases the perceived sweetness based on a sweet standard by greater than 10%, by greater than 20%, by greater than 30%, by greater than 40%, greater than by 50% or greater than by 60% compared to a composition comprising the sweetener without the sweet taste modulator, or the combination of sweet taste modulators.

In some embodiments, the perception of sweetness intensity of the sweetener (*i.e.,* the perception of sweet taste of the sweetener in the edible composition) is enhanced by up to 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%. In some embodiments, the perception of sweetness intensity of the sweetener is enhanced beyond 100%, for example, by 125%, 150%, 175%, 200%, 225%, 250%, 275%, 300%, 325%, 350%, 375%, 400%, 425%, 450%, 475%, 500% or increments in between those recited. In some embodiments, the perception of sweetness intensity is enhanced by up to 25%. In other embodiments, the perception of sweetness intensity is enhanced by up to 50%. In other embodiments, the perception of sweetness intensity is enhanced by up to 75%. In other embodiments, the sweetness intensity is enhanced by up to 100%. In some embodiments, the perception of sweetness intensity is enhanced by about 5% to about 100%, about 5% to about 90%, about 5% to about 80%, about 5% to about 70%, about 5% to about 60%, about 5% to about 50%, about 5% to about 40%, about 5% to about 30%, about 10% to about 30%, about 10% to about 25%. about 20% to about 80%, about 20% to about 70%, about 20% to about 60%, about 20% to about 50%, about 20% to about 40%, about 20% to about 30%, about 25% to about 80%, about 25% to about 70%, about 25% to about 60%, about 25% to about 50%, about 25% to about 40%, or about 25% to about 30%. These amounts are not meant to be limiting, and increments between the recited percentages are specifically envisioned as part of the disclosure.

It is contemplated that the combination of at least one sweetness enhancer and at least one sweetener may be carried out in any pH range that does not materially or adversely affect the taste of the sweetener composition or the sweetened composition. A non-limiting example of the pH range may be from about 1.5 to about 9.0. Further examples include a pH range from about 2.0 to about 8.5, from about 2.0 to about 8.0, from about 2.0 to about 7.5, from about 2.0 to about 7.0, from about 2.5 to about 7.0, and from about 3.0 to about 7.0. Additional examples of pH ranges include from about 2.0 to about 4.0, from about 2.5 to about 4.5, from about 3.5 to about 5.5, from about 5.0 to about 6.0, from about 4.0 to about 5.5, from about 5.0 to about 6.0, from about 6.5 to about 7.5, and from about 6.0 to about 8.0. In some embodiments, the pH is about 3.0 or about 7.0. The temperature of the composition may, for example, range from about -4° C to about 90° C.

One of ordinary skill in the art may combine the sweetener(s) and sweet taste modulator(s), or the combination of sweet taste modulators, in any manner.

### Sweet Taste Improving Compositions

The terms "sweet taste improving composition" and "sweet taste improving additive" are used interchangeably herein and refer to any material that imparts a more sugar-like temporal profile or sugar-like flavor profile or both to a synthetic sweetener. Suitable sweet taste improving additives useful in embodiments of this disclosure include amino acids and salts thereof, poly-amino acids and salts thereof, peptides, sugar acids and salts thereof, nucleotides and salts thereof, organic acids, inorganic acids, organic salts including organic acid salts and organic base salts, inorganic acid salts (*e.g.,* sodium chloride, potassium chloride, magnesium chloride), acid salts (*e.g.,* sodium citrate), bitter compounds, flavorants and flavoring ingredients, astringent compounds, polymers, proteins or protein hydrolysates, surfactants, emulsifiers, flavonoids, alcohols, and natural high-potency sweeteners.

The terms "sugar-like characteristic," "sugar-like taste," "sugar-like sweet," "sugary," and "sugar-like" are used interchangeably, herein, and include any characteristic similar to that of sucrose and include, but are not limited to, maximal response, flavor profile, temporal profile, adaptation behavior, mouth feel, concentration/response function behavior, tastant and flavor/sweet taste interactions, spatial pattern selectivity, and temperature effects. These characteristics are dimensions in which the taste of sucrose is different from the tastes of sweetness enhanced sweetener compositions. Suitable procedures for determining whether a composition has a more sugar-like taste are well known in the art.

The compositions of the present disclosure may also further comprise at least one additional additive, such as a sweet taste improving composition, and/or a sweet taste improving additive. For example, the composition of the disclosure may comprise at least one sweet taste improving composition for balancing the temporal and/or flavor profile of the sweetness enhanced sweetener composition. The use of sweet taste improving compositions to improve the temporal and/or flavor profile of sweetener compositions are described in detail in U.S. Patent Application Publication Nos. 2007/0128311, 2007/0275147, 2008/0292765, 2011/0160311, and US 2011/0318464.

Exemplary suitable sweet-taste improving compounds include, but are not limited to, carbohydrates, polyols, amino acids and their corresponding salts, poly-amino acids and their corresponding salts, sugar acids and their corresponding salts, nucleotides, organic acids, inorganic acids, organic salts including organic acid salts and organic base salts, inorganic salts, bitter compounds, flavorants and flavoring ingredients, astringent compounds, proteins or protein hydrolysates, surfactants, emulsifiers, flavonoids, alcohols, polymers, other sweet taste improving taste additives imparting such sugar-like characteristics, and combinations thereof. In some embodiments, the sweet-taste improving compound is erythritol. In some such embodiments, the ratio of erythritol to Compound 1 is about 1:1 to about 800:1 by weight. In other embodiments, the ratio of erythritol to Compound 1 is about (30-200):1 or about (50-100):1 by weight.

Suitable sweet taste improving amino acid additives for use in embodiments of this disclosure include, but are not limited to, aspartic acid, arginine, glycine, glutamic acid, proline, threonine, theanine, cysteine, cystine, alanine, valine, tyrosine, leucine, isoleucine, asparagine, serine, lysine, histidine, ornithine, methionine, carnitine, aminobutyric acid (α-, β-, or γ-isomers), glutamine, hydroxyproline, taurine, norvaline, sarcosine, and their salt forms such as sodium or potassium salts or acid salts. The sweet taste improving amino acid additives also may be in the D- or L-configuration and in the mono-, di-, or tri-form of the same or different amino acids. Additionally, the amino acids may be α-, β-, γ-, δ-, and ε-isomers if appropriate. Combinations of the foregoing amino acids and their corresponding salts (*e.g.,* sodium, potassium, calcium, magnesium salts or other alkali or alkaline earth metal salts thereof, or acid salts) also are suitable sweet taste improving additives in some embodiments. The amino acids may be natural or synthetic. The amino acids also may be modified. Modified amino acids refers to any amino acid wherein at least one atom has been added, removed, substituted, or combinations thereof (*e.g.,* N-alkyl amino acid, N-acyl amino acid, or N-methyl amino acid). Non-limiting examples of modified amino acids include amino acid derivatives such as trimethyl glycine, N-methyl-glycine, and N-methyl-alanine. As used herein, modified amino acids encompass both modified and unmodified amino acids. As used herein, amino acids also encompass both peptides and polypeptides (*e.g.,* dipeptides, tripeptides, tetrapeptides, and pentapeptides) such as glutathione and L-alanyl-L-glutamine. Suitable sweet taste improving polyamino acid additives include poly-L-aspartic acid, poly-L-lysine (*e.g*., poly-L-α-lysine or poly-L-ε-lysine), poly-L-ornithine (*e.g*., poly-L-α-omithine or poly-L-ε-ornithine), poly-L-arginine, other polymeric forms of amino acids, and salt forms thereof (*e.g.*, calcium, potassium, sodium, or magnesium salts such as L-glutamic acid mono sodium salt). The sweet taste improving poly-amino acid additives also may be in the D- or L-configuration. Additionally, the poly-amino acids may be α-, β-, γ-, δ-, and ε-isomers if appropriate. Combinations of the foregoing poly-amino acids and their corresponding salts (*e.g*., sodium, potassium, calcium, magnesium salts or other alkali or alkaline earth metal salts thereof or acid salts) also are suitable sweet taste improving additives in some embodiments. The poly-amino acids described herein also may comprise co-polymers of different amino acids. The poly-amino acids may be natural or synthetic. The poly-amino acids also may be modified, such that at least one atom has been added, removed, substituted, or combinations thereof (*e.g.,* N-alkyl poly-amino acid or N-acyl poly-amino acid). As used herein, poly-amino acids encompass both modified and unmodified poly-amino acids. For example, modified poly-amino acids include, but are not limited to poly-amino acids of various molecular weights (MW), such as poly-L-α-lysine with a MW of 1,500, MW of 6,000, MW of 25,200, MW of 63,000, MW of 83,000, or MW of 300,000. In some embodiments, the taste improving amino acid additive is glycine, alanine, taurine, serine or proline. In such embodiments, the taste improving amino acid additive is present in a concentration of about 10 ppm to about 25,000 ppm or about 100 ppm to about 1000 ppm.

Suitable sweet taste improving sugar acid additives include, for example, but are not limited to aldonic, uronic, aldaric, alginic, gluconic, glucuronic, glucaric, galactaric, galacturonic, and salts thereof (*e.g.,* sodium, potassium, calcium, magnesium salts or other physiologically acceptable salts), and combinations thereof.

For example, suitable sweet taste improving nucleotide additives include, but are not limited to, inosine monophosphate ("IMP"), guanosine monophosphate ("GMP"), adenosine monophosphate ("AMP"), cytosine monophosphate ("CMP"), uracil monophosphate ("UMP"), inosine diphosphate, guanosine diphosphate, adenosine diphosphate, cytosine diphosphate, uracil diphosphate, inosine triphosphate, guanosine triphosphate, adenosine triphosphate, cytosine triphosphate, uracil triphosphate, alkali or alkaline earth metal salts thereof, and combinations thereof. The nucleotides described herein also may comprise nucleotide-related additives, such as nucleosides or nucleic acid bases (*e.g.,* guanine, cytosine, adenine, thymine, uracil).

Suitable sweet taste improving organic acid additives include any compound which comprises a-COOH moiety. Suitable sweet taste improving organic acid additives, for example, include but are not limited to C₂-C₃₀ carboxylic acids, substituted hydroxyl C₂-C₃₀ carboxylic acids, benzoic acid, substituted benzoic acids (*e.g.,* 2,4-dihydroxybenzoic acid), substituted cinnamic acids, hydroxyacids, substituted hydroxybenzoic acids, substituted cyclohexyl carboxylic acids, tannic acid, lactic acid, tartaric acid, citric acid, gluconic acid, glucoheptonic acids, adipic acid, hydroxycitric acid, malic acid, fruitaric acid (a blend of malic, fumaric, and tartaric acids), fumaric acid, maleic acid, succinic acid, chlorogenic acid, salicylic acid, creatine, caffeic acid, bile acids, acetic acid, ascorbic acid, alginic acid, erythorbic acid, polyglutamic acid, glucono delta lactone, and their alkali or alkaline earth metal salt derivatives thereof. In addition, the organic acid additives also may be in either the D- or L-configuration.

For example, suitable sweet taste improving organic acid additive salts include, but are not limited to, sodium, calcium, potassium, and magnesium salts of all organic acids, such as salts of citric acid, malic acid, tartaric acid, fumaric acid, lactic acid (*e.g.,* sodium lactate), alginic acid (*e.g.,* sodium alginate), ascorbic acid (*e.g.,* sodium ascorbate), benzoic acid (*e.g.,* sodium benzoate or potassium benzoate), and adipic acid. The examples of the sweet taste improving organic acid additives described optionally may be substituted with at least one group chosen from hydrogen, alkyl, alkenyl, alkynyl, halo, haloalkyl, carboxyl, acyl, acyloxy, amino, amido, carboxyl derivatives, alkylamino, dialkylamino, arylamino, alkoxy, aryloxy, nitro, cyano, sulfo, thiol, imine, sulfonyl, sulfenyl, sulfinyl, sulfamyl, carboxalkoxy, carboxamido, phosphonyl, phosphinyl, phosphoryl, phosphino, thioester, thioether, anhydride, oximino, hydrazino, carbamyl, phospho, phosphonato, and any other viable functional group provided the substituted organic acid additives function to improve the sweet taste of a synthetic sweetener.

For example, suitable sweet taste improving inorganic acid additives include but are not limited to phosphoric acid, phosphorous acid, polyphosphoric acid, hydrochloric acid, sulfuric acid, carbonic acid, sodium dihydrogen phosphate, and alkali or alkaline earth metal salts thereof (e.g., inositol hexaphosphate Mg/Ca).

Suitable sweet taste improving bitter compound additives, for example, include but are not limited to caffeine, quinine, urea, bitter orange oil, naringin, quassia, and salts thereof.

### Edible compositions

The edible compositions according to the invention comprise a (i) sweetener; and (ii) a compound of Formula (I), Compound 1, or a comestibly or biologically acceptable salt, solvate, or enantiomer thereof, or a combination of any of the foregoing compounds.

The terms "edible composition," "orally ingestible composition" and "sweetenable composition" are used interchangeably, herein, and refer to a composition suitable for consumption, typically via the oral cavity (although consumption may occur via non-oral means such as inhalation). Edible compositions may be present in any form including, but not limited to, liquids, solids, semi-solids, tablets, lozenges, powders, gels, gums, pastes, slurries, syrups, aerosols and sprays. As used herein, edible compositions include food products, pharmaceutical compositions, and consumer products. The term edible composition also refers to, for example, dietary and nutritional supplements. As used herein, edible compositions also include compositions that are placed within the oral cavity but not swallowed, including professional dental products, such as dental treatments, fillings, packing materials, molds and polishes. The term "comestible" refers to similar compositions and is generally used as a synonym to the term "edible."

The term "food product" refers to any composition comprising one or more processed foodstuffs. Food products include, but are not limited to, confectionaries, bakery products (including, but not limited to, doughs, breads, biscuits, crackers, cakes, pastries, pies, tarts, quiches, and cookies), ice creams (including but not limited to impulse ice cream, take-home ice cream, frozen yogurt, gelato, sorbet, sherbet and soy, oat, bean and rice-based ice cream), dairy products (including, but not limited to, drinking milk, cheese, yogurt, and sour milk drinks), cheeses (including, but not limited to, natural cheeses and processed cheeses), butter, margarine, sweet and savory snacks (including but not limited to fruit snacks, chips/crisps, tortilla/corn chips, popcorn, pretzels, chocolates, and nuts), hot and cold beverages (including, but not limited to, beverages, beverage mixes, concentrates, juices, carbonated beverages, non-carbonated beverages, alcoholic beverages, non-alcoholic beverages, soft drinks, sports drinks, isotonic drinks, coffees, teas, bottled waters, and beverages prepared from botanicals and botanical extracts (including cold beverages that are prepared with botanical or fungi extracts as ingredients, and drinks that are prepared in various ways, such as infusions, decoctions, or other means of extraction or distillation of various plant parts, including, but not limited to leaves, flowers, stems, fruits, roots, rhizomes, stems, bark, volatile oils, or even the whole plant), snack bars (including, but not limited to granola bars, muesli bars, protein bars, breakfast bars, energy bars, and fruit bars), meal replacement products, ready meals (including, but not limited to canned meals, preserved meals, frozen meals, dried meals, chilled meals, dinner mixes, macaroni and cheese, frozen pizza, chilled pizza, and prepared salads), soups (including but not limited to broth-like soups and cream-based soups), broth, gravy, soy sauce, meats and fish (including raw, cooked, and dried meats), deli products (including but not limited to meats and cheeses suitable for slicing or pre-sliced meats and cheeses, *e.g.,* turkey, chicken, ham, bologna, salami, bierwurst, capicola, chorizo, corned beef, Dutch loaf, Serrano, prosciutto, head cheese, liverwurst, meatloaf (including olive loaf, pepper loaf, pimento loaf, and ham and cheese loaf), mortadella, pastrami, pepperoni, roast beef, roast pork, saucisson, smoked meat, summer sausage, tongue, American cheese, blue cheese, cheddar cheese, Colby cheese, Colby-Jack cheese, gouda, Monterey Jack cheese, Muenster cheese mozzarella, Parmigiano cheese, pepper jack cheese, provolone, Romano cheese, string cheese, spray cheese, and Swiss cheese), vegetables (including, but not limited to, raw, pickled, cooked, and dried vegetables, such as French fries), fruits (including raw, cooked, and dried fruits), grains (including, but not limited to, dried cereals and breads), prepared foods (including, but not limited to, dried, canned, or jarred sauces and soups), snack foods, pastas (including, but not limited to, fresh pasta, chilled pasta, frozen pasta, dried pasta, and macaroni), noodles (including, but not limited to, egg noodles, wheat noodles, rice noodles, mung bean noodles, potato noodles, buckwheat noodles, corn noodles, cellophane noodles, chow mein, fettuccini, fusilli, gnocchi, lasagna, linguini, lo mein, macaroni, manicotti, pad thai, penne, ramen, rice vermicelli, rigatoni, soba, spaghetti, spatzle, udon, and ziti), canned foods, frozen foods, dried foods, chilled foods, oils and fats, baby food, spreads, salads, cereals (including, but not limited to, hot and cold cereals), sauces (including, but not limited to, cheese sauces (*e.g.,* for macaroni and cheese) tomato pastes, tomato purees, bouillon cubes, stock cubes, table sauces, bouillabaisse sauces, pasta sauces, cooking sauces, marinades, dry sauces, powder mixes, ketchups, mayonnaises, salad dressings, vinaigrettes, mustards, and dips), jellies, jams, preserves, honey, puddings, recipe mixes, syrups, icings, fillings, infused foods, salt-preserved food, marinated foods and condiments (such as ketchup, mustard and steak sauce). In some embodiments, the food product is animal feed. For example, the food product may be a pet food product, *i.e.* a food product for consumption by a household pet. In other embodiments, the food product is a livestock food product, *i.e.* a food product for consumption by livestock.

The term "foodstuff" refers to an unprocessed ingredient or a basic nutrient or flavor containing element used to prepare a food product. Non-limiting examples of foodstuffs include: fruits, vegetables, meats, fishes, grains, milks, eggs, tubers, sugars, sweeteners, oils, herbs, snacks, sauces, spices and salts.

The term "processed foodstuff' refers to a foodstuff has been subjected to any process which alters its original state (excluding, e.g., harvesting, slaughtering, and cleaning). Examples of methods of processing foods include, but are not limited to, removal of unwanted outer layers, such as potato peeling or the skinning of peaches; chopping or slicing; mincing or macerating; liquefaction, such as to produce fruit juice; fermentation (*e.g*. beer); emulsification; cooking, such as boiling, broiling, frying, heating, steaming or grilling; deep frying; baking; mixing; addition of gas such as air entrainment for bread or gasification of soft drinks; proofing; seasoning (with, e.g., herbs, spices, salts); spray drying; pasteurization; packaging (e.g., canning or boxing); extrusion; puffing; blending; and preservation (*e.g*., adding salt, sugar, potassium lactate or other preservatives).

The term "consumer product" refers to health and beauty products for the personal use and/or consumption by a subject. Consumer products may be present in any form including, but not limited to, liquids, solids, semi-solids, tablets, capsules, lozenges, strips, powders, gels, gums, pastes, slurries, syrups, aerosols and sprays. Non-limiting examples of consumer products include nutriceuticals, nutritional supplements, lipsticks, lip balms, soaps, shampoos, gums, adhesives (e.g., dental adhesives), toothpastes, oral analgesics, breath fresheners, mouthwashes, tooth whiteners, and other dentifrices.

The edible composition of the disclosure may comprise (i) a sweet taste modulator of the disclosure, or combinations thereof; and (ii) a sweetener. In some embodiments, the sweet taste modulating compound or the combination of sweet taste modulators, is a neoflavonoid compound having a molecular weight less than about 1000, 500, or 300 daltons. In certain embodiments, the sweet taste modulating compound is a compound of Formula (I), comestibly or biologically acceptable salts, solvates, enantiomers, or derivatives thereof, or combinations of any of the foregoing. In other embodiments, the sweet taste modulating compound is Compound 1, or comestibly or biologically acceptable salts, solvates, enantiomers, or derivatives thereof.

In some embodiments, the edible composition naturally or inherently comprises a sweetener. For example, the sweetener may be an inherent component of a food product or of a food stuff, such as fruit or a fruit product (*e.g*., fruit sauce). Accordingly, the compounds, or the combination of compounds, of the present disclosure may be added to edible compositions to which no sweetener is added.

In another embodiments, the edible composition is a sweetened composition comprising (i) a sweet taste modulator of the disclosure (*e.g*., a compound of Formula (I), Compound 1, or a comestibly or biologically acceptable salt, solvate, or enantiomer thereof, or a combination of any of the foregoing compounds); and (ii) a sweetener.

In some embodiments, the compound of Formula (I), Compound 1, or a comestibly or biologically acceptable salt, solvate, or enantiomer thereof, or a combination of any of the foregoing compounds, may be used to enhance the sweet taste or perception of any suitable natural or synthetic sweetener, such as any suitable caloric, low-caloric or non-caloric sweetener. The compound of Formula (I), Compound 1, or a comestibly or biologically acceptable salt, solvate, or enantiomer thereof, or a combination of any of the foregoing compounds, may have an inherent sweet taste and, in some embodiments, it is present at or above its sweetness threshold, but is not the primary sweetener in the composition. Rather, the compound of Formula (I), Compound 1, or a comestibly or biologically acceptable salt, solvate, or enantiomer thereof, or a combination of any of the foregoing compounds, serves to enhance the sweet taste of the sweetener. The compound of Formula (I), or Compound 1, or a comestibly or biologically acceptable salt, solvate, or enantiomer thereof, or a combination of any of the foregoing compounds, may be present at or below its sweetness threshold. In such cases, the compound, or combination of compounds, serves only to enhance the sweet taste of the sweetener. A person of skill in the art will be able to select the concentration of the sweet taste modulator, or the combination of sweet taste modulators, so that it may impart the perception of enhanced sweetness to a composition comprising a sweetener. For example, a skilled artisan may select a concentration for the sweet taste modulator, or the combination of sweet taste modulators, so that it does not impart any perceptible sweetness to a composition that does not comprise a sweetener. Non-limiting examples of such sweeteners include caloric carbohydrate sweeteners, natural carbohydrate sweeteners, non-natural carbohydrate sweeteners, natural high-potency sweeteners, non-natural high-potency sweeteners, synthetic high potency sweeteners, synthetic carbohydrate sweeteners, and combinations thereof.

In some embodiments, the edible composition further comprises functional ingredients. The term "functional ingredient" refers to compound which provide a real or perceived heath benefit to the composition. Functional ingredients include, but are not limited to, saponins, antioxidants, dietary fiber sources, fatty acids, vitamins, glucosamine, minerals, preservatives, hydration agents, probiotics, prebiotics, weight management agents, osteoporosis management agents, phytoestrogens, long chain primary aliphatic saturated alcohols, phytosterols, and combinations thereof.

In some embodiments the edible compositions are beverages. In further embodiments, the beverage can also contain one or more functional ingredients, which provide a real or perceived heath benefit to the composition. Functional ingredients include, but are not limited to, saponins, antioxidants, dietary fiber sources, fatty acids, vitamins, glucosamine, minerals, preservatives, hydration agents, probiotics, prebiotics, weight management agents, osteoporosis management agents, phytoestrogens, long chain primary aliphatic saturated alcohols, phytosterols and combinations thereof.

In certain embodiments, the functional ingredient is at least one saponin. As used herein, the at least one saponin may comprise a single saponin or a plurality of saponins as a functional ingredient for the edible compositions (*e.g*., beverages) provided herein. Generally, according to particular embodiments of this disclosure, the at least one saponin is present in the edible composition (*e.g*., beverage) in a concentration sufficient to promote health and wellness.

Saponins are glycosidic natural plant products comprising an aglycone ring structure and one or more sugar moieties. The combination of the nonpolar aglycone and the water soluble sugar moiety gives saponins surfactant properties, which allow them to form a foam when shaken in an aqueous solution.

The saponins are grouped together based on several common properties. In particular, saponins are surfactants which display hemolytic activity and form complexes with cholesterol. Although saponins share these properties, they are structurally diverse. The types of aglycone ring structures forming the ring structure in saponins can vary greatly. Non-limiting examples of the types of aglycone ring structures in saponin for use in particular embodiments of the disclosure include steroids, triterpenoids, and steroidal alkaloids. Non-limiting examples of specific aglycone ring structures for use in particular embodiments of the disclosure include soyasapogenol A, soyasapogenol B and soyasopogenol E. The number and type of sugar moieties attached to the aglycone ring structure can also vary greatly. Non-limiting examples of sugar moieties for use in particular embodiments of the disclosure include glucose, galactose, glucuronic acid, xylose, rhamnose, and methylpentose moieties. Non-limiting examples of specific saponins for use in particular embodiments of the disclosure include group A acetyl saponin, group B acetyl saponin, and group E acetyl saponin.

Saponins can be found in a large variety of plants and plant products, and are especially prevalent in plant skins and barks where they form a waxy protective coating. Several common sources of saponins include soybeans, which have approximately 5% saponin content by dry weight, soapwort plants (*Saponaria*)*,* the root of which was used historically as soap, as well as alfalfa, aloe, asparagus, grapes, chickpeas, yucca, and various other beans and weeds. Saponins may be obtained from these sources by using extraction techniques well known to those of ordinary skill in the art. A description of conventional extraction techniques can be found in U.S. Pat. Appl. No. 2005/0123 662.

In certain embodiments, the functional ingredient is at least one antioxidant. As used herein, the at least one antioxidant may comprise a single antioxidant or a plurality of antioxidants as a functional ingredient for the edible compositions (*e.g*., beverages) provided herein. Generally, according to particular embodiments of this disclosure, the at least one antioxidant is present in the edible composition (*e.g*., beverage) in a concentration sufficient to promote health and wellness.

As used herein "antioxidant" refers to any substance which inhibits, suppresses, or reduces oxidative damage to cells and biomolecules. Without being bound by theory, it is believed that antioxidants inhibit, suppress, or reduce oxidative damage to cells or biomolecules by stabilizing free radicals before they can cause harmful reactions. As such, antioxidants may prevent or postpone the onset of some degenerative diseases.

Examples of suitable antioxidants for embodiments of this disclosure include, but are not limited to, vitamins, vitamin cofactors, minerals, hormones, carotenoids, carotenoid terpenoids, non-carotenoid terpenoids, flavonoids, flavonoid polyphenolics (e.g., bioflavonoids), flavonols, flavones, phenols, polyphenols, esters of phenols, esters of polyphenols, nonflavonoid phenolics, isothiocyanates, and combinations thereof. In some embodiments, the antioxidant is vitamin A, vitamin C, vitamin E, ubiquinone, mineral selenium, manganese, melatonin, α-carotene, β-carotene, lycopene, lutein, zeanthin, crypoxanthin, reservatol, eugenol, quercetin, catechin, gossypol, hesperetin, curcumin, ferulic acid, thymol, hydroxytyrosol, tumeric, thyme, olive oil, lipoic acid, glutathinone, gutamine, oxalic acid, tocopherol-derived compounds, butylated hydroxyanisole (BHA), butylated hydroxytoluene (BHT), ethylenediaminetetraacetic acid (EDTA), tert-butylhydroquinone, acetic acid, pectin, tocotrienol, tocopherol, coenzyme Q10, zeaxanthin, astaxanthin, canthaxantin, saponins, limonoids, kaempfedrol, myricetin, isorhamnetin, proanthocyanidins, quercetin, rutin, luteolin, apigenin, tangeritin, hesperetin, naringenin, erodictyol, flavan-3-ols (*e.g*., anthocyanidins), gallocatechins, epicatechin and its gallate forms, epigallocatechin and its gallate forms (ECGC) theaflavin and its gallate forms, thearubigins, isoflavone phytoestrogens, genistein, daidzein, glycitein, anythocyanins, cyaniding, delphinidin, malvidin, pelargonidin, peonidin, petunidin, ellagic acid, gallic acid, salicylic acid, rosmarinic acid, cinnamic acid and its derivatives (*e.g*., ferulic acid), chlorogenic acid, chicoric acid, gallotannins, ellagitannins, anthoxanthins, betacyanins and other plant pigments, silymarin, citric acid, lignan, antinutrients, bilirubin, uric acid, R-α-lipoic acid, N-acetylcysteine, emblicanin, apple extract, apple skin extract (applephenon), rooibos extract red, rooibos extract, green, hawthorn berry extract, red raspberry extract, green coffee antioxidant (GCA), aronia extract 20%, grape seed extract (VinOseed), cocoa extract, hops extract, mangosteen extract, mangosteen hull extract, cranberry extract, pomegranate extract, pomegranate hull extract, pomegranate seed extract, hawthorn berry extract, pomella pomegranate extract, cinnamon bark extract, grape skin extract, bilberry extract, pine bark extract, pycnogenol, elderberry extract, mulberry root extract, wolfberry (goji) extract, blackberry extract, blueberry extract, blueberry leaf extract, raspberry extract, turmeric extract, citrus bioflavonoids, black currant, ginger, acai powder, green coffee bean extract, green tea extract, and phytic acid, or combinations thereof. In alternate embodiments, the antioxidant is a synthetic antioxidant such as butylated hydroxytolune or butylated hydroxyanisole, for example. Other sources of suitable antioxidants for embodiments of this disclosure include, but are not limited to, fruits, vegetables, tea, cocoa, chocolate, spices, herbs, rice, organ meats from livestock, yeast, whole grains, or cereal grains.

Particular antioxidants belong to the class of phytonutrients called polyphenols (also known as "polyphenolics"), which are a group of chemical substances found in plants, characterized by the presence of more than one phenol group per molecule. A variety of health benefits may be derived from polyphenols, including prevention of cancer, heart disease, and chronic inflammatory disease and improved mental strength and physical strength, for example. Suitable polyphenols for embodiments of this disclosure, include catechins, proanthocyanidins, procyanidins, anthocyanins, quercerin, rutin, reservatrol, isoflavones, curcumin, punicalagin, ellagitannin, hesperidin, naringin, citrus flavonoids, chlorogenic acid, other similar materials, and combinations thereof.

In particular embodiments, the antioxidant is a catechin such as, for example, epigallocatechin gallate (EGCG). Suitable sources of catechins for embodiments of this disclosure include, but are not limited to, green tea, white tea, black tea, oolong tea, chocolate, cocoa, red wine, grape seed, red grape skin, purple grape skin, red grape juice, purple grape juice, berries, pycnogenol, and red apple peel.

In some embodiments, the antioxidant is chosen from proanthocyanidins, procyanidins or combinations thereof. Suitable sources of proanthocyanidins and procyanidins for embodiments of this disclosure include, but are not limited to, red grapes, purple grapes, cocoa, chocolate, grape seeds, red wine, cacao beans, cranberry, apple peel, plum, blueberry, black currants, choke berry, green tea, sorghum, cinnamon, barley, red kidney bean, pinto bean, hops, almonds, hazelnuts, pecans, pistachio, pycnogenol, and colorful berries.

In particular embodiments, the antioxidant is an anthocyanin. Suitable sources of anthocyanins for embodiments of this disclosure include, but are not limited to, red berries, blueberries, bilberry, cranberry, raspberry, cherry, pomegranate, strawberry, elderberry, choke berry, red grape skin, purple grape skin, grape seed, red wine, black currant, red currant, cocoa, plum, apple peel, peach, red pear, red cabbage, red onion, red orange, and blackberries.

In some embodiments, the antioxidant is chosen from quercetin, rutin or combinations thereof. Suitable sources of quercetin and rutin for embodiments of this disclosure include, but are not limited to, red apples, onions, kale, bog whortleberry, lingonberrys, chokeberry, cranberry, blackberry, blueberry, strawberry, raspberry, black currant, green tea, black tea, plum, apricot, parsley, leek, broccoli, chili pepper, berry wine, and ginkgo.

In some embodiments, the antioxidant is resveratrol. Suitable sources of resveratrol for embodiments of this disclosure include, but are not limited to, red grapes, peanuts, cranberry, blueberry, bilberry, mulberry, Japanese Itadori tea, and red wine.

In particular embodiments, the antioxidant is an isoflavone. Suitable sources of isoflavones for embodiments of this disclosure include, but are not limited to, soy beans, soy products, legumes, alfalfa spouts, chickpeas, peanuts, and red clover.

In some embodiments, the antioxidant is curcumin. Suitable sources of curcumin for embodiments of this disclosure include, but are not limited to, turmeric and mustard.

In particular embodiments, the antioxidant is chosen from punicalagin, ellagitannin or combinations thereof. Suitable sources of punicalagin and ellagitannin for embodiments of this disclosure include, but are not limited to, pomegranate, raspberry, strawberry, walnut, and oak-aged red wine.

In some embodiments, the antioxidant is a citrus flavonoid, such as hesperidin or naringin. Suitable sources of citrus flavonoids, such as hesperidin or naringin, for embodiments of this disclosure include, but are not limited to, oranges, grapefruits, and citrus juices.

In particular embodiments, the antioxidant is chlorogenic acid. Suitable sources of chlorogenic acid for embodiments of this disclosure include, but are not limited to, green coffee, yerba mate, red wine, grape seed, red grape skin, purple grape skin, red grape juice, purple grape juice, apple juice, cranberry, pomegranate, blueberry, strawberry, sunflower, Echinacea, pycnogenol, and apple peel.

In certain embodiments, the functional ingredient is at least one dietary fiber source. As used herein, the at least one dietary fiber source may comprise a single dietary fiber source or a plurality of dietary fiber sources as a functional ingredient for the edible compositions (*e.g*., beverages) provided herein. Generally, according to particular embodiments of this disclosure, the at least one dietary fiber source is present in the edible composition (*e.g*., beverage) in a concentration sufficient to promote health and wellness.

Numerous polymeric carbohydrates having significantly different structures in both composition and linkages fall within the definition of dietary fiber. Such compounds are well known to those skilled in the art, non-limiting examples of which include non-starch polysaccharides, lignin, cellulose, methylcellulose, the hemicelluloses, β-glucans, pectins, gums, mucilage, waxes, inulins, oligosaccharides, fructooligosaccharides, cyclodextrins, chitins, and combinations thereof.

Polysaccharides are complex carbohydrates composed of monosaccharides joined by glycosidic linkages. Non-starch polysaccharides are bonded with β-linkages, which humans are unable to digest due to a lack of an enzyme to break the β-linkages. Conversely, digestable starch polysaccharides generally comprise α(1-4) linkages.

Lignin is a large, highly branched and cross-linked polymer based on oxygenated phenylpropane units. Cellulose is a linear polymer of glucose molecules joined by a β(1-4) linkage, which mammalian amylases are unable to hydrolyze. Methylcellulose is a methyl esther of cellulose that is often used in foodstuffs as a thickener, and emulsifier. It is commercially available (*e.g*., Citrucel by GlaxoSmithKline, Celevac by Shire Pharmaceuticals). Hemicelluloses are highly branched polymers consisting mainly of glucurono- and 4-O-methylglucuroxylans. β-Glucans are mixed-linkage (1-3), (1-4) β-D-glucose polymers found primarily in cereals, such as oats and barley. Pectins, such as beta pectin, are a group of polysaccharides composed primarily of D-galacturonic acid, which is methoxylated to variable degrees.

Gums and mucilages represent a broad array of different branched structures. Guar gum, derived from the ground endosperm of the guar seed, is a galactomannan. Guar gum is commercially available (*e.g*., Benefiber by Novartis AG). Other gums, such as gum arabic and pectins, have still different structures. Still other gums include xanthan gum, gellan gum, tara gum, psylium seed husk gum, and locust been gum.

Waxes are esters of ethylene glycol and two fatty acids, generally occurring as a hydrophobic liquid that is insoluble in water.

Inulins comprise naturally occurring oligosaccharides belonging to a class of carbohydrates known as fructans. They generally are comprised of fructose units joined by β(2-1) glycosidic linkages with a terminal glucose unit. Oligosaccharides are saccharide polymers containing typically three to six component sugars. They are generally found either O- or N-linked to compatible amino acid side chains in proteins or to lipid molecules. Fructooligosaccharides are oligosaccharides consisting of short chains of fructose molecules.

Food sources of dietary fiber include, but are not limited to, grains, legumes, fruits, and vegetables. Grains providing dietary fiber include, but are not limited to, oats, rye, barley, wheat. Legumes providing fiber include, but are not limited to, peas and beans such as soybeans. Fruits and vegetables providing a source of fiber include, but are not limited to, apples, oranges, pears, bananas, berries, tomatoes, green beans, broccoli, cauliflower, carrots, potatoes, celery. Plant foods such as bran, nuts, and seeds (such as flax seeds) are also sources of dietary fiber. Parts of plants providing dietary fiber include, but are not limited to, the stems, roots, leaves, seeds, pulp, and skin.

Although dietary fiber generally is derived from plant sources, indigestible animal products such as chitins are also classified as dietary fiber. Chitin is a polysaccharide composed of units of acetylglucosamine joined by β(1-4) linkages, similar to the linkages of cellulose.

Sources of dietary fiber often are divided into categories of soluble and insoluble fiber based on their solubility in water. Both soluble and insoluble fibers are found in plant foods to varying degrees depending upon the characteristics of the plant. Although insoluble in water, insoluble fiber has passive hydrophilic properties that help increase bulk, soften stools, and shorten transit time of fecal solids through the intestinal tract.

Unlike insoluble fiber, soluble fiber readily dissolves in water. Soluble fiber undergoes active metabolic processing via fermentation in the colon, increasing the colonic microflora and thereby increasing the mass of fecal solids. Fermentation of fibers by colonic bacteria also yields end-products with significant health benefits. For example, fermentation of the food masses produces gases and short-chain fatty acids. Acids produced during fermentation include butyric, acetic, propionic, and valeric acids that have various beneficial properties such as stabilizing blood glucose levels by acting on pancreatic insulin release and providing liver control by glycogen breakdown. In addition, fiber fermentation may reduce atherosclerosis by lowering cholesterol synthesis by the liver and reducing blood levels of LDL and triglycerides. The acids produced during fermentation lower colonic pH, thereby protecting the colon lining from cancer polyp formation. The lower colonic pH also increases mineral absorption, improves the barrier properties of the colonic mucosal layer, and inhibits inflammatory and adhesion irritants. Fermentation of fibers also may benefit the immune system by stimulating production of T-helper cells, antibodies, leukocytes, splenocytes, cytokinins and lymphocytes.

In certain embodiments, the functional ingredient is at least one fatty acid.

As used herein, the at least one fatty acid may be single fatty acid or a plurality of fatty acids as a functional ingredient for the sweetener edible compositions (*e.g.*, beverages) provided herein. Generally, according to particular embodiments of this disclosure, the at least one fatty acid is present in the edible composition (*e.g*., beverage) in a concentration sufficient to promote health and wellness.

As used herein, "fatty acid" refers to any straight chain monocarboxylic acid and includes saturated fatty acids, unsaturated fatty acids, long chain fatty acids, medium chain fatty acids, short chain fatty acids, fatty acid precursors (including omega-9 fatty acid precursors), and esterified fatty acids. As used herein, "long chain polyunsaturated fatty acid" refers to any polyunsaturated carboxylic acid or organic acid with a long aliphatic tail. As used herein, "omega-3 fatty acid" refers to any polyunsaturated fatty acid having a first double bond as the third carbon-carbon bond from the terminal methyl end of its carbon chain. In particular embodiments, the omega-3 fatty acid may comprise a long chain omega-3 fatty acid. As used herein, "omega-6 fatty acid" any polyunsaturated fatty acid having a first double bond as the sixth carbon-carbon bond from the terminal methyl end of its carbon chain.

Suitable omega-3 fatty acids for use in embodiments of the present disclosure can be derived from algae, fish, animals, plants, or combinations thereof, for example. Examples of suitable omega-3 fatty acids include, but are not limited to, linolenic acid, alpha-linolenic acid, eicosapentaenoic acid, docosahexaenoic acid, stearidonic acid, eicosatetraenoic acid and combinations thereof. In some embodiments, suitable omega-3 fatty acids can be provided in fish oils, (e.g., menhaden oil, tuna oil, salmon oil, bonito oil, and cod oil), microalgae omega-3 oils or combinations thereof. In particular embodiments, suitable omega-3 fatty acids may be derived from commercially available omega-3 fatty acid oils such as Microalgae DHA oil (from Martek, Columbia, MD), OmegaPure (from Omega Protein, Houston, TX), Marinol C-38 (from Lipid Nutrition, Channahon, IL), Bonito oil and MEG-3 (from Ocean Nutrition, Dartmouth, NS), Evogel (from Symrise, Holzminden, Germany), Marine Oil, from tuna or salmon (from Arista Wilton, CT), OmegaSource 2000, Marine Oil, from menhaden and Marine Oil, from cod (from OmegaSource, RTP, NC).

Suitable omega-6 fatty acids include, but are not limited to, linoleic acid, gamma-linolenic acid, dihommo-gamma-linolenic acid, arachidonic acid, eicosadienoic acid, docosadienoic acid, adrenic acid, docosapentaenoic acid and combinations thereof.

Suitable esterified fatty acids for embodiments of the present disclosure may include, but are not limited to, monoacylgycerols containing omega-3 and/or omega-6 fatty acids, diacylgycerols containing omega-3 and/or omega-6 fatty acids, or triacylgycerols containing omega-3 and/or omega-6 fatty acids and combinations thereof.

In certain embodiments, the functional ingredient is at least one vitamin. As used herein, the at least one vitamin may be single vitamin or a plurality of vitamins as a functional ingredient for the edible compositions (*e.g*., beverages) provided herein. Generally, according to particular embodiments of this disclosure, the at least one vitamin is present in the edible composition (*e.g*., beverage) in a concentration sufficient to promote health and wellness.

Vitamins are organic compounds that the human body needs in small quantities for normal functioning. The body uses vitamins without breaking them down, unlike other nutrients such as carbohydrates and proteins. To date, thirteen vitamins have been recognized, and one or more can be used in the functional sweetener and sweetened compositions herein. Suitable vitamins include, vitamin A, vitamin D, vitamin E, vitamin K, vitamin B1, vitamin B2, vitamin B3, vitamin B5, vitamin B6, vitamin B7, vitamin B9, vitamin B12, and vitamin C. Many of vitamins also have alternative chemical names, non-limiting examples of which are provided below.

| **Vitamin** | **Alternative names** |
|---|---|
| Vitamin A | Retinol, Retinaldehyde, Retinoic acid, Retinoids, Retinal, Retinoic ester |
| Vitamin D (vitamins D1-D5) | Calciferol, Cholecalciferol, Lumisterol, Ergocalciferol, Dihydrotachysterol, 7-dehydrocholesterol |
| Vitamin E | Tocopherol, Tocotrienol |
| Vitamin K | Phylloquinone, Naphthoquinone |
| Vitamin B1 | Thiamin |
| Vitamin B2 | Riboflavin, Vitamin G |
| Vitamin B3 | Niacin, Nicotinic acid, Vitamin PP |
| Vitamin B5 | Pantothenic acid |
| Vitamin B6 | Pyridoxine, Pyridoxal, Pyridoxamine |
| Vitamin B7 | Biotin, Vitamin H |
| Vitamin B9 | Folic acid, Folate, Folacin, Vitamin M, Pteroyl-L-glutamic acid |
| Vitamin B12 | Cobalamin, Cyanocobalamin |
| Vitamin C | Ascorbic acid |

Various other compounds have been classified as vitamins by some authorities. These compounds may be termed pseudo-vitamins and include, but are not limited to, compounds such as ubiquinone (coenzyme Q10), pangamic acid, dimethylglycine, taestrile, amygdaline, flavanoids, para-aminobenzoic acid, adenine, adenylic acid, and s-methylmethionine. As used herein, the term vitamin includes pseudo-vitamins.

In some embodiments, the vitamin is a fat-soluble vitamin chosen from vitamin A, D, E, K and combinations thereof.

In other embodiments, the vitamin is a water-soluble vitamin chosen from vitamin B1, vitamin B2, vitamin B3, vitamin B6, vitamin B12, folic acid, biotin, pantothenic acid, vitamin C and combinations thereof.

In certain embodiments, the functional ingredient is glucosamine. Generally, according to particular embodiments of this disclosure, glucosamine is present in the edible composition (*e.g.*, beverage) in a concentration sufficient to promote health and wellness.

Glucosamine, also called chitosamine, is an amino sugar that is believed to be an important precursor in the biochemical synthesis of glycosylated proteins and lipids. D-glucosamine occurs naturally in the cartilage in the form of glucosamine-6-phosphate, which is synthesized from fructose-6-phosphate and glutamine. However, glucosamine also is available in other forms, non-limiting examples of which include glucosamine hydrochloride, glucosamine sulfate, N-acetyl-glucosamine, or any other salt forms or combinations thereof. Glucosamine may be obtained by acid hydrolysis of the shells of lobsters, crabs, shrimps, or prawns using methods well known to those of ordinary skill in the art. In a particular embodiment, glucosamine may be derived from fungal biomass containing chitin, as described in U.S. Patent Publication No. 2006/0172392.

The edible composition (*e.g*., beverage) can further comprise chondroitin sulfate.

In certain embodiments, the functional ingredient is at least one mineral.

As used herein, the at least one mineral may be single mineral or a plurality of minerals as a functional ingredient for the edible compositions (*e.g.,* beverages) provided herein. Generally, according to particular embodiments of this disclosure, the at least one mineral is present in the edible composition (*e.g.,* beverage) in a concentration sufficient to promote health and wellness.

Minerals, in accordance with the teachings of this disclosure, comprise inorganic chemical elements required by living organisms. Minerals are comprised of a broad range of compositions (*e.g.,* elements, simple salts, and complex silicates) and also vary broadly in crystalline structure. They may naturally occur in foods and beverages, may be added as a supplement, or may be consumed or administered separately from foods or beverages.

Minerals may be categorized as either bulk minerals, which are required in relatively large amounts, or trace minerals, which are required in relatively small amounts. Bulk minerals generally are required in amounts greater than or equal to about 100 mg per day and trace minerals are those that are required in amounts less than about 100 mg per day.

In particular embodiments of this disclosure, the mineral is chosen from bulk minerals, trace minerals or combinations thereof. Non-limiting examples of bulk minerals include calcium, chlorine, magnesium, phosphorous, potassium, sodium, and sulfur. Non-limiting examples of trace minerals include chromium, cobalt, copper, fluorine, iron, manganese, molybdenum, selenium, zinc, and iodine. Although iodine generally is classified as a trace mineral, it is required in larger quantities than other trace minerals and often is categorized as a bulk mineral.

In other particular embodiments of this disclosure, the mineral is a trace mineral, believed to be necessary for human nutrition, non-limiting examples of which include bismuth, boron, lithium, nickel, rubidium, silicon, strontium, tellurium, tin, titanium, tungsten, and vanadium.

The minerals embodied herein may be in any form known to those of ordinary skill in the art. For example, in a particular embodiment the minerals may be in their ionic form, having either a positive or negative charge. In another particular embodiment the minerals may be in their molecular form. For example, sulfur and phosphorous often are found naturally as sulfates, sulfides, and phosphates.

In certain embodiments, the functional ingredient is at least one preservative. As used herein, the at least one preservative may be single preservative or a plurality of preservatives as a functional ingredient for the edible compositions (e.g., beverages) provided herein. Generally, according to particular embodiments of this disclosure, the at least one preservative is present in the edible composition (e.g., beverage) in a concentration sufficient to promote health and wellness.

In particular embodiments of this disclosure, the preservative is chosen from antimicrobials, antioxidants, antienzymatics or combinations thereof. Non-limiting examples of antimicrobials include sulfites, propionates, benzoates, sorbates, nitrates, nitrites, bacteriocins, salts, sugars, acetic acid, dimethyl dicarbonate (DMDC), ethanol, and ozone.

According to a particular embodiment, the preservative is a sulfite. Sulfites include, but are not limited to, sulfur dioxide, sodium bisulfite, and potassium hydrogen sulfite.

According to another particular embodiment, the preservative is a propionate. Propionates include, but are not limited to, propionic acid, calcium propionate, and sodium propionate.

According to yet another particular embodiment, the preservative is a benzoate. Benzoates include, but are not limited to, sodium benzoate and benzoic acid.

In another particular embodiment, the preservative is a sorbate. Sorbates include, but are not limited to, potassium sorbate, sodium sorbate, calcium sorbate, and sorbic acid.

In still another particular embodiment, the preservative is a nitrate and/or a nitrite. Nitrates and nitrites include, but are not limited to, sodium nitrate and sodium nitrite.

In yet another particular embodiment, the at least one preservative is a bacteriocin, such as, for example, nisin.

In another particular embodiment, the preservative is ethanol.

In still another particular embodiment, the preservative is ozone.

Non-limiting examples of antienzymatics suitable for use as preservatives in particular embodiments of the disclosure include ascorbic acid, citric acid, and metal chelating agents such as ethylenediaminetetraacetic acid (EDTA).

In certain embodiments, the functional ingredient is at least one hydration agent. As used herein, the at least one hydration agent may be single hydration agent or a plurality of hydration agents as a functional ingredient for the edible compositions (*e.g.,* beverages) provided herein. Generally, according to particular embodiments of this disclosure, the at least one hydration agent is present in the edible composition (*e.g.,* beverage) in a concentration sufficient to promote health and wellness.

Hydration products help the body to replace fluids that are lost through excretion. For example, fluid is lost as sweat in order to regulate body temperature, as urine in order to excrete waste substances, and as water vapor in order to exchange gases in the lungs. Fluid loss can also occur due to a wide range of external causes, non-limiting examples of which include physical activity, exposure to dry air, diarrhea, vomiting, hyperthermia, shock, blood loss, and hypotension. Diseases causing fluid loss include diabetes, cholera, gastroenteritis, shigellosis, and yellow fever. Forms of malnutrition that cause fluid loss include the excessive consumption of alcohol, electrolyte imbalance, fasting, and rapid weight loss.

In a particular embodiment, the hydration product is a composition that helps the body replace fluids that are lost during exercise. Accordingly, in a particular embodiment, the hydration product is an electrolyte, non-limiting examples of which include sodium, potassium, calcium, magnesium, chloride, phosphate, bicarbonate, and combinations thereof. Suitable electrolytes for use in particular embodiments of this disclosure are also described in U.S. Patent No. 5,681,569. In particular embodiments, the electrolytes are obtained from their corresponding water-soluble salts. Non-limiting examples of salts for use in particular embodiments include chlorides, carbonates, sulfates, acetates, bicarbonates, citrates, phosphates, hydrogen phosphates, tartates, sorbates, citrates, benzoates, or combinations thereof. In other embodiments, the electrolytes are provided by juice, fruit extracts, vegetable extracts, tea, or teas extracts.

In particular embodiments of this disclosure, the hydration product is a carbohydrate to supplement energy stores burned by muscles. Suitable carbohydrates for use in particular embodiments of this disclosure are described in U.S. Patent Numbers 4,312,856, 4,853,237, 5,681,569, and 6,989,171. Non-limiting examples of suitable carbohydrates include monosaccharides, disaccharides, oligosaccharides, complex polysaccharides or combinations thereof. Non-limiting examples of suitable types of monosaccharides for use in particular embodiments include trioses, tetroses, pentoses, hexoses, heptoses, octoses, and nonoses. Non-limiting examples of specific types of suitable monosaccharides include glyceraldehyde, dihydroxyacetone, erythrose, threose, erythrulose, arabinose, lyxose, ribose, xylose, ribulose, xylulose, allose, altrose, galactose, glucose, gulose, idose, mannose, talose, fructose, psicose, sorbose, tagatose, mannoheptulose, sedoheltulose, octolose, and sialose. Non-limiting examples of suitable disaccharides include sucrose, lactose, and maltose. Non-limiting examples of suitable oligosaccharides include saccharose, maltotriose, and maltodextrin. In other particular embodiments, the carbohydrates are provided by a corn syrup, a beet sugar, a cane sugar, a juice, or a tea.

In another particular embodiment, the hydration is a flavanol that provides cellular rehydration. Flavanols are a class of natural substances present in plants, and generally comprise a 2-phenylbenzopyrone molecular skeleton attached to one or more chemical moieties. Non-limiting examples of suitable flavanols for use in particular embodiments of this disclosure include catechin, epicatechin, gallocatechin, epigallocatechin, epicatechin gallate, epigallocatechin 3-gallate, theaflavin, theaflavin 3-gallate, theaflavin 3'-gallate, theaflavin 3,3' gallate, thearubigin or combinations thereof. Several common sources of flavanols include tea plants, fruits, vegetables, and flowers. In preferred embodiments, the flavanol is extracted from green tea.

In a particular embodiment, the hydration product is a glycerol solution to enhance exercise endurance. The ingestion of a glycerol containing solution has been shown to provide beneficial physiological effects, such as expanded blood volume, lower heart rate, and lower rectal temperature.

In certain embodiments, the functional ingredient is chosen from at least one probiotic, prebiotic and combination thereof. As used herein, the at least one probiotic or prebiotic may be single probiotic or prebiotic or a plurality of probiotics or prebiotics as a functional ingredient for the edible compositions (*e.g.,* beverages) provided herein. Generally, according to particular embodiments of this disclosure, the at least one probiotic, prebiotic or combination thereof is present in the edible composition (*e.g.,* beverage) in a concentration sufficient to promote health and wellness.

Probiotics, in accordance with the teachings of this disclosure, comprise microorganisms that benefit health when consumed in an effective amount. Desirably, probiotics beneficially affect the human body's naturally-occurring gastrointestinal microflora and impart health benefits apart from nutrition. Probiotics may include, without limitation, bacteria, yeasts, and fungi.

According to particular embodiments, the probiotic is a beneficial microorganisms that beneficially affects the human body's naturally-occurring gastrointestinal microflora and imparts health benefits apart from nutrition. Examples of probiotics include, but are not limited to, bacteria of the genus *Lactobacilli, Bifidobacteria, Streptococci,* or combinations thereof, that confer beneficial effects to humans.

In particular embodiments of the disclosure, the at least one probiotic is chosen from the genus *Lactobacilli. Lactobacilli* (i.e., bacteria of the genus *Lactobacillus,* hereinafter "*L*.") have been used for several hundred years as a food preservative and for promoting human health. Non-limiting examples of species of *Lactobacilli* found in the human intestinal tract include *L. acidophilus, L. casei, L. fermentum, L. saliva roes, L. brevis, L. leichmannii, L. plantarum, L. cellobiosus, L. reuteri, L. rhamnosus, L. GG, L. bulgaricus,* and *L. thermophilus,.*

According to other particular embodiments of this disclosure, the probiotic is chosen from the genus *Bifidobacteria. Bifidobacteria* also are known to exert a beneficial influence on human health by producing short chain fatty acids (*e.g.,* acetic, propionic, and butyric acids), lactic, and formic acids as a result of carbohydrate metabolism. Non-limiting species of *Bifidobacteria* found in the human gastrointestinal tract include *B. angulatum, B. animalis, B. asteroides, B. bifidum, B. boum, B. breve, B. catenulatum, B. choerinum, B. coryneforme, B. cuniculi, B. dentium, B. gallicum, B. gallinarum, B indicum, B. longum, B. magnum, B. merycicum, B. minimum, B. pseudocatenulatum, B. pseudolongum, B. psychraerophilum, B. pullorum, B. ruminantium, B. saeculare, B. scardovii, B. simiae, B. subtile, B. thermacidophilum, B. thermophilum, B. urinalis, and B. sp.*

According to other particular embodiments of this disclosure, the probiotic is chosen from the genus *Streptococcus. Streptococcus thermophilus* is a gram-positive facultative anaerobe. It is classified as lactic acid bacteria, commonly found in milk and milk products, and is used in the production of yogurt. Other non-limiting probiotic species of this bacteria include *Streptococcus salivarus* and *Streptococcus cremoris.*

Probiotics that may be used in accordance with this disclosure are well-known to those of skill in the art. Non-limiting examples of foodstuffs comprising probiotics include yogurt, sauerkraut, kefir, kimchi, fermented vegetables, and other foodstuffs containing a microbial element that beneficially affects the host animal by improving the intestinal microbalance.

Prebiotics, in accordance with the teachings of this disclosure, are compositions that promote the growth of beneficial bacteria in the intestines. Prebiotic substances can be consumed by a relevant probiotic, or otherwise assist in keeping the relevant probiotic alive or stimulate its growth. When consumed in an effective amount, prebiotics also beneficially affect the human body's naturally-occurring gastrointestinal microflora and thereby impart health benefits apart from just nutrition. Prebiotic foods enter the colon and serve as substrate for the endogenous bacteria, thereby indirectly providing the host with energy, metabolic substrates, and essential micronutrients. The body's digestion and absorption of prebiotic foods is dependent upon bacterial metabolic activity, which salvages energy for the host from nutrients that escaped digestion and absorption in the small intestine.

Prebiotics, in accordance with the embodiments of this disclosure, include, without limitation, mucopolysaccharides, oligosaccharides, polysaccharides, amino acids, vitamins, nutrient precursors, proteins and combinations thereof.

According to a particular embodiment of this disclosure, the prebiotic is chosen from dietary fibers, including, without limitation, polysaccharides and oligosaccharides. These compounds have the ability to increase the number of probiotics, which leads to the benefits conferred by the probiotics. Non-limiting examples of oligosaccharides that are categorized as prebiotics in accordance with particular embodiments of this disclosure include fructooligosaccharides, inulins, isomalto-oligosaccharides, lactilol, lactosucrose, lactulose, pyrodextrins, soy oligosaccharides, transgalacto-oligosaccharides, and xylo-oligosaccharides.

According to other particular embodiments of the disclosure, the prebiotic is an amino acid. Although a number of known prebiotics break down to provide carbohydrates for probiotics, some probiotics also require amino acids for nourishment.

Prebiotics are found naturally in a variety of foods including, without limitation, bananas, berries, asparagus, garlic, wheat, oats, barley (and other whole grains), flaxseed, tomatoes, Jerusalem artichoke, onions and chicory, greens (*e.g.,* dandelion greens, spinach, collard greens, chard, kale, mustard greens, turnip greens), and legumes (*e.g.,* lentils, kidney beans, chickpeas, navy beans, white beans, black beans).

In certain embodiments, the functional ingredient is at least one weight management agent. As used herein, the at least one weight management agent may be single weight management agent or a plurality of weight management agents as a functional ingredient for the edible compositions (*e.g.,* beverages) provided herein. Generally, according to particular embodiments of this disclosure, the at least one weight management agent is present in the edible composition (*e.g.,* beverage) in a concentration sufficient to promote health and wellness.

As used herein, "a weight management agent" includes an appetite suppressant and/or a thermogenesis agent. As used herein, the phrases "appetite suppressant", "appetite satiation compositions", "satiety agents", and "satiety ingredients" are synonymous. The phrase "appetite suppressant" describes macronutrients, herbal extracts, exogenous hormones, anorectics, anorexigenics, pharmaceutical drugs, and combinations thereof, that when delivered in an effective amount, suppress, inhibit, reduce, or otherwise curtail a person's appetite. The phrase "thermogenesis agent" describes macronutrients, herbal extracts, exogenous hormones, anorectics, anorexigenics, pharmaceutical drugs, and combinations thereof, that when delivered in an effective amount, activate or otherwise enhance a person's thermogenesis or metabolism.

Suitable weight management agents include macronutrient selected from the group consisting of proteins, carbohydrates, dietary fats, and combinations thereof. Consumption of proteins, carbohydrates, and dietary fats stimulates the release of peptides with appetite-suppressing effects. For example, consumption of proteins and dietary fats stimulates the release of the gut hormone cholecytokinin (CCK), while consumption of carbohydrates and dietary fats stimulates release of Glucagon-like peptide 1 (GLP-1).

Suitable macronutrient weight management agents also include carbohydrates. Carbohydrates generally comprise sugars, starches, cellulose and gums that the body converts into glucose for energy. Carbohydrates often are classified into two categories, digestible carbohydrates (e.g., monosaccharides, disaccharides, and starch) and non-digestible carbohydrates (*e.g.,* dietary fiber). Studies have shown that non-digestible carbohydrates and complex polymeric carbohydrates having reduced absorption and digestibility in the small intestine stimulate physiologic responses that inhibit food intake. Accordingly, the carbohydrates embodied herein desirably comprise non-digestible carbohydrates or carbohydrates with reduced digestibility. Non-limiting examples of such carbohydrates include polydextrose; inulin; monosaccharide-derived polyols such as erythritol, mannitol, xylitol, and sorbitol; disaccharide-derived alcohols such as isomalt, lactitol, and maltitol; and hydrogenated starch hydrolysates. Carbohydrates are described in more detail herein below.

In another particular embodiment weight management agent is a dietary fat. Dietary fats are lipids comprising combinations of saturated and unsaturated fatty acids. Polyunsaturated fatty acids have been shown to have a greater satiating power than mono-unsaturated fatty acids. Accordingly, the dietary fats embodied herein desirably comprise poly-unsaturated fatty acids, non-limiting examples of which include triacylglycerols.

In a particular embodiment, the weight management agents is an herbal extract. Extracts from numerous types of plants have been identified as possessing appetite suppressant properties. Non-limiting examples of plants whose extracts have appetite suppressant properties include plants of the genus *Hoodia, Trichocaulon, Caralluma, Stapelia, Orbea, Asclepias,* and *Camelia.* Other embodiments include extracts derived from Gymnema Sylvestre, Kola Nut, Citrus Auran tium, Yerba Mate, Griffonia Simplicifolia, Guarana, myrrh, guggul Lipid, and black current seed oil.

The herbal extracts may be prepared from any type of plant material or plant biomass. Non-limiting examples of plant material and biomass include the stems, roots, leaves, dried powder obtained from the plant material, and sap or dried sap. The herbal extracts generally are prepared by extracting sap from the plant and then spray-drying the sap. Alternatively, solvent extraction procedures may be employed. Following the initial extraction, it may be desirable to further fractionate the initial extract (*e.g.,* by column chromatography) in order to obtain an herbal extract with enhanced activity. Such techniques are well known to those of ordinary skill in the art.

In a particular embodiment, the herbal extract is derived from a plant of the genus *Hoodia,* species of which include *H. alstonii, H. currorii, H. dregei, H. flava, H. gordonii, H. jutatae, H. mossamedensis, H. officinalis, H. parviflorai, H. pedicellata, H. pilifera, H. ruschii,* and *H. triebneri. Hoodia* plants are stem succulents native to southern Africa. A sterol glycoside of *Hoodia,* known as P57, is believed to be responsible for the appetite-suppressant effect of the *Hoodia* species.

In another particular embodiment, the herbal extract is derived from a plant of the genus *Caralluma,* species of which include *C. indica, C. fimbriata, C. attenuate, C. tuberculata, C. edulis, C. adscendens, C. stalagmifera, C. umbellate, C. penicillata, C. russeliana, C. retrospicens, C. Arabica,* and *C. lasiantha. Carralluma* plants belong to the same Subfamily as *Hoodia,* Asclepiadaceae. *Caralluma* are small, erect and fleshy plants native to India having medicinal properties, such as appetite suppression, that generally are attributed to glycosides belonging to the pregnane group of glycosides, non-limiting examples of which include caratuberside A, caratuberside B, bouceroside I, bouceroside II, bouceroside III, bouceroside IV, bouceroside V, bouceroside VI, bouceroside VII, bouceroside VIII, bouceroside IX, and bouceroside X.

In another particular embodiment, the at least one herbal extract is derived from a plant of the genus *Trichocaulon. Trichocaulon* plants are succulents that generally are native to southern Africa, similar to *Hoodia,* and include the species *T. piliferum* and *T. officinale.*

In another particular embodiment, the herbal extract is derived from a plant of the genus *Stapelia* or *Orbea,* species of which include *S. gigantean* and *O*. *variegate,* respectively. Both *Stapelia* and *Orbea* plants belong to the same Subfamily as *Hoodia,* Asclepiadaceae. Not wishing to be bound by any theory, it is believed that the compounds exhibiting appetite suppressant activity are saponins, such as pregnane glycosides, which include stavarosides A, B, C, D, E, F, G, H, I, J, and K.

In another particular embodiment, the herbal extract is derived from a plant of the genus *Asclepias. Asclepias* plants also belong to the Asclepiadaceae family of plants. Non-limiting examples of *Asclepias* plants include *A. incarnate, A. curassayica, A. syriaca,* and *A. tuberose.* Not wishing to be bound by any theory, it is believed that the extracts comprise steroidal compounds, such as pregnane glycosides and pregnane aglycone, having appetite suppressant effects.

In a particular embodiment, the weight management agent is an exogenous hormone having a weight management effect. Non-limiting examples of such hormones include CCK, peptide YY, ghrelin, bombesin and gastrin-releasing peptide (GRP), enterostatin, apolipoprotein A-IV, GLP-1, amylin, somastatin, and leptin.

In another embodiment, the weight management agent is a pharmaceutical drug. Non-limiting examples include phentenime, diethylpropion, phendimetrazine, sibutramine, rimonabant, oxyntomodulin, floxetine hydrochloride, ephedrine, phenethylamine, or other stimulants.

The at least one weight management agent may be utilized individually or in combination as a functional ingredient for the edible compositions (*e.g.,* beverages) provided in this disclosure.

In certain embodiments, the functional ingredient is at least one osteoporosis management agent. As used herein, the at least one osteoporosis management agent may be single osteoporosis management agent or a plurality of osteoporosis management agent as a functional ingredient for the edible compositions (*e.g.,* beverages) provided herein. Generally, according to particular embodiments of this disclosure, the at least one osteoporosis management agent is present in the edible composition (*e.g.,* beverage) in a concentration sufficient to promote health and wellness.

Osteoporosis is a skeletal disorder of compromised bone strength, resulting in an increased risk of bone fracture. Generally, osteoporosis is characterized by reduction of the bone mineral density (BMD), disruption of bone micro-architecture, and changes to the amount and variety of non-collagenous proteins in the bone.

In certain embodiments, the osteoporosis management agent is at least one calcium source. According to a particular embodiment, the calcium source is any compound containing calcium, including salt complexes, solubilized species, and other forms of calcium. Non-limiting examples of calcium sources include amino acid chelated calcium, calcium carbonate, calcium oxide, calcium hydroxide, calcium sulfate, calcium chloride, calcium phosphate, calcium hydrogen phosphate, calcium dihydrogen phosphate, calcium citrate, calcium malate, calcium citrate malate, calcium gluconate, calcium tartrate, calcium lactate, solubilized species thereof, and combinations thereof.

According to a particular embodiment, the osteoporosis management agent is a magnesium soucrce. The magnesium source is any compound containing magnesium, including salt complexes, solubilized species, and other forms of magnesium. Non-limiting examples of magnesium sources include magnesium chloride, magnesium citrate, magnesium gluceptate, magnesium gluconate, magnesium lactate, magnesium hydroxide, magnesium picolate, magnesium sulfate, solubilized species thereof, and mixtures thereof. In another particular embodiment, the magnesium source comprises an amino acid chelated or creatine chelated magnesium.

In other embodiments, the osteoporosis agent is chosen from vitamins D, C, K, their precursors and/or beta-carotene and combinations thereof.

Numerous plants and plant extracts also have been identified as being effective in the prevention and treatment of osteoporosis. Not wishing to be bound by any theory, it is believed that the plants and plant extracts stimulates bone morphogenic proteins and/or inhibits bone resorption, thereby stimulating bone regeneration and strength. Non-limiting examples of suitable plants and plant extracts as osteoporosis management agents include species of the genus *Taraxacum* and *Amelanchier,* as disclosed in U.S. Patent Publication No. 2005/0106215, and species of the genus *Lindera, Artemisia, Acorus, Carthamus, Carum, Cnidium, Curcuma, Cyperus, Juniperus, Prunus, Iris, Cichorium, Dodonaea, Epimedium, Erigonoum, Soya, Mentha, Ocimum, thymus, Tanacetum, Plantago, Spearmint, Bixa, Vitis, Rosemarinus, Rhus,* and *Anethum,* as disclosed in U.S. Patent Publication No. 2005/0079232.

In certain embodiments, the functional ingredient is at least one phytoestrogen. As used herein, the at least one phytoestrogen may be single phytoestrogen or a plurality of phytoestrogens as a functional ingredient for the edible compositions (e.g., beverages) provided herein. Generally, according to particular embodiments of this disclosure, the at least one phytoestrogen is present in the edible compositions (e.g., beverages) in a concentration sufficient to promote health and wellness.

Phytoestrogens are compounds found in plants which can typically be delivered into human bodies by ingestion of the plants or the plant parts having the phytoestrogens. As used herein, "phytoestrogen" refers to any substance which, when introduced into a body causes an estrogen-like effect of any degree. For example, a phytoestrogen may bind to estrogen receptors within the body and have a small estrogen-like effect.

Examples of suitable phytoestrogens for embodiments of this disclosure include, but are not limited to, isoflavones, stilbenes, lignans, resorcyclic acid lactones, coumestans, coumestroI, equol, and combinations thereof. Sources of suitable phytoestrogens include, but are not limited to, whole grains, cereals, fibers, fruits, vegetables, black cohosh, agave root, black currant, black haw, chasteberries, cramp bark, dong quai root, devil's club root, false unicorn root, ginseng root, groundsel herb, licorice, liferoot herb, motherwort herb, peony root, raspberry leaves, rose family plants, sage leaves, sarsaparilla root, saw palmetto berried, wild yam root, yarrow blossoms, legumes, soybeans, soy products (*e.g.,* miso, soy flour, soymilk, soy nuts, soy protein isolate, tempen, or tofu) chick peas, nuts, lentils, seeds, clover, red clover, dandelion leaves, dandelion roots, fenugreek seeds, green tea, hops, red wine, flaxseed, garlic, onions, linseed, borage, butterfly weed, caraway, chaste tree, vitex, dates, dill, fennel seed, gotu kola, milk thistle, pennyroyal, pomegranates, southernwood, soya flour, tansy, and root of the kudzu vine (pueraria root) and the like, and combinations thereof.

Isoflavones belong to the group of phytonutrients called polyphenols. In general, polyphenols (also known as "polyphenolics"), are a group of chemical substances found in plants, characterized by the presence of more than one phenol group per molecule.

Suitable phytoestrogen isoflavones in accordance with embodiments of this disclosure include genistein, daidzein, glycitein, biochanin A, formononetin, their respective naturally occurring glycosides and glycoside conjugates, matairesinol, secoisolariciresinol, enterolactone, enterodiol, textured vegetable protein, and combinations thereof.

Suitable sources of isoflavones for embodiments of this disclosure include, but are not limited to, soy beans, soy products, legumes, alfalfa spouts, chickpeas, peanuts, and red clover.

In certain embodiments, the functional ingredient is at least one long chain primary aliphatic saturated alcohol. As used herein, the at least one long chain primary aliphatic saturated alcohol may be single long chain primary aliphatic saturated alcohol or a plurality of long chain primary aliphatic saturated alcohols as a functional ingredient for the edible compositions (*e.g.,* beverages) provided herein. Generally, according to particular embodiments of this disclosure, the at least one long chain primary aliphatic saturated alcohol is present in the edible composition (*e.g.*, beverage) in a concentration sufficient to promote health and wellness.

Long-chain primary aliphatic saturated alcohols are a diverse group of organic compounds. The term alcohol refers to the fact these compounds feature a hydroxyl group (-OH) bound to a carbon atom. The term primary refers to the fact that in these compounds the carbon atom which is bound to the hydroxyl group is bound to only one other carbon atom. The term saturated refers to the fact that these compounds feature no carbon to carbon pi bonds. The term aliphatic refers to the fact that the carbon atoms in these compounds are joined together in straight or branched chains rather than in rings. The term long-chain refers to the fact that the number of carbon atoms in these compounds is at least 8 carbons).

Non-limiting examples of particular long-chain primary aliphatic saturated alcohols for use in particular embodiments of the disclosure include the 8 carbon atom 1-octanol, the 9 carbon 1-nonanol, the 10 carbon atom 1-decanol, the 12 carbon atom 1-dodecanol, the 14 carbon atom 1-tetradecanol, the 16 carbon atom 1-hexadecanol, the 18 carbon atom 1-octadecanol, the 20 carbon atom 1-eicosanol, the 22 carbon 1-docosanol, the 24 carbon 1-tetracosanol, the 26 carbon 1-hexacosanol, the 27 carbon 1-heptacosanol, the 28 carbon 1-octanosol, the 29 carbon 1-nonacosanol, the 30 carbon 1-triacontanol, the 32 carbon 1-dotriacontanol, and the 34 carbon 1-tetracontanol.

In a particularly desirable embodiment of the disclosure, the long-chain primary aliphatic saturated alcohols are policosanol. Policosanol is the term for a mixture of long-chain primary aliphatic saturated alcohols composed primarily of 28 carbon 1-octanosol and 30 carbon 1-triacontanol, as well as other alcohols in lower concentrations such as 22 carbon 1-docosanol, 24 carbon 1-tetracosanol, 26 carbon 1-hexacosanol, 27 carbon 1-heptacosanol, 29 carbon 1-nonacosanol, 32 carbon 1-dotriacontanol, and 34 carbon 1-tetracontanol.

Long-chain primary aliphatic saturated alcohols are derived from natural fats and oils. They may be obtained from these sources by using extraction techniques well known to those of ordinary skill in the art. Policosanols can be isolated from a variety of plants and materials including sugar cane (*Saccharum officinarium*), yams (*e.g. Dioscorea opposite),* bran from rice (*e.g. Oryza sativa),* and beeswax. Policosanols may be obtained from these sources by using extraction techniques well known to those of ordinary skill in the art. A description of such extraction techniques can be found in U.S. Pat. Appl. No. 2005/0220868.

In certain embodiments, the functional ingredient is at least one phytosterol, phytostanol or combination thereof. Generally, according to particular embodiments of this disclosure, the at least one phytosterol, phytostanol or combination thereof is present in the edible composition (*e.g.,* beverage) in a concentration sufficient to promote health and wellness.

As used herein, the phrases "stanol", "plant stanol" and "phytostanol" are synonymous.

Plant sterols and stanols are present naturally in small quantities in many fruits, vegetables, nuts, seeds, cereals, legumes, vegetable oils, bark of the trees and other plant sources. Although people normally consume plant sterols and stanols every day, the amounts consumed are insufficient to have significant cholesterol-lowering effects or other health benefits. Accordingly, it would be desirable to supplement food and beverages with plant sterols and stanols.

Sterols are a subgroup of steroids with a hydroxyl group at C-3. Generally, phytosterols have a double bond within the steroid nucleus, like cholesterol; however, phytosterols also may comprise a substituted sidechain (R) at C-24, such as an ethyl or methyl group, or an additional double bond. The structures of phytosterols are well known to those of skill in the art.

At least 44 naturally-occurring phytosterols have been discovered, and generally are derived from plants, such as corn, soy, wheat, and wood oils; however, they also may be produced synthetically to form compositions identical to those in nature or having properties similar to those of naturally-occurring phytosterols. According to particular embodiments of this disclosure, non-limiting examples of phytosterols well known to those or ordinary skill in the art include 4-desmethyl sterols (e.g., β-sitosterol, campesterol, stigmasterol, brassicasterol, 22-dehydrobrassicasterol, and Δ5-avenasterol), 4-monomethyl sterols, and 4,4-dimethyl sterols (triterpene alcohols) (e.g., cycloartenol, 24-methylenecycloartanol, and cyclobranol).

As used herein, the phrases "stanol", "plant stanol" and "phytostanol" are synonymous. Phytostanols are saturated sterol alcohols present in only trace amounts in nature and also may be synthetically produced, such as by hydrogenation of phytosterols. According to particular embodiments of this disclosure, non-limiting examples of phytostanols include β-sitostanol, campestanol, cycloartanol, and saturated forms of other triterpene alcohols.

Both phytosterols and phytostanols, as used herein, include the various isomers such as the α and β isomers (*e.g*., α-sitosterol and β-sitostanol, which comprise one of the most effective phytosterols and phytostanols, respectively, for lowering serum cholesterol in mammals).

The phytosterols and phytostanols of the present disclosure also may be in their ester form. Suitable methods for deriving the esters of phytosterols and phytostanols are well known to those of ordinary skill in the art, and are disclosed in U.S. Patent Numbers 6,589,588, 6,635,774, 6,800,317, and U.S. Patent Publication Number 2003/0045473. Non-limiting examples of suitable phytosterol and phytostanol esters include sitosterol acetate, sitosterol oleate, stigmasterol oleate, and their corresponding phytostanol esters. The phytosterols and phytostanols of the present disclosure also may include their derivatives.

Generally, the amount of functional ingredient in the edible composition (*e.g.,* beverage) varies widely depending on the particular edible composition (*e.g.,* beverage) and the desired functional ingredient. Those of ordinary skill in the art will readily ascertain the appropriate amount of functional ingredient for each beverage.

In some embodiments, the edible composition further comprises a solubilizing agent, as discussed herein. Compounds - such as sweet taste modulators - have a particular solubility in aqueous solutions. As would be evident to one of skill in the art, the solubility of a compound depends on a number of factors including, but not limited to, the chemical structure of the compound, the solvent, the pH of the solvent, etc. Solubilizing agents may be used to increase the amount of a compound, such as a sweet taste modulator, or the combination of sweet taste modulators, that may be dissolved in a particular amount of solvent.

Methods for solubilizing compounds of the present invention include but are not limited to chemical, physical or mechanical means. Additives, solubilizing or stabilizing agents may provide chemical means for increasing the concentration of compounds of the present invention in solution. Application of mechanical forces resulting in shearing, dispersion or emulsification of compounds of the present invention may also result in an increase in the concentration of compounds of the present invention in solution. Changes in temperature, pressure, and/or pH are non-limiting physical means for increasing the solubility of compounds of the present invention and/or maintaining the concentration of the compound in solution. The mechanical, physical or chemical means may be used in combination, in the presence or absence of cosolvents, surfactant systems, complexing agents and also including self-assembling nanomicelles, nanosuspensions, micronization and cocrystallizations. The methods as well as the importance of increasing the solubility of compounds in solution are well known in the art, for example, "Drug solubility: importance and enhancement techniques", ISRN Pharmaceutics, volume 2012, article ID 195727, Ketan T. Savjani, Anuradha K. Gajjar and Jignasa K. Savjani.

Solubilizing agents include, but are not limited to, glycoprotein-polysaccharides, such as Gum Arabic; homopolymers, such as poly(N-vinyl-pyrrolidone); medium chain mono- and diglycerides, such as Capmul MCM; oligosaccharides, such as Hp-beta-cyclodextrin, alpha-cyclodextrin, beta-cyclodextrin or gamma-cyclodextrin, and cellulose; polyglycerol esters, such as Caprol PEG 860®, Caprol 10G40® or Drewpol 10-1-CC®; polysorbates, such as Tween 20® (polysorbate 20), Tween 60® (polysorbate 60), and Tween 80® (polysorbate 80); and saponin/triterpene glycoside, such as quillaja saponin or Q-NATURALE®. For example, solubilizing agents include, but are not limited to, GRINDSTED® ACETEM, alpha-Cyclodextrin, beta-Cyclodextrin, DATEM, Decaglycerol dioleate, Decaglycerol monooleate, Decaglycerol monostearate, Ethoxylated monoglyceride, gamma-Cyclodextrin, Glycerol monoleate, Glycerol monostearate, Glyerol dioleate, Gum Arabic, Hexaglycerol dioleate, Hp-beta-Cyclodextrin, Lecithin, Methyl cellulose, Oleic acid, Poly(N-vinyl-pyrrolidone), Polyoxyethylene (20) sorbitan monooleate, Polyoxyethylene (20) sorbitan monopalmitate, Polyoxyethylene (20) sorbitan monostearate, Polyoxyethylene (20) sorbitan trioleate, Polyoxyethylene (20) sorbitan tristearate, Polysaccharides, polysorbate 20, polysorbate 60, polysorbate 80, Potassium oleate, Propylene glycol monostearate, Propylene glycol monolaurate, Quillaja saponins, Sodium lauryl sulfate, Sodium oleate, Sodium stearoyllactylate, Sorbitan monolaurate, Sorbitan trioleate, Sorbitan tristearate, Sorbitan monooleate, Sorbitan monostearate, Sucrose monoester, or Sucrose monolaurate. In some embodiments, the solubilizing agent is alpha-Cyclodextrin, beta-Cyclodextrin, gamma-Cyclodextrin, Gum Arabic, Hp-beta-Cyclodextrin, Lecithin, Methyl cellulose, Poly(N-vinyl-pyrrolidone), or Quillaja saponins (Q-NATURALE®). In some embodiments, the solubilizing agent is alpha-Cyclodextrin. In some embodiments, the solubilizing agent is beta-Cyclodextrin. In some embodiments, the solubilizing agent is gamma-Cyclodextrin. In some embodiments, the solubilizing agent is Gum Arabic. In some embodiments, the solubilizing agent is Hp-beta-Cyclodextrin. In some embodiments, the solubilizing agent is Lecithin. In some embodiments, the solubilizing agent is Methyl cellulose. In some embodiments, the solubilizing agent is Poly(N-vinyl-pyrrolidone). In some embodiments, the solubilizing agent is Quillaja saponins. Solubilizing agents may be used at concentrations between 0.001% to 50% to solubilize the compounds of the invention. In some embodiments, concentrations of solubilizing agents in the final product range from about 0.05% to about 2%.

Solvents for dissolving the sweet taste modulator of the combination of sweet taste modulators of the invention include, but are not limited to, 1,3-butylene glycol, amyl acetate, benzyl alcohol, butane-1,3-diol, castor oil, diethyl tartrate, diethylene glycol monoethyl ether, ethyl acetate, ethyl alcohol, glycerin, glycerol, glycerol diacetate, isopropyl alcohol, NEOBEE® M-5 oil, propylene glycol, and triacetin. In some embodiments, the solvent is 1,3-butylene glycol. In some embodiments, the solvent is amyl acetate. In some embodiments, the solvent is benzyl alcohol. In some embodiments, the solvent is butane-1,3-diol. In some embodiments, the solvent is castor oil. In some embodiments, the solvent is diethyl tartrate. In some embodiments, the solvent is diethylene glycol monoethyl ether. In some embodiments, the solvent is ethyl acetate. In some embodiments, the solvent is ethyl alcohol. In some embodiments, the solvent is glycerin. In some embodiments, the solvent is glycerol. In some embodiments, the solvent is glycerol diacetate. In some embodiments, the solvent is isopropyl alcohol. In some embodiments, the solvent is propylene glycol. In some embodiments, the solvent is triacetin. Solvents may be used at concentrations between 0.001% to 50% to solubilize the compounds of the invention. In some embodiments, solvent concentrations in the final product range from about 0.05 to about 2%.

In some embodiments, the edible composition further comprises a surfactant to increase or decrease the effectiveness of the compounds, or the combination of compounds, of the present disclosure as sweet taste modulators. Suitable surfactants include, but are not limited to, non-ionic surfactants (*e.g.,* mono and diglycerides, fatty acid esters, sorbitan esters, propylene glycol esters, and lactylate esters) anionic surfactants (*e.g.,* sulfosuccinates and lecithin) and cationic surfactants (*e.g.,* quaternary ammonium salts).

The rate of release of the sweet taste modulator, or the combination of sweet taste modulators, of the present disclosure may be regulated. The release rate of the sweet taste modulator, or the combination of sweet taste modulators, of the present disclosure can be altered by, for example, varying its solubility in water. Rapid release can be achieved by encapsulating the sweet taste modulator, or the combination of sweet taste modulators, of the present disclosure with a material with high water solubility. Delayed release of the sweet taste modulator, or the combination of sweet taste modulators, of the present disclosure can be achieved by encapsulating the sweet taste modulator, or the combination of sweet taste modulators, of the present disclosure with a material with low water solubility. The sweet taste modulator, or the combination of sweet taste modulators, of the present disclosure can be co-encapsulated with carbohydrates or masking tastants such as sweeteners. The rate of release of the sweet taste modulator, or the combination of sweet taste modulators, of the present disclosure can also be regulated by the degree of encapsulation. In some embodiments, the sweet taste modulator, or the combination of sweet taste modulators, of the present disclosure is fully encapsulated. In other embodiments, the compounds, or the combination of compounds, of the present disclosure are partially encapsulated. In some embodiments, the rate of release may be regulated so as to release with the sweet taste modulator, or the combination of sweet taste modulators. In some embodiments, the rate of release may be regulated in a manner that is dependent on the structure of the sweetener and sweet taste modulator, or the combination of sweet taste modulators.

The edible compositions of this disclosure are prepared according to techniques well-known in the art. In general, an edible composition of the disclosure is prepared by mixing a component or ingredient of the edible composition, such as a sweetener, with a sweet taste modulating compound, or a combination of sweet taste modulators, of the disclosure. Alternatively, a sweet taste modulating compound, or a combination of sweet taste modulators, of the disclosure can be added directly to the edible composition comprising a sweetener. In some embodiments, a sweetener is added simultaneously or sequentially with a sweet taste modulating compound, or a combination of sweet taste modulators, of the disclosure. If sequentially, the sweetener may be added before or after the sweet taste modulating compound, or the combination of sweet taste modulators, of the disclosure. In some embodiments, the edible composition is a food product. In some embodiments, the edible composition is a pharmaceutical composition. In some embodiments, the edible composition is a consumer product. In some embodiments, the edible composition is in the form of, for example, a gum, lozenge, sauce, condiment, meat matrix, meat slurry, paste, suspension, spread, coating, a liquid, a gel, an emulsion, granules, or seasoning.

The amount of both a sweet taste modulating compound, or a combination of sweet taste modulators, of the present disclosure and a sweetener used in an edible composition depends upon a variety of factors, including the purpose of the composition and the desired or acceptable perception of sweetness. The amount may depend on the nature of the edible composition, the particular compound or combination of compounds added, the sweetener, other compounds present in the composition, the method of preparation (including amount of heat used), and the pH of the edible composition. Those of skill in the art will know how to determine the amounts needed to produce the desired taste(s).

When the edible compositions are formulated for ingestion via the oral cavity, a sweet taste modulating compound, or the combination of sweet taste modulating compounds, of the disclosure may be present at any of the concentrations effective for modulating the sweetness of a sweetener listed above.

In some embodiments, the edible compositions are formulated as a concentrate, which is intended for dilution prior to consumption. Such concentrates include syrups, frozen concentrates, dry mixes, and food additives. When present in a concentrate, the sweet taste modulating compound of Formula (I), Compound 1, as described herein, or a comestibly or biologically acceptable salt, solvate, or enantiomer thereof, or a combination of any of the foregoing compounds, are present at a concentration about 2-fold, about 3-fold, about 4-fold, about 5-fold, about 6-fold, about 7-fold, about 8-fold, about 9-fold, about 10-fold, about 15-fold, about 20-fold, about 25-fold, about 30-fold, about 35-fold, about 40-fold, about 45-fold, about 50-fold, about 55-fold, about 60-fold, about 65-fold, about 70-fold, about 75-fold, about 80-fold, about 85-fold, about 90-fold, about 95-fold, about 100-fold, about 150-fold, about 200-fold, about 250-fold, about 300-fold, about 350-fold, about 400-fold, about 450-fold, about 500-fold, about 550-fold, about 600-fold, about 650-fold, about 700-fold, about 750-fold, about 800-fold, about 850-fold, about 900-fold, about 950-fold, or about 1000-fold above any of the effective concentrations discussed herein. Accordingly, the sweet taste modulating compounds, or the combination of sweet taste modulating compounds, may be present in a concentrate - for later dilution - at concentration between about 1 ppm and about 5000 ppm. In some embodiments, the sweet taste modulator of the present disclosure (e.g., a compound of Formula (I), or Compound 1, or a comestibly or biologically acceptable salt, solvate, or enantiomer thereof, or a combination of any of the foregoing compounds) may be present in a concentrate at a concentration between about 1 ppm to about 3000 ppm; about 10 ppm to about 1000 ppm; about 50 ppm to about 500 ppm; about 50 ppm to about 250 ppm; about 50 ppm to about 100 ppm; about 100 ppm to about 500 ppm; about 100 ppm to about 1000 ppm; about 100 ppm to about 3000 ppm; about 500 ppm to about 1000 ppm; about 500 ppm to about 3000 ppm; or about 1000 ppm to about 3000 ppm. In some embodiments, the sweet taste modulator of the present disclosure (e.g., a compound of Formula (I), or Compound 1, or a comestibly or biologically acceptable salt, solvate, or enantiomer thereof, or a combination of any of the foregoing compounds) may be present in a concentrate for dilution at a concentration of about 50 ppm to about 3000 ppm, about 50 ppm to about 1000 ppm or about 50 ppm to about 500 ppm. In additional embodiments, the sweet taste modulator, or the combination of sweet taste modulators, of the present disclosure (*e.g.,* a compound of Formula (I), or Compound 1, or a comestibly or biologically acceptable salt, solvate, or enantiomer thereof, or a combination of any of the foregoing compounds) may be present in a concentrate for dilution at a concentration of about 1 ppm to about 500 ppm; about 1 ppm to about 250 ppm; about 1 ppm to about 100 ppm; about 1 ppm to about 50 ppm; about 10 ppm to about 500 ppm; about 10 ppm to about 250 ppm; about 10 ppm to about 100 ppm; about 50 ppm to about 500 ppm; about 50 ppm to about 250 ppm; or about 50 ppm to about 100 ppm.

The sweet taste modulator, or the combination of sweet taste modulators, of the present disclosure (*e.g.,* a compound of Formula (I), or Compound 1, or a comestibly or biologically acceptable salt, solvate, or enantiomer thereof, or a combination of any of the foregoing compounds) may be present in a concentrate for dilution at a concentration of about 50 ppm, about 75 ppm, about 100 ppm, about 150 ppm, about 200 ppm, about 250 ppm, about 300 ppm, about 350 ppm, about 400 ppm, about 450 ppm, about 500 ppm, about 550 ppm, about 600 ppm, about 650 ppm, about 700 ppm, about 750 ppm, about 800 ppm, about 850 ppm, about 900 ppm, about 950 ppm, about 1000 ppm, about 1050 ppm, about 1100 ppm, about 1150 ppm, about 1200 ppm, about 1250 ppm, about 1300 ppm, about 1350 ppm, about 1400 ppm, about 1450 ppm, about 1500 ppm, about 1550 ppm, about 1600 ppm, about 1650 ppm, about 1700 ppm, about 1750 ppm, about 1800 ppm, about 1850 ppm, about 1900 ppm, about 1950 ppm, about 2000 ppm, about 2050 ppm, about 2100 ppm, about 2150 ppm, about 2200 ppm, about 2250 ppm, about 2300 ppm, about 2350 ppm, about 2400 ppm, about 2450 ppm, about 2500 ppm, about 2550 ppm, about 2600 ppm, about 2650 ppm, about 2700 ppm, about 2750 ppm, about 2800 ppm, about 2850 ppm, about 2900 ppm, about 2950 ppm, or about 3000 ppm.

In some embodiments, the edible composition further comprises a sweet taste improving composition.

Preferred auxiliaries or carriers include maltodextrin, starch, natural or artificial polysaccharides and/or vegetable gums such as modified starches or gum arabic, coloring agents, for example permitted foodstuff dyes, coloring plant extracts, stabilizers, preservatives, antioxidants, viscosity-influencing substances

The edible compositions may be included in a package. Optionally, the edible composition is packaged in bulk, in which the package contains more of the compositions than would typically be used for a single dish or serving of food or beverage. Such bulk packages can be in the form of paper, plastic, or cloth bags or cardboard boxes or drums. Such bulk packages may be fitted with plastic or metal spouts to facilitate the dispensing of the edible composition.

The package may contain an edible composition comprising a sweet taste modulating compound, or a combination of sweet taste modulating compounds, of the present disclosure and a sweetener. In some embodiments, the package contains an edible composition comprising a sweet taste modulating compound, or a combination of sweet taste modulating compounds, of the present disclosure and caloric carbohydrate sweetener. In some embodiments, the package contains an edible composition comprising a sweet taste modulating compound, or a combination of sweet taste modulating compounds, of the present disclosure and glucose, fructose, sucrose, or a mixture thereof. In some embodiments, the package contains an edible composition comprising a sweet taste modulating compound, or a combination of sweet taste modulating compounds, of the present disclosure and a synthetic sweetener. In some embodiments, the package contains an edible composition comprising a sweet taste modulating compound, or a combination of sweet taste modulating compounds, of the present disclosure and a natural high-potency sweetener.

The edible compositions may be used for medicinal or hygienic purposes, for example, in mouthwash, medicines, pharmaceuticals, cough syrup, throat spray, toothpaste, dental adhesives, tooth whiteners, glues (*e.g.,* on stamps and envelopes), and toxins used in insect and rodent control.

In some embodiments of the disclosure, the sweetener composition is in a form of a tabletop sweetener composition comprising at least one sweet taste modulator according to Formula (I), Compound 1, or a comestibly or biologically acceptable salt, solvate, or enantiomer thereof, or a combination of any of the foregoing compounds, at least one sweetener, at least one bulking agent, and optionally at least one sweet taste improving composition and/or anti-caking agent with improved temporal and/or flavor profile.

For example, suitable "bulking agents" include, but are not limited to maltodextrin (10 DE, 18 DE, or 5 DE), corn syrup solids (20 or 36 DE), sucrose, fructose, glucose, invert sugar, sorbitol, xylose, ribulose, mannose, xylitol, mannitol, galactitol, erythritol, maltitol, lactitol, isomalt, maltose, tagatose, lactose, inulin, glycerol, propylene glycol, polyols, polydextrose, fructooligosaccharides, cellulose and cellulose derivatives, and mixtures thereof. Additionally, the at least one bulking agent is chosen from, granulated sugar (sucrose) or other caloric sweeteners such as crystalline fructose, other carbohydrates, and sugar alcohols. In one embodiment, a bulking agent may be used as a sweet taste improving composition.

As used herein the phrase "anti-caking agent" is understood to mean any composition which prevents, reduces, inhibits, or suppresses at least one sweetener molecule from attaching, binding, or contacting to another sweetener molecule. Alternatively, "anti-caking agent" may refer to any composition which assists in content uniformity and uniform dissolution. In accordance with some embodiments, non-limiting examples of anti-caking agents include cream of tartar, calcium silicate, silicon dioxide, microcrystalline cellulose (Avicel, FMC BioPolymer, Philadelphia, Pa.), and tricalcium phosphate. In at least one embodiment, the anti-caking agents are present in the tabletop sweetener composition in an amount from about 0.001 to about 3% by weight of the tabletop sweetener composition.

Tabletop sweetener compositions may be embodied and packaged in numerous different forms, and may be of any form known in the art. For example, and not by way of limitation, the tabletop sweetener compositions may be in the form of powders, granules, packets, tablets, sachets, pellets, cubes, solids, or liquids.

### Method of preparing an edible composition

According to another aspect, the disclosure provides a method of preparing an edible composition. The method comprises: (a) providing a sweetener; and (b) adding to the sweetener of (a) a compound of Formula (I), or Compound 1, as described herein, or a comestibly or biologically acceptable salt, solvate, or enantiomer thereof, or a combination of any of the foregoing compounds. In some embodiments, the sweet taste modulator of the disclosure has been solubilized prior to the addition step (b). In other embodiments, a solubilizing agent is added to the composition. In some embodiments, the sweetener of step (a) is provided in a comestibly acceptable carrier. The skilled artisan will appreciate that method steps (a) and (b) can be performed in any order - *i.e.,* the method may comprise: (a) adding a compound of Formula (I), or Compound 1, as described herein, or a comestibly or biologically acceptable salt, solvate, or enantiomer thereof, or a combination of any of the foregoing compounds; and (b) adding a sweetener to the Compound(s) of (a).

In general, the method of preparing an edible composition of the disclosure comprises mixing a component or ingredient of the edible composition, such as a sweetener, with a sweet taste modulating compound, or a combination of sweet taste modulating compounds, of the disclosure. Alternatively, a sweet taste modulating compound, or a combination of sweet taste modulating compounds, of the disclosure can be added directly to the edible composition comprising a sweetener. In some embodiments, the sweetener is added to the edible composition simultaneously or sequentially with a sweet taste modulating compound, or a combination of sweet taste modulating compounds, of the disclosure. If sequentially, the sweetener may be added before or after the sweet taste modulating compound, or a combination of sweet taste modulating compounds, of the disclosure. When solubilizing agents are utilized, the method includes the addition of the solubilizing agent at any point. For example, if the composition compromises three components - the sweetener, the sweet taste modulating agent, or a combination of sweet taste modulating agents, and the solubilizing compound - the solubilizing compound may be added as the first, second, or third component. The solubilizing agent may also be added concurrently with any other component.

In some embodiments, the methods of preparing an edible composition further comprise adding at least one additional additive, such as a sweet taste improving composition, and/or a sweet taste improving additive.

The edible compositions may be a food product, a pharmaceutical composition, or a consumer product. In some embodiments, the edible composition is in the form of, for example, a gum, lozenge, sauce, condiment, meat matrix, meat slurry, paste, suspension, spread, coating, a liquid, a gel, an emulsion, granules, or seasoning.

### Method of enhancing or potentiating the perception of sweet taste

According to another aspect, the disclosure provides a method of enhancing the perception of sweet taste in a subject. The method comprises the use of an edible composition of the present disclosure, where the edible composition comprises a compound according to Formula (I), or Compound 1, as described herein, or a comestibly or biologically acceptable salt, solvate, or enantiomer thereof, or a combination of any of the foregoing compounds. Optionally, the edible composition comprises (i) a compound according to Formula (I), or Compound 1, as described herein, or a comestibly or biologically acceptable salt, solvate, or enantiomer thereof, or a combination of any of the foregoing compounds; (ii) a sweetener; and optionally (iii) a solubilizing agent.

The terms "perception of a sweet taste," "perception of sweetness," "perception of a flavor" and similar terms, refer to the awareness of a subject of a particular taste or flavor.

The term "subject" refers to a mammal. In preferred embodiments, the subject is human. In some embodiments, a subject is a domestic or laboratory animal, including but not limited to, household pets, such as dogs, cats, pigs, rabbits, rats, mice, gerbils, hamsters, guinea pigs, and ferrets. In some embodiments, a subject is a livestock animal. Non-limiting examples of livestock animals include: alpaca, bison, camel, cattle, deer, pigs, horses, llamas, mules, donkeys, sheep, goats, rabbits, reindeer, and yak.

The method can be used to enhance or potentiate sweet taste in any edible composition, including a foodstuff, food product, pharmaceutical composition or consumer product. The edible composition may be in any form. In some embodiments, the composition is in the form of, for example, a gum, lozenge, sauce, condiment, paste, suspension, spread, coating, a liquid, a gel, an emulsion, granules, or seasoning.

The edible composition may be utilized by, for example, placement in the oral cavity or by ingestion. In some embodiments, the edible composition comprising a sweet taste modulator, or a combination of sweet taste modulators, of the disclosure is placed in the oral cavity or ingested before an edible composition, such as a foodstuff, a food product, pharmaceutical composition or consumer product, comprising a sweetener, while in other embodiments, the edible composition comprising a sweet taste modulator, or a combination of sweet taste modulators, of the disclosure is placed in the oral cavity or ingested after a sweet food stuff, food product, pharmaceutical composition or consumer product. In other embodiments, the edible composition comprising a sweet taste modulator, or a combination of sweet taste modulators, of the disclosure is placed in the oral cavity or ingested concurrently with a sweet food stuff, food product, pharmaceutical composition or consumer product, either as a separate edible composition or by incorporation in the sweet food stuff, food product, pharmaceutical composition or consumer product. For example, a sweet taste modulator, or a combination of sweet taste modulators, of the disclosure can be combined with foodstuffs or food products to enhance or potentiate the sweet taste of a foodstuff or food product. Alternatively, a sweet taste modulator, or a combination of sweet taste modulators, of the disclosure can be used, for example, in a lozenge or gum for use after exposure to a sweet food stuff, food product, pharmaceutical composition or consumer product (*e.g.,* to enhance or potentiate a sweet aftertaste).

### Method of reducing the amount of a sweetener in an edible composition

It may be desirable to reduce the amount of a caloric sweetener in an edible composition to reduce the calorie content of that edible composition. It may also be desirable to decrease the amount of a synthetic sweetener or a non-natural high potency sweetener in an edible composition to decrease an undesirable taste or aftertaste associated with the synthetic sweetener or non-natural high potency sweetener. Accordingly, another aspect of the present disclosure provides a method of reducing the amount of a sweetener in an edible composition, such as a food product, a pharmaceutical composition or a consumer product. An amount of the sweetener in the edible composition may be replaced with a sweet taste modulator according to Formula (I), Compound 1, or a comestibly or biologically acceptable salt, solvate, or enantiomer thereof, or a combination of any of the foregoing compounds. In such methods, an amount of solubilizing agent may also be added to the edible composition, as described herein.

The term "replace" or "replacing" refers to substituting one compound for another compound in or in the preparation of, for example, an edible composition, such as food product. It includes complete and partial replacements or substitutions.

In some embodiments, the method comprises: (a) replacing an amount of a sweetener used in preparing an edible composition with an amount of a sweet taste modulator according to Formula (I), Compound 1, or a comestibly or biologically acceptable salt, solvate, or enantiomer thereof, or a combination of any of the foregoing compounds. In some embodiments, the sweet taste modulating compound of the disclosure is added in the form of an edible composition comprising the sweet taste modulator, or a combination of sweet taste modulators, of the disclosure. In such methods, an amount of solubilizing agent may also be added to the edible composition, as described herein.

In some embodiments, the method of reducing the amount of a sweetener in an edible composition comprises the steps of: (a) ingesting a first edible composition, in which the amount of a sweetener has been reduced; and (b) ingesting a second edible compound, which comprises a sweet taste modulator compound, or a combination of sweet taste modulators, of the present disclosure. In some embodiments, the first edible composition is ingested before the second edible composition. In some embodiments, the first edible composition is ingested after the second edible composition. In some embodiments, the first edible composition is ingested concurrently with the second edible composition. In such methods, an amount of solubilizing agent may also be added along with the sweet taste modulator, or a combination of sweet taste modulators, of the present disclosure.

In some embodiments, the edible composition is a food product. In some embodiments, the edible composition is a pharmaceutical composition. In some embodiments, the edible composition is a consumer product. The sweetener being replaced may be a natural caloric sweetener, a natural high-potency sweetener, a synthetic sweetener, or combinations thereof. When the sweetener being replaced is a natural caloric sweetener, the sweetener may be sucrose, fructose, glucose, erythritol, high fructose corn/starch syrup, and mixtures thereof. When the sweetener being replaced is a synthetic sweetener, the sweetener may be sucralose, aspartame, potassium acesulfame, and mixtures thereof. The method also comprises replacing an amount of a natural caloric sweetener with a synthetic or natural high-potency sweetener and a sweet taste modulating compound, or a combination of sweet taste modulating compounds, of the present disclosure, such that any off-taste or after taste associated with the synthetic or natural high-potency sweetener is reduced or eliminated. In such embodiments, the "sweetener replaced" is the natural caloric sweetener.

In some embodiments, the methods of reducing sugar intake of a subject further comprise the step of identifying a subject in need thereof. The skilled worker would be able to identify a subject in need of reducing sugar intake. Non-limiting examples of such subjects include subjects that suffer from any one or more of the following disorders: diabetes, pre-diabetes, insulin resistance, obesity, excessive weight, and hyperglycemia.

In some embodiments, the amount of the sweetener replaced in the edible composition in step (a) is an amount sufficient to maintain or restore the health of a subject. For example, the amount of the sweetener replaced in the edible composition may be an amount sufficient to decrease diabetes, pre-diabetes, insulin resistance, obesity, excessive weight, and hyperglycemia in a subject. The amount of the sweetener replaced may be up to 1%, 2%, 3%, 4%, 5%, 6%, 7%, 8%, 9%, 10%, 15%, 20%, 25%, 30%, 35%, 40%, 45%, 50%, 55%, 60%, 65%, 70% 75%, 80%, 85%, 90%, 95% or 99%. These amounts are not meant to be limiting, and increments between the recited percentages are specifically envisioned as part of the disclosure.

In some embodiments, the amount of the sweet taste modulating compound, or the combination of sweet taste modulating compounds, added in step (b) is effective to enhance the perception of sweet taste in the subject.

In some embodiments, the amount of the sweet taste modulating compound, or the combination of sweet taste modulating compounds, added in step (b) is sufficient to permit replacement of up to 1%, 2%, 3%, 4%, 5%, 6%, 7%, 8%, 9%, 10%, 15%, 20%, 25%, 30%, 35%, 40%, 45%, 50%, 55%, 60%, 65%, 70% 75%, 80%, 85%, 90%, 95% or 99% of the amount of sweetener present in the edible composition. These amounts are not meant to be limiting, and increments between the recited percentages are specifically envisioned as part of the disclosure. In some embodiments, the amount of the sweet taste modulating compound, or the combination of sweet taste modulating compounds, added in step (b) is sufficient to permit replacement of up to 25% of the amount of the sweetener present in the edible composition. In other embodiments, the amount of the sweet taste modulating compound, or the combination of sweet taste modulating compounds, added in step (b) is sufficient to permit replacement of up to 50% of the amount of the sweetener present in the edible composition. In other embodiments, the amount of the sweet taste modulating compound, or the combination of sweet taste modulating compounds, added in step (b) is sufficient to permit replacement of up to 75% of the amount of the sweetener present in the edible composition. In yet other embodiments, the amount of the sweet taste modulating compound, or the combination of sweet taste modulating compounds, added in step (b) is sufficient to permit replacement of up to 99% of the amount of the sweetener present in the edible composition.

In some embodiments, the method of reducing the amount of a sweetener in an edible composition further comprises adding at least one additional additive, such as a sweet taste improving composition, and/or a sweet taste improving additive.

### Method of reducing caloric intake

Another aspect of the disclosure provides a method of reducing caloric intake of a subject. In some embodiments, the method comprises the step of providing an edible composition to the subject, wherein all or a portion of a natural caloric sweetener in the edible composition is replaced with (a) a sweet taste modulating compound according to Formula (I), or Compound 1, as described herein or a comestibly or biologically acceptable salt, solvate, or enantiomer thereof, or a combination of any of the foregoing compounds; or (b) one or more synthetic or natural high potency sweeteners and a sweet taste modulating compound according to Formula (I), or Compound 1, as described herein or a comestibly or biologically acceptable salt, solvate, or enantiomer thereof, or a combination of any of the foregoing compounds. The edible composition may be a food product, a pharmaceutical composition, or a consumer product. In such methods, an amount of solubilizing agent may also be added to the edible composition, as described herein.

The methods of reducing caloric intake of a subject may further comprise the step of identifying a subject in need thereof. The skilled worker would be able to identify a subject in need of reducing sugar intake. Non-limiting examples of such subjects include subjects that suffer from any one or more of the following disorders: diabetes, pre-diabetes, insulin resistance, obesity, excessive weight, and hyperglycemia.

In some embodiments, the amount of the natural caloric sweetener replaced in the edible composition is an amount sufficient to maintain or restore the health of a subject. For example, the amount of the natural caloric sweetener replaced in the edible composition may be an amount sufficient to result in weight loss in a subject. Alternatively, the amount of the natural caloric sweetener replaced in the edible composition may be an amount sufficient to alleviate the effects of, or treat, a disease associated with sugar consumption or excessive weight of the subject (*e.g.,* diabetes). In some embodiments, the amount of the natural caloric sweetener replaced in the edible composition is up to 1%, 2%, 3%, 4%, 5%, 6%, 7%, 8%, 9%, 10%, 15%, 20%, 25%, 30%, 35%, 40%, 45%, 50%, 55%, 60%, 65%, 70% 75%, 80%, 85%, 90%, 95% or 99%. These amounts are not meant to be limiting, and increments between the recited percentages are specifically envisioned as part of the disclosure. In some embodiments, the present method results in the subject's daily natural caloric sweetener intake being less than 250 g/day, less than 200 g/day, less than 175 g/day, less than 150 g/day, less than 125 g/day, less than 100 g/day, less than 75 g/day, less than 50 g/day, less than 25 g/day, less than 20 g/day, less than 15 g/day, less than 10 g/day, or less than 25 g/day.

In some embodiments, the amount of sweet taste modulating compound, or the combination of sweet taste modulating compounds, of the disclosure added to the edible composition is sufficient to permit reduction of a subject's natural caloric sweetener intake by up to 1%, 2%, 3%, 4%, 5%, 6%, 7%, 8%, 9%, 10%, 15%, 20%, 25%, 30%, 35%, 40%, 45%, 50%, 55%, 60%, 65%, 70% 75%, 80%, 85%, 90%, 95% or 99%. These amounts are not meant to be limiting, and increments between the recited percentages are specifically envisioned as part of the disclosure. In some embodiments, the amount of sweet taste modulating compound, or the combination of sweet taste modulating compounds, of the disclosure added to the edible composition is sufficient to permit reduction of a subject's natural caloric sweetener intake by up to 25%. In other embodiments, the amount of sweet taste modulating compound, or the combination of sweet taste modulating compounds, of the disclosure added to the edible composition is sufficient to permit reduction of a subject's natural caloric sweetener intake by up to 50%. In other embodiments, the amount of sweet taste modulating compound, or the combination of sweet taste modulating compounds, of the disclosure added to the edible composition is sufficient to permit reduction of a subject's natural caloric sweetener intake by up to 75%. In yet other embodiments, the amount of sweet taste modulating compound, or the combination of sweet taste modulating compounds, of the disclosure added to the edible composition is sufficient to permit reduction of a subject's natural caloric sweetener intake by up to 99%.

In some embodiments, the method of reducing the amount of a sweetener in an edible composition further comprises adding at least one additional additive, such as a sweet taste improving composition, and/or a sweet taste improving additive.

### Method of enhancing sweet taste of an edible composition

According to another embodiment, the disclosure provides methods of enhancing or potentiating the sweet taste in an edible composition. The edible composition may be a food product, a pharmaceutical composition, or a consumer product.

In one embodiment, the method comprises: (a) adding an effective amount of a sweet taste modulating compound according to Formula (I), or Compound 1, as described herein or a comestibly or biologically acceptable salt, solvate, or enantiomer thereof, or a combination of any of the foregoing compounds, to an edible composition comprising a sweetener such that the perception of sweetness intensity of the sweetener is enhanced. In some embodiments, the sweetener is either added to the edible composition before the sweet taste modulating compound, or the combination of sweet taste modulating compounds; concurrently with the sweet taste modulating compound, or the combination of sweet taste modulating compounds; or after the sweet taste modulating compound, or the combination of sweet taste modulating compounds. In other embodiments, the sweetener is naturally or inherently present in the edible composition when the sweet taste modulating compound, or the combination of sweet taste modulating compounds, is added. In such methods, an amount of solubilizing agent may also be added to the edible composition, as described herein.

Alternatively, the method comprises: (a) ingesting an effective amount of a sweet taste modulating compound according to Formula (I), or Compound 1, as described herein, or a comestibly or biologically acceptable salt, solvate, or enantiomer thereof, or a combination of any of the foregoing compounds, before, along with, or after the edible composition such that the perception of sweetness intensity of the sweetener is enhanced. In such methods, an amount of solubilizing agent may also be added to the edible composition, as described herein.

The edible composition may comprise a sweetener, such as a natural caloric sweetener, a natural high-potency sweetener, a synthetic sweetener, or combinations thereof. When the sweetener is a natural caloric sweetener, the sweetener may be sucrose, fructose, glucose, erythritol, high fructose corn/starch syrup, and mixtures thereof. When the sweetener is a synthetic sweetener, the sweetener may be sucralose, aspartame, potassium acesulfame, and mixtures thereof.

In some embodiments, the perception of sweetness intensity of the sweetener is enhanced by up to 1%, 2%, 3%, 4%, 5%, 6%, 7%, 8%, 9%, 10%, 15%, 20%, 25%, 30%, 35%, 40%, 45%, 50%, 55%, 60%, 65%, 70% 75%, 80%, 85%, 90%, 95% or 100%. In some embodiments, the perception of sweetness intensity of the sweetener is enhanced beyond 100%, for example, by 125%, 150%, 175%, 200%, 225%, 250%, 275%, 300%, 325%, 350%, 375%, 400%, 425%, 450%, 475%, 500% or increments in between those recited. These amounts are not meant to be limiting, and increments between the recited percentages are specifically envisioned as part of the disclosure. In some embodiments, the perception of sweetness intensity of the sweetener is enhanced by up to 25%. In other embodiments, the perception of sweetness intensity of the sweetener is enhanced by up to 50%. In other embodiments, the perception of sweetness intensity of the sweetener is enhanced by up to 75%. In other embodiments, the perception of sweetness intensity of the sweetener is enhanced by up to 100%. In some embodiments, the perception of sweetness intensity of the sweetener is enhanced by about 5% to about 100%, about 5% to about 90%, about 5% to about 80%, about 5% to about 70%, about 5% to about 60%, about 5% to about 50%, about 5% to about 40%, about 5% to about 30%, about 10% to about 30%, about 10% to about 25%, about 20% to about 80%, about 20% to about 70%, about 20% to about 60%, about 20% to about 50%, about 20% to about 40%, about 20% to about 30%, about 25% to about 80%, about 25% to about 70%, about 25% to about 60%, about 25% to about 50%, about 25% to about 40%, or about 25% to about 30%. These amounts are not meant to be limiting, and increments between the recited percentages are specifically envisioned as part of the disclosure.

In some embodiments, the method of enhancing the sweet taste attributed to a sweetener in an edible composition further comprises adding at least one additional additive, such as a sweet taste improving composition, and/or a sweet taste improving additive.

### Method of enhancing activation of a sweet taste receptor

Another aspect of the disclosure provides a method of enhancing or potentiating activation and/or signaling of a sweet taste receptor. In some embodiments, the method comprises contacting a sweet taste receptor with a compound according to Formula (I), or Compound 1, as described herein, or a comestibly or biologically acceptable salt, solvate, or enantiomer thereof, or a combination of any of the foregoing compounds, in the presence of a sweetener.

In some embodiments, the method comprises contacting a sweet taste receptor with an edible composition comprising a compound according to Formula (I), or Compound 1, as described herein, or a comestibly or biologically acceptable salt, solvate, or enantiomer thereof, or a combination of any of the foregoing compounds, in the presence of a sweetener. The edible composition may be a food product, a pharmaceutical composition, or a consumer product. In such methods, an amount of solubilizing agent may also be present in the edible composition, as described herein.

In various embodiments, the sweet taste receptor is an *ex vivo* or *in vivo* receptor present in, for example, an assay. The sweet taste receptor also may be an *in vivo* receptor present in a subject. In such embodiments, the sweet taste receptor is typically present in the oral cavity or gastrointestinal tract of the subject. In some embodiments, the sweet receptor is in the oral cavity of a human. Alternatively, the sweet receptor is in the oral cavity of a non-human animal, such as an animal model.

An *in vivo* sweet responsive assay means an assay where an assessment of increased perception of sweetness can be ascribed. Such an assay may be, for example, but not limited to, a human sensory descriptive analysis panel, a human sensory discriminative panel, and/or an expert flavorist assessment. Non-human assessments of sweet response include, but not limited to, operant conditioned animal studies of sweetness taste perception and/or lick rate/amount bottle preference tests

An *in vitro* sweet responsive assay refers to an assay where an assessment of increased sweet response or interaction can be ascribed. An example of such an assay may be, but is not limited to, *in vitro* receptor binding assays, *in vitro* receptor cell-based assays, and/or electronic tongue taste analysis.

In some embodiments, enhancement of a sweet taste receptor activation will affect a physiological process or condition. Non-limiting examples of physiological processes and conditions affected by the enhancement of sweet taste receptor activation include sweet taste, effects on the gastrointestinal tract, appetite, nutrition, nutrient absorption, satiety, hunger, diabetes, obesity, blood glucose levels, blood glucose regulation, metabolism, diet, and eating disorders.

### Methods and Preparations for Blocking or Masking Unpleasant Taste

In addition to sweetness modulation, compounds of Formula (I), Compound 1 or a comestibly or biologically acceptable salt, solvate, or enantiomer thereof, or a combination of any of the foregoing compounds, can be suitable for masking or reducing an unpleasant taste sensation, in particular a bitter taste sensation of a bitter-tasting substance. Compounds of Formula (I), Compound 1 or a comestibly or biologically acceptable salt, solvate, or enantiomer thereof, or a combination of any of the foregoing compounds, can both synergistically enhance the sweet taste sensation of sweeteners like steviolglycosides (for example occurring naturally in Stevia ssp.) and mask or reduce the bitter taste sensation of such steviolglycosides or other bitter tasting sweeteners such as acesulfame-K, saccharin, steviosides, or rebaudiosides. Preparations comprising the bitter blocking compounds or the combination of bitter blocking compounds of the disclosure can be made in the same or similar manner and concentration as the preparations comprising the sweet taste enhancing preparations of the disclosure.

### Preparation of the Compounds of the Invention

In one embodiment, Compound 1 is prepared by the multi-step sequences described below. One of skill in the art would be able to readily adapt the described conditions for the synthesis of any of the compounds of Formula (I).

### 1: Preparation of 5-hydroxy-4-(4-hydroxy-3-methoxyphenyl)-7-methylchroman-2-one:

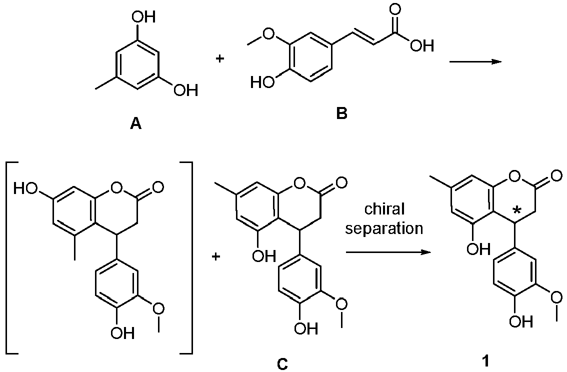

Orcinol **A** (12.4 g, 100.0 mmol) and ferulic acid **B** (19.4 g, 100.0 mmol) were suspended in 1,4-dioxane (100 mL) in a 250 mL flask at room temperature. Concentrated sulfuric acid (98%, 5 mL) was added slowly over 15 minutes. The suspension was heated to 110 °C overnight. The dark hot solution was poured to ice-water and extracted with ethyl acetate/diethyl ether (2:1) twice. The combined organic phase was dried over sodium sulfate and evaporated under vacuum to give a solid, which was purified with a 330 g column with 0 to 40% ethyl acetate in hexane to give 23.6 g of a mixture of two regioisomers (1;1 ratio by LC-MS). The solid mixture was taken up in DMSO and separated by HPLC using a preparative C18 column (250 mm×50 mm, Xbridge, Waters, USA) with an isocratic gradient of water/MeCN (60/40, v/v, 0.1% formic acid added to water) at 100ml\min flow rate. The desired regioisomer was collected between 6.5min and 7.5min. Pure fractions were combined and evaporated under vacuum to remove MeCN. The aqueous suspension was extracted with ethyl acetate twice. Combined organic phase was dried over sodium sulfate and evaporated under vacuum to give Compound **C** as white solid. LC-MS (retention time: 2.28 min, 301 [M+1]).¹H NMR (DMSO-D₆): 9.87 (s, 1H), 8.92 (s, 1H), 6.80 (d, J = 1.9 Hz, 1H), 6.64 (d, J = 8.0 Hz, 1H), 6.51 (s, 1H), 6.46 (s, 1H), 6.32 (dd, J = 8.1, 2.0 Hz, 1H), 4.44 (d, J = 6.1 Hz, 1H), 3.72 (s, 3H), 2.84-3.19 (m, 2H), 2.26 (s, 3H).

2.83 g of racemic sample of Compound **C** was separated by SFC chiral chromatography to provide 1.3 g of enantiomer, Compound **1** (chiral column: CHIRALPAK AD-H 25x3 cm, isopropanol/CO₂ (20/80), 100 bar, flow rate: 31.5 mL/min) with >98% ee, Compound 2 [α]²⁰_{D} = +20 (c = 1.0 mg/mL, ethanol). Compound **1** is (+)-5-hydroxy-4-(4-hydroxy-3-methoxyphenyl)-7-methylchroman-2-one and was determined to be (R)-5-hydroxy-4-(4-hydroxy-3-methoxyphenyl)-7-methylchroman-2-one by crystallography.

### Examples

In order that this disclosure be more fully understood, the following examples are set forth. These examples are for the purpose of illustration only and are not to be construed as limiting the scope of the disclosure in any way.

The test compounds used in the following examples may be obtained from the synthetic methods disclosed above.

The taste test panelists used in the Example 1 were selected using a sensory acuity screening program. Candidate taste panelists were recruited, with prescreening and personal interviews, and were assessed and for their ability to detect, recognize and differentiate basic taste attributes or mixtures thereof as part of a standardized acuity test. This included basic tastes of sweet, sour, salty, bitter and umami, as well as having the capacity to focus on specific aspects of sensory character such as ranking the various tastes perceived and identifying the basic tastes presented at threshold levels.

Due to the complex nature of taste perception in subjects and the inherently subjective nature of the following experiments, individual taste test trials may yield different results for a given compound. The data presented in the following Examples is illustrative of the taste testing results observed.

The taste testing experiments below were conducted with panels of varying size (*i.e*., panels comprising varying numbers of panelists).

### Example 1: 2 Alternative Forced Choice

### Effect of test compounds on the perception of sweet taste of high fructose corn syrup (HFCS) two-alternative forced choice method (2AFC).

The effect of the test compounds on the perception of the sweet taste of an aqueous solution of sweetener in humans was evaluated using a two-alternative forced choice "sip and spit" method (2AFC).

### Preparation of Samples for sensory taste tests:

The aqueous sweetener solutions were prepared by adding sucrose equivalent (SE) quantities of high fructose corn syrup (HFCS) (by weight), to water to achieve the desired concentration. The aqueous solutions ranged in pH from 2.5-pH 7. Compounds were first prepared as a 500-fold concentrated stock solution in 100% ethanol. These concentrated stocks were then added to the aqueous sweetener solutions to result in a final ethanol concentration of 0.2%. The control (positive and negative) samples were also normalized to 0.2% ethanol. This level of ethanol was not seen to contribute any perceived sweetness.

### Sensory Methodology: Assessment of sweetness perception using 2AFC method

The 2AFC test used for compound evaluation was a double blinded and randomized test where taste panelists (n≥15) evaluate a pair of sweetener solutions at a time - one sample contains aqueous sweetener solution plus compound (*i.e.* test) while the other contains aqueous sweetener solution at higher concentration without compound *(i.e.* positive control). Each test sample was compared against a positive control. For example, if the test sample contained IX sweetener concentration with compound (*e.g*. 5brix HFCS with compound), the positive control sample contained a 1.1X sweetener concentration (*e.g*. 5.5brix HFCS without compound). In this manner, compounds were not assessed simply for an increase in perceived sweetness, but a significant increase in perceived sweetness above the positive control.

Panelists were instructed not to eat or drink (except water) for at least 1h before the test. During the test, panelists were instructed to sip each sample, swirl it around their mouth and then expectorate. After tasting each sample in the pair, panelists were instructed to record the sample that is "sweeter" in taste. Panelists cleansed their palates by rinsing with water, eating a cracker and waiting for an interval of about 5 minutes. All samples were tasted at ambient temperatures. Data were analyzed using binomial probabilities. The results of the 2AFC analysis are presented in Table 1, below.

**Table 1. Sensory Data Summary 2AFC assessment of increase in perceived sweetness**

| **Compound No.** | **Sweetener Concentration for Compound Containing Sample (Test sample)** | **Sweetener Concentration of positive control surprassed by test sample** | **Compound concentration with positive 2AFC results (ppm)** | **Fold increase in perceived sweetnes** |
|---|---|---|---|---|
| **1** | 5brix HFCS | 7.5brix HFCS | 15 | >1.5X |

Approximate levels of sweetness enhancement in column 5 of Table 2 were calculated as follows:
Approximate levels of sweetness enhancement = Sweetener concentration of positive control surpassed by the test sample, divided by sweetener concentration for compound-containing sample (*e.g*. 1.5X = 7.5brix HFCS/5brix HFCS). This only indicates that the perceived increase is greater than the positive control, but does not indicate the degree with which the positive control concentration was surpassed.

### Example 2: Inherent sweetness

### Inherent Sweetness Assessment

### Preparation of Samples for sensory taste tests:

The aqueous sweetener solutions were prepared by adding sucrose equivalent quantities of HFCS (by weight), to water to achieve the desired concentration. The aqueous solutions ranged in pH from 2.5-pH 7.

Compounds were first prepared as a 500-fold concentrated stock solution in 100% ethanol. These concentrated stocks were then added to the aqueous sweetener solutions to result in a final ethanol concentration of 0.2%. The control (positive and negative) samples were also normalized to 0.2% ethanol. This level of ethanol was not seen to contribute any perceived sweetness

### Sensory Methodology: Inherent Sweetness Assessment using 2AFC method

The intrinsic effect of test compounds on the perception of sweet taste, in an aqueous solution, in humans was evaluated using 2AFC methodology. This test was a double-blinded, randomized study where taste panelists (n≥20) evaluate a pair of sweetener solutions at a time- one sample contains aqueous solutions, without sweetener, plus compound (*i.e.* test) while the other contains aqueous solution containing 1.5% sucrose equivalent (SE), HFCS (*i.e.* control). Each test sample is compared against the sweetener control to observe if the test is significantly *less sweet* than the control sample.

Panelists were instructed not to eat or drink (except water) for at least 1h before the test. During the test, panelists were instructed to sip each sample, swirl it around their mouth and then expectorate. After tasting each sample in the pair, panelists were instructed to record the sample that is "sweeter" in taste. Panelists cleansed their palates by rinsing with water, eating a cracker and waiting for an interval of about 5 minutes. Each pair was tasted twice. All samples were tasted at ambient temperatures. Data were analyzed using binomial probabilities

The results of the inherent sweetness analysis are presented in Table 2, below.

**Table 2: Sensory Data: Inherent Sweetness Assessment**

| **Compound No.** | **Compound concentration tested (ppm)** | **Number of total panelists** | **Number picking control** | **Reverse P value (contol)** |
|---|---|---|---|---|
| 1 | 15 | 20 | 15 | 0.021 |

For the compound listed in Table 2: The control sample of 1.5% SE HFCS is significantly sweeter than the test sample.

### Example 3: Descriptive Analysis

### Effect of test compounds on the perception of sweet taste in humans using a trained descriptive analysis

### (DA) panel

The effect of the test compound on the perception of the sweet taste in a flavored aqueous environment, in the presence of carbohydrate sweeteners, was evaluated using a descriptive analysis methodology with a group of trained panelists, as follows.

Candidate panelists were recruited, with prescreening and personal interviews, and were assessed for their ability to detect, recognize and differentiate basic taste attributes or mixtures thereof as part of a standardized acuity test. These candidate panelists were also assessed for their innate ability to identify flavors, and to rank on intensity scales. Other senses such as smell and vision were also included as part of the assessment. Candidates also were screened for their ability to use language to describe and articulate ideas about their taste, smell and visual perception of samples.

Selected candidates proceeded to training as a group in three phases: (1) Lexicon development, (2) Concept alignment, and (3) Scaling descriptors. During lexicon development, panelists evaluated products appropriate for use in the study to generate and align on terms describing the flavor, taste, aromatic, trigeminal, and temporal attributes. During concept alignment, the panel evaluated the products mentioned above to clarify and confirm the attributes that were generated during lexicon development using reference standards that appropriately define each attribute; these are either physical references (*e.g.* sucrose solutions) or verbal descriptions (*e.g.* overall flavor). Product terms and concepts were validated during this portion of the study.

In the last phase, scaling descriptors, the panel participated in a series of exercises focused on ordering and ranking samples according to relative attribute intensity, measuring attribute intensity using a defined length of line scale. Whenever possible, different levels or concentration of references were used as anchors to facilitate use of the scale. Panelists were provided with blinded references at this stage to evaluate their understanding and perception of the scale.

The panel used in this example was trained to reference sweet attributes in an absolute manner, such that a particular concentration of sweetener is always measured by the panelists at the same point using a 15cm line scale where each cm represents a 1% increase in sucrose to cover the range of 0% to 15% sucrose. For the rest of the attributes, the panel was trained using a hybrid approach between descriptive analysis methods well-known to those skilled in the art. Panel performance was measured at regular intervals, and for data presented, the cohort of 12 panelists demonstrated good performance for reproducibility measured as a panel average of 82 %, and defined as the number of attributes with reproducibility comparable to group performance (p<0.05), discrimination measured as a panel average of 87%, and defined as the number of attributes significantly different at a 90% confidence level individually (p<0.1), and agreement measured with a cohort average of 75% and defined as the number of attributes that correlate well to the panel consensus (R>0.7).

The trained panel was then used for a descriptive analysis assessment for Compound in flavored lemon lime matrix. Compound was assessed at a dose of 20ppm for sweetness enhancement in high fructose corn syrup (HFCS) sweetened lemon lime matrix.

The flavored sweetener solutions were prepared by adding sucrose equivalent quantities of HFCS (by weight), to lemon lime matrix to achieve the desired concentration. Compound was first prepared as a 500-fold concentrated stock solution in 100% ethanol. The concentrated stocks was then added to the flavored sweetener solutions to result in a final ethanol concentration of 0.2%. The control (positive and negative) samples were also normalized to 0.2% ethanol. This level of ethanol was not seen to contribute any perceived sweetness

Both control (flavored sweetener solutions without compound) and variant (flavored sweetener solutions with compound) samples were blinded and randomized. Samples were presented in monadic order and panelists were instructed to sip the sample, swirl it in their mouths and expectorate. A total of 7 attributes including sweet, bitter, sour, licorice and thickness were assessed for each sample. For the sweet attribute, panelists made an absolute scoring on a 15cm line scale where each cm represents a 1% increase in sucrose to cover the range of 0% to 15% sucrose. After each sample assessment, panelists performed a standard palate cleansing protocol, and observed an inter-sample interval time (ISI). All samples were evaluated at ambient temperatures.

Data was collected and exported electronically utilizing FIZZ sensory software. Data analysis was conducted using SENPAQ version 5.0 software that uses tools such as ANOVA, Fisher's LSD, correlation to determine panel performance as well as significant differences between samples and attributes.
Illustrative results of this DA assessment are presented in Table 3.

**Table 3. DA assessment of sweetness enhancing effects of Compound 2 in lemon lime flavored matrix**

| Sample | Sweetener Concentration in Lemon Lime matrix | Compound 1 | Average Sweetness Intensity Score w/ANOVA (on 15 cm scale) | Standard Error | Fold enhancement of sweetness perception as compared to control sample |
|---|---|---|---|---|---|
| 1 (negative control) | 6.67brix HFCS | | 7.2^{c} | 0.09 | 0.76X |
| 2 (variant) | 6.67brix HFCS | @ 20ppm | 10.5^{a} | 0.30 | 1.11X |
| 3 (positive control) | 10brix HFCS | | 9.4^{b} | 0.12 | 1.0X |

Sweetness intensities observed for compound-containing solution (*i.e. variant*) was significantly different from control solutions, as determined by ANOVA. Fold enhancement (increase in perceived sweetness) was calculated by dividing the DA panel average intensity score observed for a variant sample by that observed for the negative control sample.

### Effect of test compounds on the perception of non-sweet taste attributes in humans using a trained descriptive analysis (DA) panel

The effect of the test compound on the perception of non-sweet taste attributes (such as bitterness) in a flavored aqueous environment, in the presence of carbohydrate sweeteners, was evaluated using a descriptive analysis methodology with a group of trained panelists, as follows. This occurred as part of DA assessment described previously in Example 3.

Candidate panelists were recruited, and trained as described previously, in Example 3, above.

The trained panel was then used for a descriptive analysis assessment for Compound 1 in lemon lime flavored matrix, sweetened with high fructose corn syrup (HFCS), at a test dose of 20 ppm.

All control and variant samples were prepared as described in Example 3. Both control (flavored sweetener solutions without compound) and variant (flavored sweetener solutions with compound) samples were blinded and randomized. Samples were presented in monadic order and panelists were instructed to sip the sample, swirl it in their mouths and expectorate. A total of 7 attributes including sweet, bitter, sour, licorice and astringent were assessed for each sample. After each sample assessment, panelists performed a standard palate cleansing protocol, and observed an inter-sample interval time (ISI). All samples were evaluated at ambient temperatures.

Data was collected and exported electronically utilizing FIZZ sensory software. Data analysis was conducted using SENPAQ version 5.0 software that uses tools such as ANOVA, Fisher's LSD, correlation to determine panel performance as well as significant differences between samples and attributes.

Illustrative results of this DA assessment are presented as sensory Spider Plots in Figure 1.

## Claims

1. An edible composition comprising a sweetener and a compound of Formula (I): 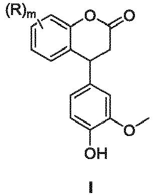 where R is -OH, C₁-C₄ alkyl, -CO₂H, acyl or formyl; m is 2 or 3; and at least one R is not OH; or a comestibly acceptable salt, enantiomer, or solvate thereof, or compound 1 having the structure:
| | |
|---|---|
| Compound 1 | 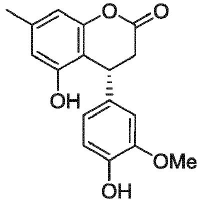 |
| | (R)5-hydroxy-4-(4-hydroxy-3-methoxyphenyl)-7-methylchroman-2-one, |
or a comestibly or biologically acceptable salt, solvate, or enantiomer, or a combination of any of the foregoing compounds, wherein said composition is capable of enhancing the sweet taste of the sweetener.

2. A method of enhancing the sweet taste of a sweetener in an edible composition, wherein said method comprises adding an effective amount of a compound of Formula (I), Compound 1, or a comestibly or biologically acceptable salt, solvate, or enantiomer, or a combination of any of the foregoing compounds, to said edible composition, such that the perception of sweetness intensity of said sweetener is enhanced.

3. The method of Claim 2, wherein an effective amount of Compound 1 or a solvate thereof is added.

4. The composition of claim 1 or the method of Claims 2 or 3, wherein the sweetener is a caloric sweetener, an artificial sweetener, an artificial high-potency sweetener, a natural high-potency sweetener, a sugar alcohol, a rare sugar, or a combination thereof.

5. The composition or method of Claim 4, wherein the caloric sweetener is a carbohydrate selected from sucrose, high fructose corn or starch syrup, glucose, and fructose.

6. The composition or method of Claim 5, wherein the caloric sweetener is high fructose corn or starch syrup.

7. The composition of any one of claims 1, 4, 5, or 6 or the method of any one of Claims 2-6, wherein said edible composition further comprises one or more sweet taste improving additive.

8. The composition or method of Claim 7, wherein said sweet taste improving additive is selected from the group consisting of a carbohydrate, a polyol, a glycoside, an amino acid, a sugar acid, a polyamino acid, a nucleotide, a salt, an organic acid, an organic ester, a flavoring agent, a sweet flavor, an alcohol, a flavonoid, a bitter compound, a protein, a protein hydrolysate, an emulsifier, a surfactant and a polymer.

9. A method of preparing an edible composition comprising:
(a) providing a comestibly acceptable carrier; and
(b) adding to said comestibly acceptable carrier a compound of Formula (I), Compound 1, or a comestibly or biologically acceptable salt, solvate, or enantiomer, or a combination of any of the foregoing compounds.

10. The method of Claim 9, wherein the comestibly acceptable carrier comprises a sweetener.

11. A tabletop sweetener composition comprising a composition according to claim 1.

12. A delivery system selected from the group consisting of a co-crystallized flavor composition with a sugar or a polyol, an agglomerated flavor composition, a compacted flavor composition, a dried flavor composition, a particle flavor composition, a spheronized flavor composition, a granular flavor composition or a liquid flavor composition, wherein the flavor composition comprises a composition according to claim 1.

13. The edible composition of claim 1, wherein said composition is selected from a food product, beverage composition, consumer product, or pharmaceutical composition.

## Patentansprüche

1. Eine essbare Zusammensetzung, umfassend einen Süßstoff und eine Verbindung der Formel (I): 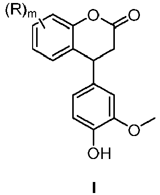 wobei R -OH, C₁-C₄-Alkyl, -CO₂H, Acyl oder Formyl ist; m 2 oder 3 ist; und mindestens ein R nicht OH ist; oder ein genießbar verträgliches Salz, Enantiomer oder Solvat davon oder Verbindung 1 mit der Struktur
| | |
|---|---|
| Verbindung 1 | 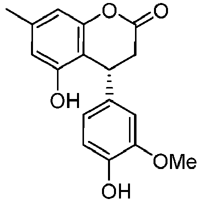 |
| | (R)5-Hydroxy-4-(4-hydroxy-3-methoxyphenyl)-7-methylchroman-2-on, |
oder ein genießbar oder biologisch verträgliches Salz, Solvat oder Enantiomer oder eine Kombination irgendwelcher der vorstehenden Verbindungen, wobei besagte Zusammensetzung in der Lage ist, den süßen Geschmack des Süßstoffs zu verstärken.

2. Ein Verfahren zur Verstärkung des süßen Geschmacks eines Süßstoffs in einer essbaren Zusammensetzung, wobei besagtes Verfahren das Hinzufügen einer wirksamen Menge einer Verbindung der Formel (I), Verbindung 1 oder eines genießbar oder biologisch verträglichen Salzes, Solvats oder Enantiomers oder einer Kombination irgendwelcher der vorgenannten Verbindungen zur besagten essbaren Zusammensetzung umfasst, sodass die Wahrnehmung der Süßeintensität besagten Süßstoffs verstärkt wird.

3. Das Verfahren nach Anspruch 2, wobei eine wirksame Menge von Verbindung 1 oder einem Solvat davon hinzugegeben wird.

4. Die Zusammensetzung nach Anspruch 1 oder das Verfahren nach Anspruch 2 oder 3, wobei der Süßstoff ein kalorischer Süßstoff, ein künstlicher Süßstoff, ein künstlicher hochwirksamer Süßstoff, ein natürlicher hochwirksamer Süßstoff, ein Zuckeralkohol, ein seltener Zucker oder eine Kombination davon ist.

5. Die Zusammensetzung oder das Verfahren nach Anspruch 4, wobei der kalorische Süßstoff ein Kohlenhydrat ist, ausgewählt aus Saccharose, Mais mit hohem Fruktosegehalt oder Stärkesirup, Glucose und Fruktose.

6. Die Zusammensetzung oder das Verfahren nach Anspruch 5, wobei der kalorische Süßstoff Mais mit hohem Fruktosegehalt oder Stärkesirup ist.

7. Die Zusammensetzung nach einem der Ansprüche 1, 4, 5 oder 6 oder das Verfahren nach einem der Ansprüche 2-6, wobei die essbare Zusammensetzung ferner ein oder mehrere den süßen Geschmack verbesserndes Additiv umfasst.

8. Die Zusammensetzung oder das Verfahren nach Anspruch 7, wobei besagtes den süßen Geschmack verbesserndes Additiv ausgewählt ist aus der Gruppe bestehend aus einem Kohlenhydrat, einem Polyol, einem Glykosid, einer Aminosäure, einer Zuckersäure, einer Polyaminosäure, einem Nukleotid, einem Salz, einer organischen Säure, einem organischen Ester, einem Aromastoff, einem süßen Aroma, einem Alkohol, einem Flavonoid, einer Bitterverbindung, einem Protein, einem Proteinhydrolysat, einem Emulgator, einem oberflächenaktiven Mittel und einem Polymer.

9. Ein Verfahren zur Herstellung einer essbaren Zusammensetzung, umfassend:
(a) Bereitstellung eines genießbar verträglichen Trägers; und
(b) Hinzufügen einer Verbindung der Formel (I), Verbindung 1 oder eines genießbar oder biologisch verträglichen Salzes, Solvats oder Enantiomers oder einer Kombination irgendwelcher der vorstehenden Verbindungen zu besagtem genießbar verträglichen Träger.

10. Das Verfahren nach Anspruch 9, wobei der genießbar verträgliche Träger einen Süßstoff umfasst.

11. Eine Tafelsüßstoffzusammensetzung, umfassend eine Zusammensetzung nach Anspruch 1.

12. Ein Abgabesystem, ausgewählt aus der Gruppe bestehend aus einer co-kristallisierten Aromazusammensetzung mit einem Zucker oder einem Polyol, einer agglomerierten Aromazusammensetzung, einer verdichteten Aromazusammensetzung, einer getrockneten Aromazusammensetzung, einer Partikelaromazusammensetzung, einer sphäronisierten Aromazusammensetzung, einer granularen Aromazusammensetzung oder einer flüssigen Aromazusammensetzung, worin die Aromazusammensetzung eine Zusammensetzung nach Anspruch umfasst.

13. Die essbare Zusammensetzung nach Anspruch 1, wobei besagte Zusammensetzung ausgewählt ist aus einem Lebensmittel, einer Getränkezusammensetzung, einem Konsumprodukt oder einer pharmazeutischen Zusammensetzung.

## Revendications

1. Composition consommable comprenant un édulcorant et un composé de Formule (I) : 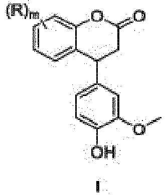 où R représente un -OH, alkyle en C₁-C₄, -CO₂H, acyle ou formyle ; m prend la valeur 2 ou 3 ; et un R au moins ne représente pas un -OH ; ou un sel, énantiomère, ou solvate de celui-ci acceptable sur le plan comestible, ou Composé 1 ayant la structure : 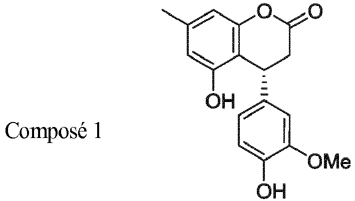 (R)-5-hydroxy-4-(4-hydroxy-3-méthoxyphényl)-7-méthylchroman-2-one,
un sel, énantiomère, ou solvate de celui-ci acceptable sur le plan comestible ou biologique, ou une combinaison des composés précédents, où ladite composition est capable de renforcer le goût sucré de l'édulcorant.

2. Procédé de renforcement du goût sucré d'un édulcorant dans une composition consommable, où ledit procédé comprend l'addition d'une quantité efficace d'un composé de Formule (I), du Composé 1, ou d'un sel, solvate ou énantiomère acceptable sur le plan comestible ou biologique ou d'une combinaison de l'un quelconque des composés précédents à ladite composition consommable, de sorte que la perception de l'intensité de la saveur sucrée dudit édulcorant est renforcée.

3. Procédé de la revendication 2, où une quantité efficace du Composé 1 ou d'un solvate de celui-ci est ajoutée.

4. Composition de la revendication 1 ou procédé des revendications 2 ou 3, où l'édulcorant est un édulcorant calorifique, un édulcorant artificiel, un édulcorant artificiel de haute puissance, un édulcorant naturel de haute puissance, un alcool de sucre, un sucre rare ou une combinaison de ceux-ci.

5. Composition ou procédé de la revendication 4, où l'édulcorant calorifique est un hydrate de carbone sélectionné parmi le sucrose, un sirop de maïs ou d'amidon riche en fructose, le glucose et le fructose.

6. Composition ou procédé de la revendication 5, où l'édulcorant calorifique est un sirop de maïs ou d'amidon riche en fructose.

7. Composition de l'une quelconque des revendications 1, 4, 5 ou 6 ou procédé de l'une quelconque des revendications 2-6, où ladite composition consommable comprend en outre un ou plusieurs additifs de renforcement du goût sucré.

8. Composition ou procédé de la revendication 7, où ledit additif de renforcement du goût sucré est sélectionné dans le groupe consistant en un hydrate de carbone, un polyol, un glycoside, un acide aminé, un acide de sucre, un polyamino-acide, un nucléotide, un sel, un acide organique, un ester organique, un aromatisant, un arôme sucré, un alcool, un flavonoïde, un composé amer, une protéine, un hydrolysat protéique, un émulsifiant, un agent tensio-actif et un polymère.

9. Procédé de préparation d'une composition consommable comprenant les étapes qui consistent à :
(a) se munir d'un véhicule acceptable sur le plan comestible ; et
(b) ajouter au véhicule acceptable sur le plan comestible un composé de Formule (I), un Composé 1 ou un sel, solvate ou énantiomère acceptable sur le plan comestible ou biologique ou une combinaison de quelconques des composés précédents.

10. Procédé de la revendication 9, où le véhicule acceptable sur le plan comestible comprend un édulcorant.

11. Composition édulcorante de table comprenant une composition selon la revendication 1.

12. Système de distribution sélectionné dans le groupe consistant en une composition aromatisante co-cristallisée avec un sucre ou un polyol, une composition aromatisante agglomérée, une composition aromatisante compacte, une composition aromatisante sèche, une composition aromatisante particulaire, une composition aromatisante sphéronisée, une composition aromatisante granulaire ou une composition aromatisante liquide, où la composition aromatisante comprend une composition selon la revendication 1.

13. Composition consommable de la revendication 1, où ladite composition est sélectionnée parmi un produit alimentaire, une composition buvable, un produit de consommation ou une composition pharmaceutique.
